# EUROPEAN PATENT APPLICATION

(11) **EP 4 177 246 A1**
(43) Date of publication of application: **10.05.2023**
(21) Application number: 21382995.5
(22) Date of filing: 04.11.2021
(51) Int. Cl.: C07D 267/20, C07D 413/04, A61P 35/00, A61K 31/553

(54) **FUSED OXAZEPINES AS INHIBITORS OF PEROXIREDOXINS**

(71) Applicant: Centro Atlántico del Medicamento, SA, 38204 Santa Cruz de Tenerife (ES)
(72) Inventor: McNaughton-Smith, Grant, 38470 Los Silos (Santa Cruz de Tenerife) (ES); Hueso-Falcón, Idaira, 38206 San Cristóbal de La Laguna (Santa Cruz de Tenerife) (ES); Díaz Chico, Juan Carlos, 35214 Telde (Las Palmas de Gran canaria) (ES); Spinola Lasso, Elena, 35010 Las Palmas de Gran Canaria (ES); Montero González, Juan Carlos, 37184 Villares de la Reina (Salamanca) (ES); Pandiella Alonso, Atanasio, 37006 Salamanca (ES); Díaz Chico, Bonifacio Nicolás, 35310 Monte Lentiscal (Las Palmas de Gran Canaria) (ES)
(74) Representative: Manuel Illescas y Asociados, S.L.

(57) **Abstract**

Novel quinone-fused oxazepines compounds that inhibit peroxiredoxins, pharmaceutical compositions comprising such compounds, are disclosed. Additionally, methods of treating or alleviating conditions associated with elevated peroxiredoxins levels, and in particular cancer, are also disclosed.

## Description

### FIELD OF THE INVENTION

The present invention discloses novel quinone-fused oxazepines compounds that inhibit peroxiredoxins, pharmaceutical compositions comprising the same, and methods of treating or alleviating conditions associated with elevated peroxiredoxins levels, and in particular cancer, by administering said compounds.

### BACKGROUND OF THE INVENTION

Peroxiredoxins (PRDXs, also abbreviated as Prxs) enzymes are a family of cysteine-dependent peroxidases that catalyse the reduction of H₂O₂, alkyl hydroperoxides and peroxynitrite to water, the corresponding alcohol and nitrite, respectively (ref. 1). PRDXs are emerging as arguably the most important and widespread peroxide and peroxynitrite scavenging enzymes in all of biology (refs. 2,3). Hydrogen peroxide works as a second messenger; therefore, its generation and removal are strictly regulated. Although other groups of enzymes such as catalases and glutathione peroxidases also scavenge hydrogen peroxide, PRDXs are crucial antioxidants due to their abundance and prevalence in various cellular compartments. PRDXs are highly involved in the control of cellular physiological functions, including growth, differentiation, apoptosis, embryonic development, lipid metabolism, the immune response, as well in the maintenance of cellular homeostasis (ref. 4). They are known for example to also directly interact with a variety of key signalling proteins, such as MAPKs (ref. 5), c-ABL (ref. 6), c-MYC (ref. 7), tyrosine phosphatase PTEN (ref. 8), ASK1 (ref. 9) and STATs (ref. 10).

In mammals, there are six PRDXs, classified depending on the number and localization of the catalytic cysteine residues into 3 subtypes: typical 2-Cys (PRDX1-4), atypical 2-Cys (PRDX5), and 1-Cys (PRDX6) peroxiredoxins. PRDX1, PRDX2, PRDX3, PRDX5 and PRDX6 are localized in the cytosol, in the mitochondria, in the nuclei and in the peroxisomes (ref. 11-13), whereas PRDX4 is mainly present in the endoplasmic reticulum, or it is secreted (ref. 14).

Typical 2-Cys PRDXs (PRDX1-4) form decamers or dodecamers in the reduced or hyperoxidized state, acquiring the ability to exercise other functions as chaperones, binding partners, enzyme activators and/or redox sensors, while the oxidized form is preferentially present as dimers (ref. 15). Atypical 2-Cys PRDXs are able to undergo protein-protein interactions with functional implications, although their level of polymerization is less compared with that of typical 2-Cys PRDXs. By contrast, 1-Cys PRDXs are not able to form decamers, and this is probably the reason why they serve mainly an antioxidant function rather than a molecular chaperone function, despite their enzymatic mechanism being very similar to that of 2-Cys PRDXs (ref. 16).

For all subtypes, there are two steps in peroxide reduction. Firstly, the thiol of a peroxidatic Cys attacks the peroxide oxygen and becomes oxidized to sulfenic acid. Secondly, the sulfenic acid is attacked by a resolving Cys thiol, and a disulfide bond is formed. In typical 2-Cys PRDXs, which are head-to-tail functional homodimers, the resolving Cysteine comes from the C-terminus of the other monomer. Atypical 2-Cys and 1-Cys PRDXs, with resolving cysteines in the same subunit or coming from other proteins or peptides, respectively, are functional monomers. Finally, to complete the catalytic cycle, the disulfide bond formed in the PRDX active site is reduced by the TRX-TRXR-NADPH system (ref. 17).

Emerging data have implicated an association between alterations in the amount of two-cysteine PRDX isoform proteins and major human diseases such as hyper-proliferative diseases, as cancer, cardiovascular diseases, diabetes, bone loss, chronic kidney disease neurodegenerative diseases and immunological diseases.

For instance, tumour cells are characterized by high proliferation rate, increased metabolism, and consequently increased levels of reactive oxygen species (ROS). To counterbalance this, tumour cells up-regulate antioxidant proteins such as PRDXs (ref. 1, 19, 20). In particular, over the course of the last few years, a large body of evidence has suggested their involvement in carcinogenesis and in the development of drug resistance (ref. 18). Increased levels of PRDXs have also been associated with increased resistance to both radiotherapy and certain types of chemotherapy (ref. 21-23).

A few small molecular inhibitors of PRDXs are known, with the majority showing selectivity for PRDX1-3 over PRDX4-6. The natural product adenanthin for example, covalently interacts with both PRDX1 and PRDX2, in acute promyelocytic leukemia (APL) cells leading to increased cellular levels of H₂O₂, activation of extracellular signal regulated kinases, and finally apoptosis. Administration of adenanthin to mice with induced APL tumours, suppressed tumour growth and prolonged survival (ref. 24). A synthetically derived small peptidomimetic molecule, SK053, has also been shown to bind PRDX1, PRDX2 and PRDX3 in lymphoma cells, leading to the conversion of peroxide active monomeric forms of PRDX1 to inactive dimeric and oxidized forms (ref. 25). This resulted in the rapid activation of several kinases, and induced apoptosis. In 2015, a preclinical proof-of-concept study described the potential use of AMRI-59, a small molecule PRDX1 inhibitor, as an anticancer agent (ref. 26). More recently the natural product frenolicin B, and certain synthetic derivatives, were shown to potently bind to PRDX1 and Glutaredoxin 3 (GRX3) in a covalent manner (ref. 27). They increased cellular ROS levels, and cell death in human derived colorectal cancer (CRC) cells. Furthermore, one derivative was shown to reduce tumour growth in vivo, when administered to mice bearing the same CRC cell lines.

These findings highlight the potential applications of PRDX inhibitors, especially against hyper-proliferative diseases. Accordingly, the development of PRDX inhibitors is an important therapeutic target in particular for the treatment of cancer.

### BRIEF DESCRIPTION OF THE INVENTION

The present invention relates to compounds which act as peroxiredoxins (PRDXs) inhibitors, wherein said compounds are quinone-fused oxazepines which are characterized for having a formula (I), or a pharmaceutically acceptable salt thereof: 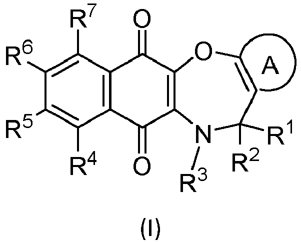 wherein
A is an aromatic carbocyclic 5- or 6-membered ring or an aromatic heterocyclic 5- or 6-membered ring comprising one or more heteroatoms selected independently from the group consisting of O, N and S, wherein said aromatic carbocyclic ring or said aromatic heterocyclic ring is unsubstituted or substituted at one or more atoms of C with one or more R⁸ substituents; wherein each R⁸ substituent is independently selected from the group consisting of: H, halogen, nitro, cyano, C₁-C₆ alkyl, C₁-C₆ haloalkyl, -OR⁹ and -C(O)OR⁹, and wherein R⁹ is selected from the group consisting of: H and C₁-C₆ alkyl;
R¹ and R² are independently selected from the group consisting of: H, halogen and C₁-C₆ alkyl;
R³ is an aromatic carbocyclic 5- or 6-membered ring or an aromatic heterocyclic 5- or 6-membered ring comprising one or more heteroatoms selected independently from the group consisting of O, N and S, wherein said aromatic carbocyclic ring or said aromatic heterocyclic ring is unsubstituted or substituted at one or more atoms of C with one or more R¹⁰ substituents; wherein each R¹⁰ substituent is independently selected from the group consisting of: H, halogen, C₁-C₆ alkyl, C₁-C₆ haloalkyl, nitro, cyano, -C(O)NR¹¹R¹², -OR¹¹, -C(O)OR¹¹, -NR¹¹R¹², -SR¹¹, -SO₂R¹¹, and -SO₂NR¹¹R¹²;
R⁴, R⁵, R⁶ and R⁷ are each independently selected from the group consisting of: H, halogen, C₁-C₆ alkyl, C₁-C₆ haloalkyl, -OR¹¹, -OC(O)R¹¹, nitro, cyano, -NR¹¹R¹², -NR¹¹SO₂R¹⁶, -SR¹¹, -SO₂R¹¹ and -SO₂NR¹¹R¹²;
wherein R¹¹ and R¹² are each independently selected from the group consisting of: H, C1-C6 alkyl, -R¹³-C(O)OR¹⁴, -R¹³-OR¹⁴ and -R¹³-NR¹⁴R¹⁵, wherein R¹³ is a group -(CH₂)*n-*where *n* is 1, 2, 3 or 4, and wherein R¹⁴ and R¹⁵ are each independently H or C₁-C₆ alkyl;
or wherein R¹¹ and R¹² when taken with the atom to which they are both attached, complete a 5- or 6-membered substituted or unsubstituted cycloalkyl or a 5- or 6-membered substituted or unsubstituted heterocycloalkyl group comprising at least one heteroatom selected from the group consisting of O, N and S; and wherein R¹⁶ is C₁-C₆ alkyl or C₁-C₆ haloalkyl.

Present invention refers to an oxazepine compound of formula (I), or a pharmaceutically acceptable salt thereof: 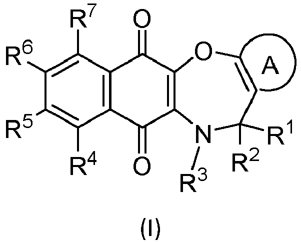 wherein
A is a phenyl ring unsubstituted or substituted with one or more R⁸ substituents, wherein each R⁸ substituent is independently selected from the group consisting of: H, halogen, -CF₃, -OR⁹ and -C(O)OR⁹, and wherein R⁹ is selected from the group consisting of: H and C₁-C₆ alkyl;
R¹ and R² are both H;
R³ is a phenyl ring, a pyridyl ring or a pyrimidinyl ring, and wherein said ring is unsubstituted or substituted at one or more atoms of C with one or more R¹⁰ substituents, wherein each R¹⁰ substituent is independently selected from the group consisting of: H, halogen, cyano, -CF₃, -C(O)NR¹¹R¹², -OR¹¹, -C(O)OR¹¹, -NR¹¹R¹² and -SO₂NR¹¹R¹²;
R⁴ and R⁵ are each independently selected from the group consisting of: H, -OR¹¹, -OC(O)R¹¹, nitro, -NR¹¹R¹² and -NR¹¹SO₂R¹⁶; and R⁵ and R⁶ are each independently selected from the group consisting of: H, halogen, -NR¹¹R¹² and -SO₂NR¹¹R¹²;
wherein R¹¹ and R¹² are each independently selected from the group consisting of: H, C1-C6 alkyl, -R¹³-C(O)OR¹⁴, -R¹³-OR¹⁴ and -R¹³-NR¹⁴R¹⁵, wherein R¹³ is a group -(CH₂)*n-*where *n* is 1 or 2, and wherein R¹⁴ and R¹⁵ are each independently H or C₁-C₆ alkyl;
or wherein R¹¹ and R¹² when taken with the atom to which they are both attached, complete a 6-membered substituted or unsubstituted heterocycloalkyl group comprising one or two heteroatoms selected from the group consisting of O and N; and wherein R¹⁶ is methyl, ethyl or -CF₃.

The present invention also refers to a compound of formula (I), according to the present invention, or to a pharmaceutically acceptable salt thereof, for use as a medicament and, in particular, for use in the prevention and/or treatment of a disease or condition responsive or ameliorated by inhibition of one or more peroxiredoxins, in particular, for use in the prevention and/or treatment of cancer.

Finally, the present invention also refers to a pharmaceutical composition comprising at least one compound of formula (I), according to the present invention, or a pharmaceutically acceptable salt thereof, and at least one pharmaceutically acceptable excipient, for use as a medicament and, in particular, for use in the prevention and/or treatment of a disease or condition responsive or ameliorated by inhibition of one or more peroxiredoxins, in particular, for use in the prevention and/or treatment of cancer.

### BRIEF DESCRIPTION OF THE FIGURES

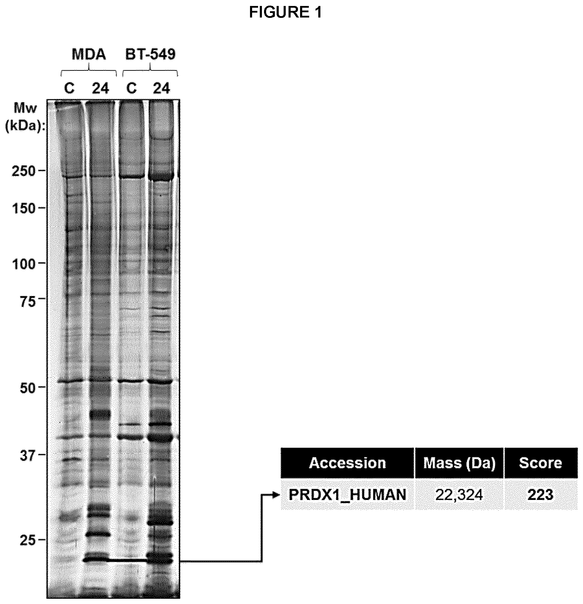

**Figure 1****: Identification of PRDX1 as a target for compound 24 using resin bound affinity chromatography.** Lysates from either MDA-MB-231 or BT-549 cells, were incubated with uncoupled, or compound 24-coupled, epoxy-activated 6B resin and the resulting complexes were isolated and analyzed by SDS-PAGE and silver staining (left). Protein bands of interest were excised from the gel and analyzed by MALDI-TOF to identify the bound proteins (right).

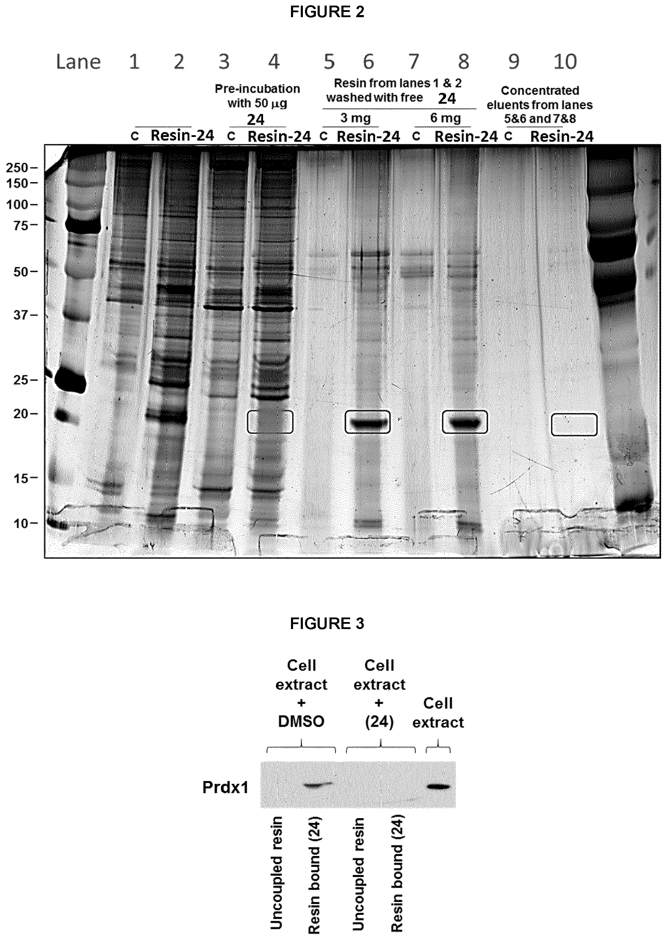

**Figure 2****: Assessment of the type of coupling of compound 24 to PRDX.** Using the same method as describe for figure 1, resins were incubated with cell lysates that had been pre-treated with free compound 24, or untreated, and the bound proteins identified by SDS-PHAGE staining. Furthermore, resins incubated with untreated cell lysates were also subjected to secondary washings with free compound 24, to determine the type of binding present between the proteins and 24.
**Figure 3****: Confirmation of PRDX1 as a target for compound 24 by competitive immunoprecipitation experiments.** Treatment of untreated MDA-MB-231 cell lysates with a 24-coupled resin were subjected to immunoblot analysis for PRDX1. PRDX1 was clearly trapped by the 24-coupled resin. Conversely, 24-coupled resin was not able to trap PRDX1 from lysates pre-treated with free compound 24.

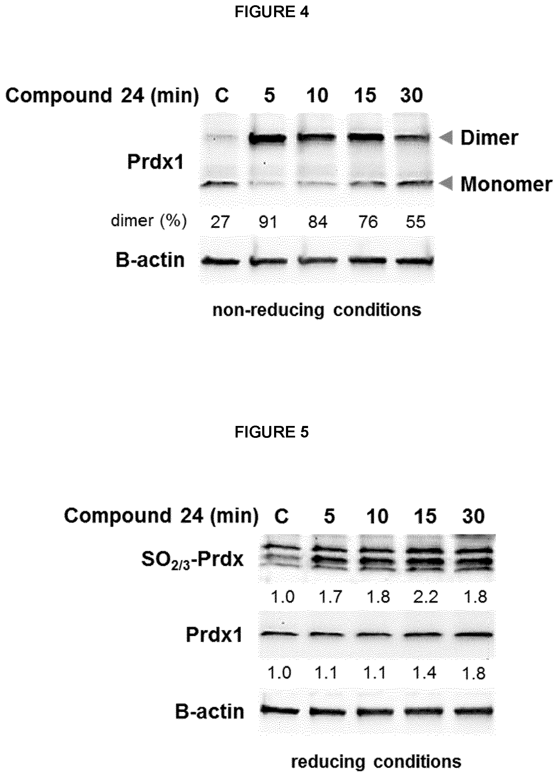

**Figure 4****: Addition of compound 24 to MDA-MB-231 cells induces dimer formation of PRDX1.** Evaluation of monomeric and dimeric forms of PRDX1 in MDA-MB-231 cells following treatment with compound 24 by western blot analysis. A temporal transition from the active monomeric form to the inactive dimeric form was observed.
**Figure 5****: Addition of 24 to MDA-MB-231 cells leads to an increase in the oxidized forms of PRDX1.** Evaluation of the oxidative states of PRDX1 in MDA-MB-231 cells following treatment with compound 24 by western blot analysis. A temporal increase in the levels of oxidized (inactivated) PRDX1 was observed.

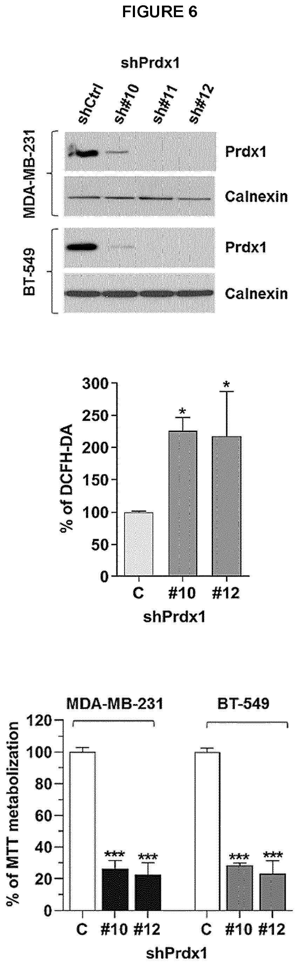

**Figure 6****: Efficacy of different shPRDX1s (short-hairpin PRDX1) to generate TNBC cell lines with PRDX1 knocked-down, and the resulting effects on ROS and cell viability.** Evaluation of the effects of downregulating PRDX1 (top image) in TNBC cells (MDA-MB-231 and BT549) using short-hairpin RNAs. Downregulation of PRDX1 induced an increase in the levels of ROS (% of DCFH-DA, middle), and a decrease in the percentage of viable cells (MTT assay, bottom).

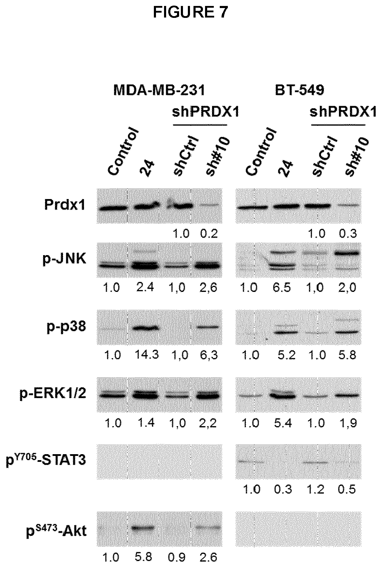

**Figure 7****: Immunoblot of phosphorylated MAPKs, STAT3, and Akt of PRDX1-knocked down TNBC cells, compared to the effects seen after the administration of compound 24 (5 µM, 1 h).** A comparison of the effects of compound 24 and knocking down PRDX1 in TNBC cells. In particular, their effects on signalling pathways important for cell survival and proliferation in TNBC cells. Numbers correspond to the amount of protein relative to its appropriate control. Both compound 24, and TNBC cells with PRDX1 knocked-down, caused an increase in the levels of activated JNK, p38, AKT and ERK1/2, and a decrease in the activation of STAT3.

### DETAILED DESCRIPTION

The present invention refers to quinone fused-oxazepines compounds of formula (I), or a pharmaceutically acceptable salt thereof, as disclosed herein, which reduce the cyto-protective functions of peroxiredoxins. The fused-oxazepine compounds of formula (I) according to the present invention are polycyclic compounds comprising a quinone moiety fused with an oxazepine moiety and are characterized for having a formula (I): 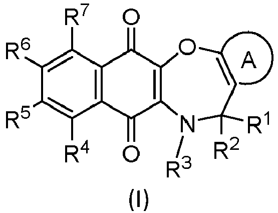 wherein
A is an aromatic carbocyclic 5- or 6-membered ring or an aromatic heterocyclic group comprising one or more heteroatoms selected independently from the group consisting of O, N and S, wherein said aromatic carbocyclic ring or said aromatic heterocyclic ring is unsubstituted or substituted at one or more atoms of C with one or more R⁸ substituents; wherein each R⁸ substituent is independently selected from the group consisting of: H, halogen, nitro, cyano, C₁-C₆ alkyl, C₁-C₆ haloalkyl, -OR⁹ and -C(O)OR⁹, and wherein R⁹ is selected from the group consisting of: H and C₁-C₆ alkyl;
R¹ and R² are independently selected from the group consisting of: H, halogen and C₁-C₆ alkyl;
R³ is an aromatic carbocyclic 5- or 6-membered ring or an aromatic heterocyclic 5- or 6-membered ring comprising one or more heteroatoms selected independently from the group consisting of O, N and S, wherein said aromatic carbocyclic ring or said aromatic heterocyclic ring is unsubstituted or substituted at one or more atoms of C with one or more R¹⁰ substituents; wherein each R¹⁰ substituent is independently selected from the group consisting of: H, halogen, C₁-C₆ alkyl, C₁-C₆ haloalkyl, nitro, cyano, -C(O)NR¹¹R¹², -OR¹¹, -C(O)OR¹¹, -NR¹¹R¹², -SR¹¹, -SO₂R¹¹, and -SO₂NR¹¹R¹²;
R⁴, R⁵, R⁶ and R⁷ are each independently selected from the group consisting of: H, halogen, C₁-C₆ alkyl, C₁-C₆ haloalkyl, -OR¹¹, -OC(O)R¹¹, nitro, cyano, -NR¹¹R¹², -NR¹¹SO₂R¹⁶, -SR¹¹, -SO₂R¹¹ and -SO₂NR¹¹R¹²;
wherein R¹¹ and R¹² are each independently selected from the group consisting of: H, C1-C6 alkyl, -R¹³-C(O)OR¹⁴, -R¹³-OR¹⁴ and -R¹³-NR¹⁴R¹⁵, wherein R¹³ is a group -(CH₂)*n-*where *n* is 1, 2, 3 or 4, and wherein R¹⁴ and R¹⁵ are each independently H or C₁-C₆ alkyl;
or wherein R¹¹ and R¹² when taken with the atom to which they are both attached, complete a 5- or 6-membered substituted or unsubstituted cycloalkyl or a 5- or 6-membered substituted or unsubstituted heterocycloalkyl group comprising at least one heteroatom selected from O, N or S; and wherein R¹⁶ is C₁-C₆ alkyl or C₁-C₆ haloalkyl.

Present disclosure refers also to the pharmaceutically acceptable salts, solvates and polymorphs of the compounds of formula (I) described according to the present invention.

The term "pharmaceutical acceptable" is employed herein to refer to those compounds, materials, compositions, and/or dosage forms which, when contacted with the tissue of human beings or animals, possess a benefit/risk ratio greater than 1. As used herein "pharmaceutically acceptable salts" refers to derivatives of the disclosed compounds wherein the parent compound is modified by converting an existing acid or base moiety to its salt form. Examples of pharmaceutically acceptable salts include, but are not limited to;
- mineral or organic acid salts of basic residues such as amines. Examples of mineral acid salts of basic residues include hydrochloride, hydrobromide, hydroiodide, phosphate and sulfatesalts. Examples of organic acid salts of basic residues include tartrate (*e*.*g*. (+)-L-tartrate), maleate, citrate, acetate, lactate, mesylate, methylsulfate and fumarate salts;
- alkali or organic salts of acidic residues such as carboxylic acids. Examples of alkali salts of acidic residues include sodium, potassium, calcium, magnesium and ammonium salts. Examples of organic salts of acidic residues include L-argininate, N-methylglucaminate, cholinate, lysinate, benethaminate, diethylaminate, 2-diethylaminoethanol base, 4-(2-hydroxyethyl)morpholine base, 1-(2-hydroxyethyl)pyrrolidine base and trimethaminate base salts;.

The salts may be formed by conventional means, such as by reacting the free base form of the compound with one or more equivalents of the appropriate acid in a solvent or medium in which the salt is insoluble, or in a solvent such as water which can be removed in vacuo or by freeze drying or by exchanging the anions of an existing salt for another anion using a suitable ion exchange resin.

The term "solvate" in accordance with this invention should be understood as meaning any form of the active compound in accordance with the invention in which said compound is bonded by a non-covalent bond to another molecule (normally a polar solvent), including especially hydrates and alcoholates.

The term "polymorph" refers to crystalline forms having the same chemical composition but different spatial arrangements of the molecules, atoms, and/or ions forming the crystal.

Certain compounds of the present invention can exist in unsolvated forms as well as solvated forms, including hydrate forms. Certain compounds of the present invention may exist in multiple crystalline or amorphous forms.

The abbreviations used herein have their conventional meaning within the chemical and biological arts. For example, DMSO, dimethylsulfoxide; MeOH, methanol; RPMI medium, Roswell Park Memorial Institute medium; THF, tetrahydrofuran; DCM, dichloromethane; EtOAc, ethyl acetate, EtOH, ethanol; MTT, 3-(4,5-methyltiazol-2yl)-2,5diphenyl-tetrazolium bromide]; FBS, Fetal bovine serum; MgSO₄, Magnesium sulphate anhydrous; HCI, hydrochloric acid, H₂SO₄, sulfuric acid, AcOH, acetic acid; ATCC, American Type Culture Collection; DMEM, Dulbecco's Modified Eagle's Medium.

For the purposes of present invention, the term "comprises" may be replaced by any of the terms "consist of", or "consists essentially of". Accordingly, "comprises" may refer to a group of features A, B and C, which additionally may include other features, such as E and D, with the condition that said features do not render the claim unworkable, but said term "comprises" also includes the situation in which the group of features "consists of" or "consists essentially" of A, B and C.

The term "C₁-C₆ alkyl" refers to straight chain or branched chain hydrocarbon groups having from 1 to 6 carbon atoms. Examples include methyl, ethyl, propyl, isopropyl, butyl, isobutyl, sec-butyl, tert-butyl, pentyl, neopentyl and hexyl, preferably ethyl, propyl, isopropyl, n-butyl, isobutyl, sec-butyl, tert-butyl, pentyl, neopentyl and hexyl, more preferably ethyl, propyl, isopropyl, n-butyl, tert-butyl or pentyl.

The term "cycloalkyl group" refers to a non-aromatic carbocycle or cyclic hydrocarbon group. Examples include cyclopropyl, cyclobutyl, cyclopentyl, cyclopentenyl, cyclohexyl, cyclohexenyl, cycloheptyl, norbornyl and adamantly.

The terms "heterocyclic group" or "heterocycloalkyl group" refer both indistinctively to non-aromatic carbocycles having one or more ring forming heteroatoms such as N, O and S atoms. Heterocyclic groups may include monocyclic and polycyclic (e.g., having 2, 3 or 4 fused rings) systems as well as spirocycles. Example groups include morpholino, thiomorpholino, piperazinyl, tetrahydrofuranyl, tetrahydrothienyl, 2,3-dihydrobenzofuryl, piperidinyl and pyrrolidinyl. Ring forming carbon atoms and heteroatoms of a heterocyclic group can be optionally substituted by oxo or sulfido. Also included in the definition of heterocyclic group are moieties that have one or more aromatic rings fused (i.e., having a bond in common with) to the non-aromatic heterocyclic ring. Examples of heterocyclic groups include without limitation, phthalimidyl, naphthalimidyl, and benzo derivatives of heterocycles. The heterocyclic group can be attached through a ring-forming carbon atom or a ring-forming heteroatom. The number of carbon atoms present in the heterocyclic group can range from 1 to 20 and may include double or triple bonds.

The terms "aryl group" or "aromatic carbocyclic group" refer both indistinctively to single, polynuclear, conjugated or fused residues of cyclic aromatic hydrocarbons, including both substituted or unsubstituted versions thereof. Examples of "aryl groups" or "aromatic carbocycle groups" include: phenyl, biphenyl, naphthyl, tetrahydronaphthyl, anthracenyl, benzanthracenyl, phenylanthrenyl, nitrophenyl, chlorophenyl, bromophenyl, fluorophenyl, iodophenyl, benzo[d][1,3]dioxol-5-yl, alkylphenyls, alkoxyphenyl, hydroxyphenyl, aminophenyl, cyanophenyl and mercaptophenyl, preferably 3-nitrophenyl, 4-nitrophenyl, 3-fluorophenyl, 4-fluorophenyl, 3-iodophenyl, 4-iodophenyl, ethylphenyl, isopropylphenyl, 3-benzamide, 4-benzamide, aminophenyl, 3-cyanophenyl, 4-cyanophenyl and mercaptophenyl, more preferably 4-nitrophenyl, 4-cyanophenyl, 4-benzamide and 3-cyanophenyl.

The term "heteroaryl group" or "aromatic heterocyclic group" refers to an aromatic carbocycle having at least one heteroatom. As used herein, the term "heteroatom" is meant to include oxygen (O), nitrogen (N), sulphur (S), silicon (Si) and phosphorus (P); preferably, said heteroatom is S, N or O. Heteroaryl groups include monocyclic and polycyclic systems. Examples of heteroaryl groups include without limitation, pyridyl, pyrimidinyl, pyrazinyl, pyridazinyl, triazinyl, furyl, quinolyl, isoquinolyl, thienyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl, preferably pyridyl, pyrimidinyl, pyrazinyl, pyridazinyl, triazinyl, furyl, quinolyl, isoquinolyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl. In some embodiments, any ring forming N can be substituted by an oxo, to form an N-oxide.

The term "halogen" refers to fluorine, chlorine, bromine and iodine. The terms "amino", "azido", "nitro", "cyano" and "hydroxyl" respectively refer to -NH₂, -N₃, - NO₂, -CN and -OH groups attached to the remainder of the molecule.

For the purpose of present disclosure the dash "-" featured in a group listed is linked to the atom by which said group is attached to the reminder of the molecule. For example, for the purposes of the present disclosure, the group -OC(O)CH₃ is an ester group which is attached to the reminder of the molecule by the oxygen atom, whereas the group -C(O)OCH₃ is an ester group which is attached to the reminder of the molecule by the carbon atom of the carbonyl moiety. When the "-" is not indicated in a group, it may be linked to the reminder of the molecule by any of the atoms which are capable of forming a bond.

The term "substituted or unsubstituted" refers to a group that may or may not be further substituted with one or more groups selected from (C₁-C₆)alkyl, Si[(C₁-C₆)alkyl]₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, amido, azido, nitro, cyano, carboxy, sulphonamide, urea, sulphone, acyl, mercapto, alkylamino, alkylthio and hydroxyl. Preferably, said term "substituted or unsubstituted" refers to a group that may or may not be further substituted with one or more groups selected from (C₁-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto and hydroxyl. In one embodiment said term "substituted or unsubstituted" refers to a group that may or may not be further substituted with one or more groups selected from (C₁-C₆)alkyl, Si(C₁-C₆)alkyl)₃, (C₁-C₆)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto, and the like. In an alternative embodiment "substituted or unsubstituted" refers to a group that may or may not be further substituted with one or more groups selected from (C₂-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, (C₂-C₆)alkoxyl, amino, azido, cyano, carboxy, sulphonamide, urea, sulphone, acyl and mercapto groups. In another embodiment "substituted or unsubstituted" refers to a group that may or may not be further substituted with one or more groups selected from (C₂-C₆)alkyl, (C₂-C₆)alkenyl, (C₂-C₆)alkoxyl, amino, cyano, carboxy, acyl, mercapto and hydroxyl groups, further preferably (C₂-C₆)alkyl, (C₂-C₆)alkenyl, (C₂-C₆)alkoxyl, amino, cyano, carboxy, acyl and mercapto groups.

According to present invention A is an aromatic carbocyclic 5- or 6-membered ring or an aromatic heterocyclic 5- or 6-membered ring comprising one or more heteroatoms selected independently from the group consisting of O, N and S, wherein said aromatic carbocyclic ring or said aromatic heterocyclic ring is unsubstituted or substituted at one or more atoms of C with one or more R⁸ substituents; wherein each R⁸ substituent is independently selected from the group consisting of: H, halogen, nitro, cyano, C₁-C₆ alkyl, C₁-C₆ haloalkyl, -OR⁹ and -C(O)OR⁹, and wherein R⁹ is selected from the group consisting of: H and C₁-C₆ alkyl.

Preferably A is an aromatic carbocyclic 6-membered ring or an aromatic heterocyclic 6-membered ring comprising one or more heteroatoms selected independently from the group consisting of O, N and S, wherein said carbocyclic ring or said heterocyclic ring is unsubstituted or substituted at one or more atoms of C with one or more R⁸ substituents, wherein each R⁸ substituent is independently selected from the group consisting of: H, halogen, nitro, cyano, -CF₃, -OR⁹ and -C(O)OR⁹, and wherein R⁹ is selected from the group consisting of: H and C₁-C₆ alkyl.

More preferably A is a phenyl group or a pyridyl group unsubstituted or substituted at one or more atoms of C with one or more R⁸ substituents, wherein each R⁸ substituent is independently selected from the group consisting of: H, halogen, nitro, cyano, -CF₃, -OR⁹ and -C(O)OR⁹, and wherein R⁹ is selected from the group consisting of: H and C₁-C₆ alkyl.

Even more preferably A is a phenyl ring or a pyridyl group unsubstituted or substituted at one or more atoms of C with one or more R⁸ substituents, wherein each R⁸ substituent is independently selected from the group consisting of: H, halogen, -CF₃, -OR⁹ and -C(O)OR⁹, and wherein R⁹ is selected from the group consisting of: H and C₁-C₆ alkyl.

Yet even more preferably A is a phenyl ring unsubstituted or substituted with one or more R⁸ substituents, wherein each R⁸ substituent is independently selected from the group consisting of: H, F, Cl, Br, -OR⁹ and -C(O)OR⁹, and wherein R⁹ is H, methyl or ethyl.

According to present invention R¹ and R² are independently selected from the group consisting of: H, halogen and C₁-C₆ alkyl. Preferably R¹ and R² are each independently H or F and more preferably R¹ and R² are both H.

R³ represents a moiety that is independently selected from substituted or unsubstituted aryl (i.e. aromatic carbocyclic group) or substituted or unsubstituted heteroaryl (i.e. aromatic heterocyclic group). According to present invention R³ is an aromatic carbocyclic 5- or 6-membered ring or an aromatic heterocyclic 5- or 6-membered ring comprising one or more heteroatoms selected independently from the group consisting of O, N and S, wherein said aromatic carbocyclic group or said aromatic heterocyclic group is unsubstituted or substituted at one or more atoms of C with one or more R¹⁰ substituents; wherein each R¹⁰ substituent is independently selected from the group consisting of: H, halogen, C₁-C₆ alkyl, C₁-C₆ haloalkyl, nitro, cyano, -C(O)NR¹¹R¹², -OR¹¹, -C(O)OR¹¹, -NR¹¹R¹², -SR¹¹, -SO₂R¹¹, and -SO₂NR¹¹R¹²; wherein R¹¹ and R¹² are each independently selected from the group consisting of: H, C₁-C₆ alkyl, -R¹³-C(O)OR¹⁴, -R¹³-OR¹⁴ and -R¹³-NR¹⁴R¹⁵, wherein R¹³ is a group -(CH₂)*n*- where *n* is 1, 2, 3 or 4, and wherein R¹⁴ and R¹⁵ are each independently H or C₁-C₆ alkyl; or wherein R¹¹ and R¹² when taken with the atom to which they are both attached, complete a 5- or 6-membered substituted or unsubstituted cycloalkyl or a 5- or 6-membered substituted or unsubstituted heterocycloalkyl group comprising at least one heteroatom selected from O, N or S.

Preferably R³ is a phenyl ring, a pyridyl ring or a pyrimidinyl ring, wherein said ring is unsubstituted or substituted at one or more atoms of C with one or more R¹⁰ substituents; wherein each R¹⁰ substituent is independently selected from the group consisting of: H, halogen, C₁-C₆ alkyl, -CF₃,, nitro, cyano, -C(O)NR¹¹R¹², -OR¹¹, -C(O)OR¹¹, -NR¹¹R¹², -SR¹¹, -SO₂R¹¹, and -SO₂NR¹¹R¹²; wherein R¹¹ and R¹² are each independently selected from the group consisting of: H, C₁-C₆ alkyl, -R¹³-C(O)OR¹⁴, -R¹³-OR¹⁴ and -R¹³-NR¹⁴R¹⁵, wherein R¹³ is a group -(CH₂)*n*- where *n* is 1, 2, 3 or 4, and wherein R¹⁴ and R¹⁵ are each independently H or C₁-C₆ alkyl; or wherein R¹¹ and R¹² when taken with the atom to which they are both attached, complete a 5- or 6- membered substituted or unsubstituted cycloalkyl or a 5- or 6-membered substituted or unsubstituted heterocycloalkyl group comprising at least one heteroatom selected from O, N or S.

More preferably R³ is a phenyl ring, a pyridyl ring or a pyrimidinyl ring ring is unsubstituted or substituted at one or more atoms of C with one or more R¹⁰ substituents, wherein each R¹⁰ substituent is independently selected from the group consisting of: H, halogen, -CF₃, cyano, -C(O)NR¹¹R¹², -OR¹¹, -C(O)OR¹¹, -NR¹¹R¹² and -SO₂NR¹¹R¹²; wherein R¹¹ and R¹² are each independently selected from the group consisting of: H, C₁-C₆ alkyl, -R¹³-C(O)OR¹⁴, -R¹³-OR¹⁴ and -R¹³-NR¹⁴R¹⁵, wherein R¹³ is a group -(CH₂)*n*- where *n* is 1 or 2, and wherein R¹⁴ and R¹⁵ are each independently H or C₁-C₆ alkyl; or wherein R¹¹ and R¹² when taken with the atom to which they are both attached, complete a 6-membered substituted or unsubstituted heterocycloalkyl group comprising one or two heteroatoms selected from the group consisting of O and N. More preferably, the 6-membered heterocycloalkyl group comprises one or two heteroatoms selected from O or N and at least one C₁-C₆ alkyl substitution.

Even more preferably R³ is a phenyl ring, a pyridyl ring or a pyrimidinyl ring, and wherein said ring is unsubstituted or substituted at one or more atoms of C with one or more R¹⁰ substituents, wherein each R¹⁰ substituent is independently selected from the group consisting of: H, F, Cl, Br, cyano, -C(O)NR¹¹R¹², -OR¹¹, -C(O)OR¹¹, -NR¹¹R¹² and -SO₂NR¹¹R¹²; wherein R¹¹ and R¹² are each independently selected from the group consisting of: H, methyl, ethyl, -R¹³-C(O)OR¹⁴, -R¹³-OR¹⁴ and -R¹³-NR¹⁴R¹⁵, wherein R¹³ is a group -(CH₂)*n*- where *n* is 1 or 2, and wherein R¹⁴ and R¹⁵ are each independently H, methyl or ethyl; or wherein R¹¹ and R¹² when taken with the N atom to which they are both attached, complete a N-methyl-piperazinyl ring or a morpholyl ring.

According to the present invention R⁴, R⁵, R⁶ and R⁷ are each independently selected from the group consisting of: H, halogen, C₁-C₆ alkyl, C₁-C₆ haloalkyl, -OR¹¹, -OC(O)R¹¹,nitro, cyano, -NR¹¹R¹², -NR¹¹SO₂R¹⁶, -SR¹¹, -SO₂R¹¹ and -SO₂NR¹¹R¹²; wherein R¹¹ and R¹² are each independently selected from the group consisting of: H, C₁-C₆ alkyl, -R¹³-C(O)OR¹⁴, -R¹³-OR¹⁴ and -R¹³-NR¹⁴R¹⁵, wherein R¹³ is a group -(CH₂)*n*- where *n* is 1, 2, 3 or 4, and wherein R¹⁴ and R¹⁵ are each independently H or C₁-C₆ alkyl; or wherein R¹¹ and R¹² when taken with the atom to which they are both attached, complete a 5- or 6-membered substituted or unsubstituted cycloalkyl or a 5- or 6-membered substituted or unsubstituted heterocycloalkyl group comprising at least one heteroatom selected from O, N or S; and wherein R¹⁶ is C₁-C₆ alkyl or C₁-C₆ haloalkyl.

Preferably R⁴ and R⁵ are each independently selected from the group consisting of: H, -OR¹¹, -OC(O)R¹¹, nitro, -NR¹¹R¹² and -NR¹¹SO₂R¹⁶; and R⁵ and R⁶ are each independently selected from the group consisting of: H, halogen, -NR¹¹R¹² and -SO₂NR¹¹R¹²; wherein R¹¹ and R¹² are each independently selected from the group consisting of: H, C₁-C₆ alkyl, -R¹³-C(O)OR¹⁴, -R¹³-OR¹⁴ and -R¹³-NR¹⁴R¹⁵, wherein R¹³ is a group -(CH₂)*n*- where *n* is 1, 2, 3 or 4, and wherein R¹⁴ and R¹⁵ are each independently H or C₁-C₆ alkyl; or wherein R¹¹ and R¹² when taken with the atom to which they are both attached, complete a 5- or 6-membered substituted or unsubstituted cycloalkyl or a 5- or 6-membered substituted or unsubstituted heterocycloalkyl group comprising at least one heteroatom selected from O, N or S; and wherein R¹⁶ is C₁-C₆ alkyl or C₁-C₆ haloalkyl.

More preferably, R⁴ and R⁵ are each independently selected from the group consisting of: H, -OR¹¹, -OC(O)R¹¹, nitro, -NR¹¹R¹² and -NR¹¹SO₂R¹⁶; and R⁵ and R⁶ are each independently selected from the group consisting of: H, halogen, -NR¹¹R¹² and -SO₂NR¹¹R¹²; wherein R¹¹ and R¹² are each independently selected from the group consisting of: H, C₁-C₆ alkyl, -R¹³-C(O)OR¹⁴, -R¹³-OR¹⁴ and -R¹³-NR¹⁴R¹⁵, wherein R¹³ is a group -(CH₂)*n*- where *n* is 1 or 2, and wherein R¹⁴ and R¹⁵ are each independently H or C₁-C₆ alkyl; or wherein R¹¹ and R¹² when taken with the atom to which they are both attached, complete a 6-membered substituted or unsubstituted heterocycloalkyl group comprising at least one heteroatom selected from O or N. More preferably, the 6-membered heterocycloalkyl group comprises one or two heteroatoms selected from O or N and at least one C₁-C₆ alkyl substitution; and wherein R¹⁶ is C₁-C₆ alkyl, and more preferably R¹⁶ is methyl, ethyl or -CF₃.

Even more preferably R⁴ and R⁵ are each independently selected from the group consisting of: H, -OR¹¹, -OC(O)R¹¹, nitro, -NR¹¹R¹² and -NR¹¹SO₂R¹⁶; and R⁵ and R⁶ are each independently selected from the group consisting of: H, F, Cl, Br, -NR¹¹R¹² and -SO₂NR¹¹R¹²; wherein R¹¹ and R¹² are each independently selected from the group consisting of: H, methyl, ethyl, -R¹³-C(O)OR¹⁴, -R¹³-OR¹⁴ and -R¹³-NR¹⁴R¹⁵, wherein R¹³ is a group -(CH₂)*n*- where *n* is 1 or 2, and wherein R¹⁴ and R¹⁵ are each independently H, methyl or ethyl; or wherein R¹¹ and R¹² when taken with the N atom to which they are both attached, complete a N-methyl-piperazinyl ring or a morpholyl ring; and wherein R¹⁶ is methyl.

A particular preferred embodiment of the present invention refers to a compound of formula (I) according to the present invention, or a pharmaceutically acceptable salt thereof: 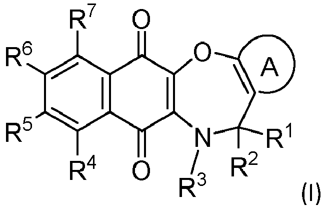 wherein
A is a phenyl ring unsubstituted or substituted with one or more R⁸ substituents, wherein each R⁸ substituent is independently selected from the group consisting of: H, halogen, -CF₃, -OR⁹ and -C(O)OR⁹, and wherein R⁹ is selected from the group consisting of: H and C₁-C₆ alkyl;
R¹ and R² are both H;
R³ is a phenyl ring, a pyridyl ring or a pyrimidinyl ring, and wherein said ring is unsubstituted or substituted at one or more atoms of C with one or more R¹⁰ substituents, wherein each R¹⁰ substituent is independently selected from the group consisting of: H, halogen, -CF₃, cyano, -C(O)NR¹¹R¹², -OR¹¹, -C(O)OR¹¹, -NR¹¹R¹² and -SO₂NR¹¹R¹²;
R⁴ and R⁵ are each independently selected from the group consisting of: H, -OR¹¹, -OC(O)R¹¹, nitro, -NR¹¹R¹² and -NR¹¹SO₂R¹⁶; and R⁵ and R⁶ are each independently selected from the group consisting of: H, halogen, -NR¹¹R¹² and -SO₂NR¹¹R¹²;
wherein R¹¹ and R¹² are each independently selected from the group consisting of: H, C1-C6 alkyl, -R¹³-C(O)OR¹⁴, -R¹³-OR¹⁴ and -R¹³-NR¹⁴R¹⁵, wherein R¹³ is a group -(CH₂)*n-*where *n* is 1 or 2, and wherein R¹⁴ and R¹⁵ are each independently H or C₁-C₆ alkyl; or wherein R¹¹ and R¹² when taken with the atom to which they are both attached, complete a 6-membered substituted or unsubstituted heterocycloalkyl group comprising at least one heteroatom selected from O or N; and wherein R¹⁶ is methyl, ethyl or -CF₃.

An even more preferred embodiment of present invention refers to a compound of formula (I) according to the present invention, or a pharmaceutically acceptable salt thereof: 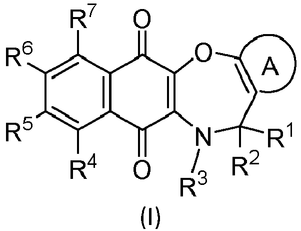 wherein
A is a phenyl ring unsubstituted or substituted with one or more R⁸ substituents, wherein each R⁸ substituent is independently selected from the group consisting of: H, F, Cl, Br, -OR⁹ and -C(O)OR⁹, and wherein R⁹ is H, methyl or ethyl;
R¹ and R² are both H;
R³ is a phenyl ring, a pyridine ring or a pyrimidine ring, and wherein said ring is unsubstituted or substituted at one or more atoms of C with one or more R¹⁰ substituents, wherein each R¹⁰ substituent is independently selected from the group consisting of: H, F, Cl, Br, cyano, -C(O)NR¹¹R¹², -OR¹¹, -C(O)OR¹¹, -NR¹¹R¹² and -SO₂NR¹¹R¹²;
R⁴ and R⁵ are each independently selected from the group consisting of: H, -OR¹¹, -OC(O)R¹¹, nitro, -NR¹¹R¹² and -NR¹¹SO₂R¹⁶; and R⁵ and R⁶ are each independently selected from the group consisting of: H, F, Cl, Br, -NR¹¹R¹² and -SO₂NR¹¹R¹²;
wherein R¹¹ and R¹² are each independently selected from the group consisting of: H, methyl, ethyl, -R¹³-C(O)OR¹⁴, -R¹³-OR¹⁴ and -R¹³-NR¹⁴R¹⁵, wherein R¹³ is a group -(CH₂)*n*- where *n* is 1 or 2, and wherein R¹⁴ and R¹⁵ are each independently H, methyl or ethyl; or wherein R¹¹ and R¹² when taken with the N atom to which they are both attached, complete a N-methyl-piperazinyl ring or a morpholyl ring; and wherein R¹⁶ is methyl.

Also within the scope of the present invention are compounds of the invention that are attached to an oligomeric or polymeric framework (e.g., polylysine, dextran, hydroxyethyl starch and a like).

The compounds of formula (I) may also be functionalized to afford compounds having a water-solubility that is enhanced relative to analogous compounds that are not similarly functionalized. Methods of enhancing the water-solubility of organic compounds are known in the art. Such methods include, but are not limited to, functionalizing an organic nucleus with a permanently charged moiety, e.g. quaternary ammonium, or a group that is charged at a physiologically relevant pH, e.g. carboxylic acid, amine. Other methods include, appending to the organic nucleus hydroxyl- or amine-containing groups, e.g. alcohols, polyols, polyethers and the like. Representative examples include, but are not limited to, polylysine, polyethyleneimine, poly(ethyleneglycol) and poly(propyleneglycol). Suitable functionalization chemistries and stragies for these compounds are known in the art. See, for example, Dunn, R.L., et al., Eds. POLYMERIC DRUGS AND DRUG DELIVERY SYSTEMS, ACS Symposium Series Vol. 469, American Chemical Society, Washington, D.C. 1991 (ref. 28).

The compounds of formula (I) according to present invention may also form prodrugs. For example, compounds of formula (I) having free amino, amido, hydroxy, phenolic, or carboxylic acid groups can be converted into prodrugs. Prodrugs of the compounds described herein are those compounds that readily undergo chemical changes under physiological conditions to provide the compounds of the present invention. Said prodrugs are, for example, ester prodrugs or amide prodrugs. Ester prodrugs include derivatives of the hereinbefore described compounds, wherein the parent compound is modified by converting an existing acid or alcohol moiety into an ester. Examples of esters include, but are not limited to, (C₁-C₆)alkyl esters of carboxylic acid moieties, or (C₁-C₆)alkanoyl esters of alcohol moieties. A (C₁-C₆)alkyl ester of a carboxylic acid moiety may be formed by condensing, for example, a (C₁-C₆)alkyl alcohol such as ethanol, with said carboxylic acid moiety. A (C₁-C₆)alkanoyl ester of an alcohol moiety may be formed by condensing, for example a (C₁-C₆)carboxylic acid such as acetic acid, with said alcohol moiety, Amide prodrugs include derivatives of the hereinbefore described compounds, wherein the parent compound is modified by converting an existing acid or amino moiety into an amide. Examples of amides include, but are not limited to, (C₁-C₆)alkyl amides of carboxylic acid moieties, or (C₁-C₆)alkanoyl amides of amine moieties. A (C₁-C₆)alkyl amide of a carboxylic acid moiety may be formed by condensing, for example, a (C₁-C₆)alkyl amine such as ethyl amine, with said carboxylic acid moiety. A (C₁-C₆)alkanoyl amide of an amine moiety may be formed by condensing, for example a (C₁-C₆)carboxylic acid such as acetic acid, with said amine moiety,

Also disclosed herein are metabolites of the compounds of formula (I), according to present invention, which are compounds formed *in vivo* upon administration of the drug. Some examples of metabolites in accordance with the invention include:
(i) where the compound of formula I contains a methyl group, an hydroxymethyl derivative thereof

   (-CH₃→ -CH₂OH);
(ii) where the compound of formula I contains an alkoxy group, a hydroxyl derivative thereof

   (-OR →-OH);
(iii) where the compound of formula I contains a tertiary amino group,a secondary amino derivative thereof (-NR¹R²→ -NHR¹ or -NHR²);
(iv) where the compound of formula I contains a secondary amino group, a primary amino derivative thereof (-NHR¹→ -NH₂); and
(v) where the compound of formula I contains a phenyl group, a phenol derivative thereof

   (-Ph→ -PhOH).

The compounds of formula (I) containing one or more asymmetric carbon atoms can exist as two or more stereoisomers. Where a compound of formula (I) contains an alkene or alkenylene group, geometric cis/trans (E/Z) isomers are possible. Where structural isomers are interconvertible via a low energy barrier, tautomeric isomerism ("tautomerism") can occur. This can take the form of proton tautomerism in compounds of formula (I) containing, for example, an imino, keto, or oxime group, or so-called valence tautomerismin compounds which contain an aromatic moiety. It follows that a single compound may exhibit more than one type of isomerism. Thus, the compounds of the present invention also include all stereoisomers, geometric isomers and tautomeric forms of the compounds of formula (I), as hereinbefore defined, including compounds that exhibit more than one type of isomerism, and mixtures of one or more thereof. Also included are acid or base salts wherein the counter ion is optically active, for example, d-lactate or I-lysine, or racemic, for example, dl-tartrate or dl-arginine.

Cis/trans isomers may be separated by conventional techniques well known to those skilled in the art, for example, chromatography or fractional crystallization.

Where a compound possesses a stable chiral center the compound can be used as a purified enantiomer or diastereomer, or as a mixture of any ratio of stereoisomers. It is however preferred that the mixture comprises at least 70%, 80%, 90%, 95%, 97.5% or 99% of the preferred isomer. Conventional techniques for the preparation/isolation of individual enantiomers include chiral synthesis from a suitable optically pure precursor or enantiomeric enrichment by resolution of the racemate (or the racemate of a salt or derivative) using for example, chiral high pressure liquid chromatography (HPLC), namely using HPLC with an asymmetric resin and a mobile phase. Alternatively, the racemate (or a racemate precursor) may be reacted with a suitable optically active compound, for example, an alcohol, or in the case where the compounds of formula I contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to those skilled in the art.

Also disclosed are pharmaceutically acceptable isotopically-labelled compounds of formula (I), as hereinbefore defined, wherein one or more atoms are replaced by atoms having the same atomic number, but an atomic mass or mass number different from the atomic mass or mass number which predominates in nature. Example of isotopes acceptable for inclusion in the compounds of the invention include isotopes of hydrogen such as ²H and ³H, carbons such as ¹¹C, ¹³C and ¹⁴C, chlorine such as ³⁶Cl, fluorine such as ¹⁸F, iodine such as ¹²³I and ¹²⁵I, nitrogen such as ¹³N and ¹⁵N, oxygen such as ¹⁵O, ¹⁷O and ¹⁸O, phosphorus such as ³²P, and sulphur such as ³⁵S. Certain isotopically-labelled compounds of formula I, for example those incorporating a radioactive isotope, are useful in drug and/or substrate tissue distribution studies. The radioactive isotopes tritium, i.e. ³H, and carbon-14, i.e. ¹⁴C are particularly useful for this purpose in view of their ease of incorporation and ready means of detection. Substitution with heavier isotopes such as deuterium i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C and ¹⁸F, ¹⁵O and ¹³N, can be useful in Positron Emission Topography (PET) studies for examining substrate receptor occupancy. Isotopically-labelled compounds of formula I can generally be prepared by conventional techniques known to those skilled in the art or by processes analogous to those described in the accompanying Examples and preparations section, using an appropriate isotopically-labelled reagent in place of the non-labelled reagent previously employed.

The compounds of formula (I) according to present invention reduce the cyto-protective functions of peroxiredoxins and are, accordingly inhibitors of one or more peroxiredoxins (PRDXs). As previously indicated in the background section, experimental evidence shows an association between alterations in the amount of two-cysteine PRDX isoform proteins and major human diseases such as hyper-proliferative diseases, as cancer, cardiovascular diseases, diabetes, bone loss, chronic kidney disease neurodegenerative diseases and immunological diseases, and therefore, inhibition of PRDXs has been identified as a useful therapeutic target for said diseases and, in particular, as a useful therapeutic target for cancer as seen in the examples of present application.

Accordingly, one embodiment of present invention refers to a pharmaceutical composition comprising at least one compound of formula (I), according to the present invention, or a pharmaceutically acceptable salt thereof, and at least one pharmaceutically acceptable excipient.

Another embodiment of present invention refers to an antibody-drug conjugate comprising a compound of formula (I) and a human or humanized monoclonal or polyclonal antibody.

For the purposes of present invention the term "antibody-drug conjugate" refers to the coupling of a compound, in particular a compound of formula (I) or a pharmaceutically acceptable salt thereof, according to present invention, with a human or humanized monoclonal or polyclonal antibody such as IgG1, IgG2 or IgG4. In an embodiment the compound of formula (I) and the human or humanized monoclonal or polyclonal antibody are linked by a cleavable or a non-cleavable linker, depending on whether or not the bond between the antibody and the compound of formula (I) may or may not be degraded or broken down by enzymes.

Yet another embodiment of present invention refers to a pharmaceutical composition comprising at least one antibody-drug conjugate comprising a compound of formula (I), according to the present invention, and at least one pharmaceutically acceptable excipient.

Additionally, present invention also refers to a compound of formula (I), or a pharmaceutically acceptable salt thereof, or to an antibody-drug conjugate comprising the same, according to the present invention, for use as a medicament. In particular, one embodiment of present invention refers to a compound of formula (I), or a pharmaceutically acceptable salt thereof, or to an antibody-drug conjugate comprising the same, according to the present invention, for use in the prevention and/or treatment of a disease or condition responsive or ameliorated by inhibition of one or more peroxiredoxins. Preferably, present invention refers to a compound of formula (I), or a pharmaceutically acceptable salt thereof, or to an antibody-drug conjugate comprising the same, according to the present invention, for use in the prevention and/or treatment of cancer.

Another embodiment disclosed in present invention refers to a pharmaceutical composition comprising at least one compound of formula (I), or a pharmaceutically acceptable salt thereof, or an antibody-drug conjugate comprising the same, according to the present invention, and at least one pharmaceutically acceptable excipient, for use as a medicament and, in particular, for use in the prevention and/or treatment of a disease or condition responsive or ameliorated by inhibition of one or more peroxiredoxins. Preferably, present invention refers to a pharmaceutical composition comprising at least one compound of formula (I), or a pharmaceutically acceptable salt thereof, or an antibody-drug conjugate comprising the same, according to the present invention, for use in the prevention and/or treatment of cancer.

Also described is the use of a compound of formula (I), or a pharmaceutically acceptable salt thereof, or an antibody-drug conjugate or a pharmaceutical composition comprising the same, according to the present invention, in the manufacturing of a medicament for preventing /and/or treating a disease or condition responsive or ameliorated by inhibition of one or more peroxiredoxins and, preferably, for prevention and/or treatment of cancer.

Additionally, described is a method for preventing and/or treating a disease or condition responsive or ameliorated by inhibition of one or more peroxiredoxins, wherein said method comprises administering an effective amount of at least one compound of formula (I), or a pharmaceutically acceptable salt thereof, or an antibody-drug conjugate or a pharmaceutical composition comprising the same, according to the present invention, to a subject in need thereof and, preferably, for prevention and/or treatment of cancer.

For the purposes of present invention, the term "disease or condition responsive or ameliorated by inhibition of one or more peroxiredoxins" refers to a disease or pathological disorder or condition which may be treated with inhibitors of one or more peroxiredoxins, i.e. a disease or pathological disorder or condition the treatment of which benefits or is ameliorated by inhibitors of one or more peroxiredoxins and, preferably, for prevention and/or treatment of cancer.

Preferably, the inhibitors of one or more peroxiredoxins are inhibitors of at least one peroxiredoxin selected from the group consisting of PRDX1, PRDX2 and PRDX3. Accordingly, a preferred embodiment refers to a disease or condition responsive or ameliorated by inhibition of one or more peroxiredoxins selected from the group consisting of PRDX1, PRDX2 and PRDX3.

For the purposes of present invention said condition responsive or ameliorated by inhibition of one or more peroxiredoxins is selected from the group consisting of: a hyper-proliferative disease, cancer, cardiovascular diseases, diabetes, bone loss, chronic kidney disease neurodegenerative diseases and immunological diseases.

Preferably, said condition responsive or ameliorated by inhibition of one or more peroxiredoxins is a cancer. More preferably, said cancer is a chemo/radio-resistant cancer or a metastatic cancer.

For the purposes of present invention, the term "chemo/radio-resistant tumour" or "chemo/radio-resistant cancer" refers to a cancer condition where a chemotherapeutic treatment, or radiotherapy, has not provided a remission of said cancer or tumour.

In a preferred embodiment, said cancer, or said chemo/radio-resistant cancer or said metastatic cancer, is selected from the group consisting of: sarcoma, breast cancer, prostate cancer, head and neck cancer, brain tumour, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.

In one embodiment, the pharmaceutical compositions disclosed comprise at least one additional active compound or therapeutic ingredient. Said additional active compound or therapeutic ingredient provides additive or synergistic biological activities. For the purposes of present description, the terms "active compound" or "therapeutic ingredient" should be taken as synonyms and mean a chemical or biological entity which exerts therapeutic effects when administered to human or animal beings. Said active compound or therapeutic ingredient exerts therapeutic effects when administered to human or animal beings, and it may be a cell therapy, a small molecule therapy, immunotherapy, radiotherapy, among others. Preferably said active compound or therapy is selected from the group consisting of chemotherapeutic, cell therapy and immunotherapeutic agents.

In a preferred embodiment said additional active compound or therapeutic agent is selected from the group consisting of a CAR-T cell therapy, a CAR-NK cell therapy, an antibody, a HIF pathway inhibitor and a chemotherapeutic agent. Said additional active compound or additional therapy may be administered at the same time, prior to, subsequently to, or at a different moment than the compound of formula (I) for use according to present invention. Preferably, said chemotherapeutic agent is selected from the group consisting of platinum-based antineoplastic agents, anti-mitotic chemotherapeutic agents, a poly adenosine diphosphate ribose polymerase (PARP) inhibitor, type I topoisomerase inhibitors, type II topoisomerase inhibitors, epothilones, cycloskeletal disruptors, alkylating agents, epothilones, histone deacetylase inhibitors, kinase inhibitors, antifolates, kinase inhibitors, peptide antibiotics, retinoids, vinca alkaloids and thymidylate synthase inhibitors. More preferably, the chemotherapeutic agent is selected from the group consisting of cyclophosphamide, ifosfamide, busulfan, temozolomide, mechlorethamine, chlorambucil, melphalan, dacarbazine, daunorubicin, doxorubicin, daunorubicin, epirubicin, idarubicin, mitoxantrone, valrubicin, paclitaxel, docetaxel, abraxane, taxotere, epothilone, vorinostat, romidepsin, irinotecan, topotecan, camptothecin, exatecan, lurtotecan, etoposide, teniposide, tafluposide, bortezomib, erlotinib, gefitinib, imatinib, vemurafenib, vismodegib, azacitadine, azathioprine, capecitabine, cytarabine, cladribine, fludarabine, doxifluridine, fluorouracil, gemcitabine, hydroxyurea, mercaptopurine, methotrexate, pemetrexed, tioguanine, bleomycin, actinomycin, carboplatin, cisplatin, oxaliplatin, tretinoin, alitretinoin, bexarotene, vinblastine, vincristine, vindesine and vinorelbine.

In another preferred embodiment said additional active ingredient is a HIF pathway inhibitor. Preferably said HIF pathway inhibitor is selected from apigenin, campothecin, topotecan, irinotecan, 2-methoxyestradiol, temsirolimus, echinomycin, romidepsin, geldanamycin, bortezomib, Vorinostat (SAHA), 2M2 NCD (panzem), 17-AAG (tanespimycin), 17-DMAG, PT2385, PT2977, EZN-2208, CRLX101 or any other compound inhibiting the HIF pathway.

In yet another preferred embodiment said additional active ingredient is a human or humanized monoclonal or polyclonal antibody.

Preferably said antibody is selected mogamulizumab, blinatumomab, ibritumomab, obinutuzumab, ofatumumab, rituximab, tositumomab, inotuzumab, moxetumomab, brentuximab, gemtuzumab, daratumumab, isatuximab, alemtuzumab, polatuzumab, cetuximab, necitumumab, nimotuzumab, panitumumab, catumaxomab, burosumab, dinutuximab, pertuzumab, trastuzumab, ertumaxomab, mepolizumab, siltuximab, enfortumab, etaracizumab, racotumomab, bevacizumab, denosumab, elotuzumab, olaratumab, ramucirumab, bermekimab, labetuzumab, pemtumomab, tacatuzumab, pembrolizumab, nivolumab, cemiplimab, atezolizumab, avelumab, durvalumab, ipilimumab or any other immunotherapeutic agent.

The compounds of formula (I) and pharmaceutical compositions comprising the same, for use according to present invention may also be administered together, prior to, or subsequently to an additional therapy. Preferably said additional therapy is radiotherapy, immunotherapy or chemotherapy.

In a preferred embodiment, said compound of formula (I), or said pharmaceutical composition for use according to present invention is administered together with, prior to or subsequently to a radiotherapeutic treatment, a chemotherapeutic treatment or an immunotherapeutic treatment.

Additionally, the compounds of formula (I) or pharmaceutically salts thereof, for use according to present invention may also be administered as an antibody-drug conjugate or administered as part of an antibody-drug conjugate, i.e., administered as an antibody-drug conjugate comprising said compounds of formula (I) or pharmaceutically salts thereof and a human or humanized monoclonal or polyclonal antibody.

The compounds of the present invention can be prepared and administered in a wide variety of oral, parenteral and topical dosage forms. Thus, the compounds of the present invention can be administered by injection, that is, intravenously, intramuscularly, intracutaneously, subcutaneously, intraduodenally, or intraperitoneally. Also, the compounds described herein can be administered by inhalation, for example, intranasally. Additionally, the compounds of the present invention can be administered transdermally.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration. A solid carrier can be one or more substances, which may also act as diluents, flavouring agents, binders, preservatives, tablet disintegrating agents, or an encapsulating material.

In powders and tablets, preferably containing from 5% to 80% of the active compound, suitable carriers are magnesium carbonate, magnesium stearate, talc, sugar, lactose, pectin, dextrin, starch, gelatine, tragacanth, methylcellulose, sodium carboxymethylcellulose, a low melting wax, cocoa butter, hydroxypropylmethylcellulose, polyethyleneglycol, and the like.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized moulds, allowed to cool, and thereby to solidify.

Liquid preparations include solutions, suspensions, and emulsions, for example, water or water/propylene glycol solutions. For peritoneal injection, liquid preparations can be formulated in solution in aqueous polyethylene glycol solution.

Aqueous solutions suitable for oral use can be prepared by dissolving the active component in water and adding suitable colorants, flavours, stabilizers, and thickening agents as desired. Aqueous suspensions suitable for oral use can be prepared by dispersing the finely divided active component in water with viscous material, such as natural or synthetic gums, resins, methylcellulose, sodium carboxymethylcellulose, and other well-known suspending agents.

Thus, a pharmaceutically acceptable carrier may comprise magnesium carbonate, magnesium stearate, talc, sugar, lactose, pectin, dextrin, starch, gelatine, tragacanth, methylcellulose, sodium carboxymethylcellulose, low melting fatty acid glycerides, cocoa butter, water, propylene glycol, natural or synthetic gums, resins, methylcellulose, and sodium carboxymethylcellulose.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 10000 mg, more typically 1.0 mg to 1000 mg, most typically 10 mg to 500 mg, according to the particular application and the potency of the active component.

The compounds of the present invention, or their pharmaceutically acceptable salts or prodrugs, are administered in a therapeutically effective amount which will vary depending upon a variety of factors including the activity of the specific compound employed, the metabolic stability and length of action of the compound, age, body weight, general health, sex, diet, mode and time of administration, rate of excretion, drug combination effects, the severity of the particular disease, and the patients side effect profile towards the compound. Determination of a therapeutically effective amount of a compound of the invention is well within the capabilities of those skilled in the art.

Patient doses for oral administration of the compounds described herein, which is the preferred mode of administration for prophylaxis treatment of an exemplary disease such as cancer, typically range from about 0.1 mg/day to about 10000 mg/day, more typically from about 1 mg/day to about 1000 mg/day, and most typically 1 mg/day to 500 mg/day. Stated in terms of patient body weight, typical dosages range from about 0.01 to about 150 mg/kg/day, more typically from about 0.1 to about 15 mg/kg/day, and most typically from about 0.5 to about 10 mg/kg/day.

For other modes of administration, dosage amount and interval, can be adjusted individually to provide plasma levels of the administered compound, that are effective for the particular clinical indication being treated.

The method by which the compounds of the present invention are prepared is not critical; compounds of the invention, including salts and prodrugs thereof, can be prepared using known organic synthesis techniques and can be synthesized according to any of numerous possible synthetic routes.

The reactions for preparing compounds of the invention can be carried out in suitable solvents which can be readily selected by one skilled in the art of organic synthesis. A given reaction can be carried out in one solvent or a mixture of more than one solvent and at temperatures that can range from the solvent's freezing temperature to the solvent's boiling temperature. In cases where sealed-tube assisted heating is used then the solvent temperature may exceed the boiling temperature of the solvent.

Preparation of compounds of the invention can involve protection and deprotection of various chemical groups. The need for protection and deprotection, and the selection of appropriate protecting groups, can be readily determined by one skilled in the art. The chemistry of protecting groups can be found, for example, in T. W. Greene and P. G.M. Wuts, "Protective Groups in Organic Synthesis", 3rd Ed., Wiley & Sons, Inc, New York (1999) (ref. 29), which is incorporated herein by reference in its entirety. Reactions can be monitored according to any suitable method known in the art. For example, product formation can be monitored by chromatographic methods such as thin layer chromatography (TLC) or high-performance liquid chromatography (HPLC), or by spectroscopic means, such as nuclear magnetic resonance spectroscopy (e.g., ¹H, ¹³C or ¹⁹F), infrared spectroscopy, spectrophotometry (e.g., UV-visible) or mass spectroscopy.

In an exemplary reaction pathway, fused-oxazepine compounds of the invention can be prepared by reacting an appropriately substituted 2-(aminomethyl)phenol (c) with an appropriately substituted 3,4-dihalo-naphthaquinone (d) following the method outlined in the general synthetic scheme A:

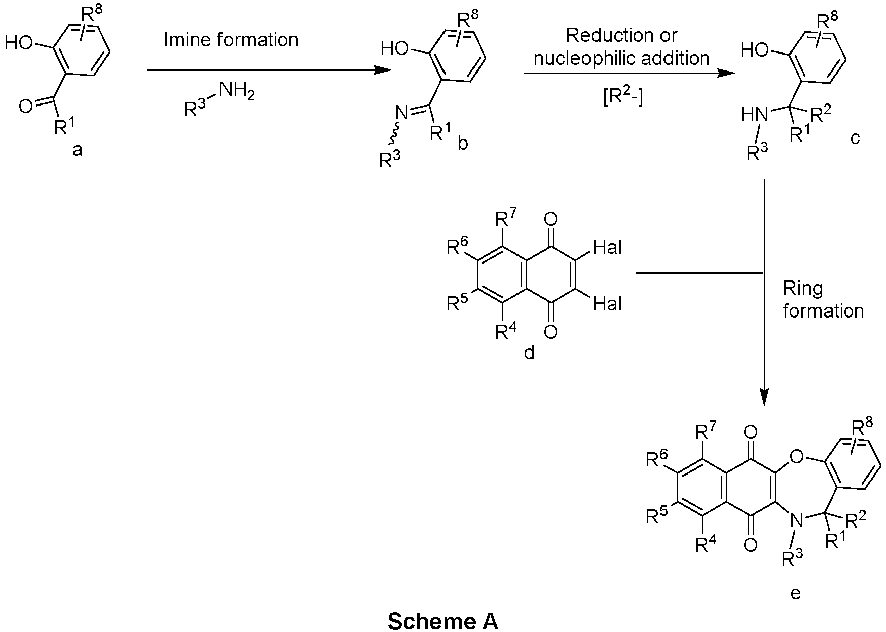

Substituted salicylic aldehydes, or ketones, (a) are transformed into the imine intermediates (b) under standard conditions. Addition of hydride or a carbanion to (b) generates the substituted 2-(aminomethyl)phenol (c) (see example 1, compounds c1 to c27 of the present disclosure). The desired 3,4-dihalo-naphthaquinone intermediate (d) can be prepared by reacting 3,4-dihalofuran-2,5-dione with substituted benzenes in a Friedal-Craft type reaction (see example 3, compounds d1 to d11 of the present disclosure). Literature examples for the preparation of these types of intermediates (d) are known from the art. Reaction of (c) and (d), respectively an amine and/or phenol acting as a nucleophile with a vinylogous acid chloride in a basic media, produces the desired fused-oxazepines (e). Compounds of the type (a) in scheme A, including substituted salicylic aldehydes, substituted 1-(2-hydroxyphenyl)ketones, and the like, are commercially available or can be prepared using known literature procedures. Literature examples for the preparation of compounds of the type (a) include Brühne, F.; Wright, E. (2005), "Benzaldehyde", Ullmann's Encyclopedia of Industrial Chemistry, Weinheim: Wiley-VCH (ref. 30) and Trond Vidar Hansen; Lars Skattebøl (2005), Ortho-Formylation of Phenols; Preparation of 3-Bromosalicylaldehyde, Org. Synth. 82:64 (ref. 31). The desired 3,4-dihalo-naphthaquinone derivatives, designated as component (d) in scheme A, were either commercially available or were prepared following standard chemical transformation techniques as those indicated in the examples included in the present disclosure.

In another exemplary reaction pathway, fused-oxazepine compounds (j) of the invention having formula (I) can be prepared by reacting an appropriately substituted 2-(aminomethyl)phenol bearing a heteroaryl group (i) with an appropriately substituted 3,4-dihalo-naphthaquinone (d) following the method outlined in general synthetic **scheme B**: 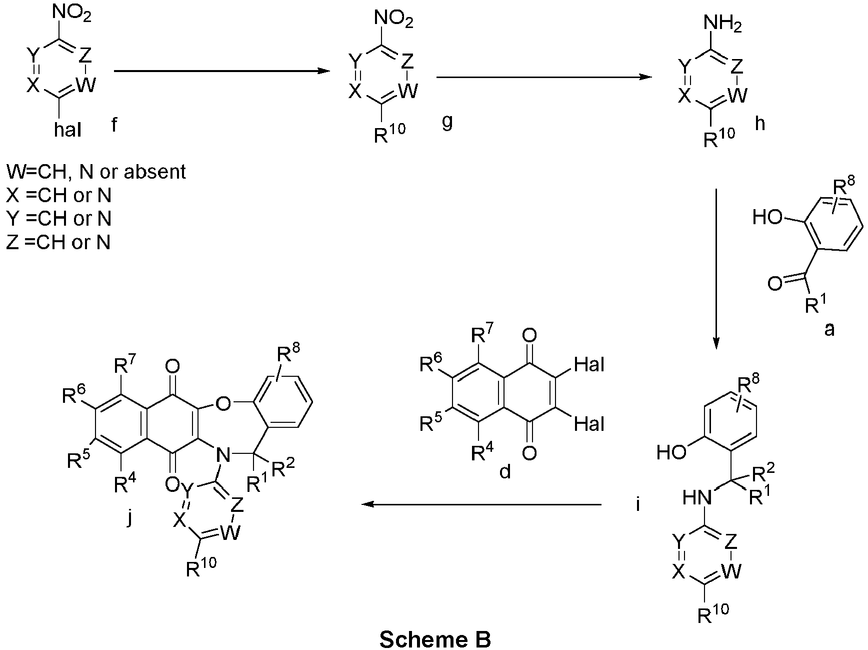 2-(Aminomethyl)phenol intermediates bearing a heteroaryl group (i) can be prepared (see example 2, compounds i1 to i10 of the present disclosure) by aromatic nucleophilic substitution of a halide group in compounds of type (f), followed by reduction of the nitro group and finally a reductive animation reaction between compounds of type (h) and (a). The oxazepines (j), of scheme B, and (e), of scheme A, were prepared reacting an appropriately substituted 2-(aminomethyl)phenol (c) or (i) with an appropriately substituted 3,4-dihalo-naphthaquinone (d).

### EXAMPLES

The following examples are offered to illustrate, but not to limit, the claimed invention.

According to, either the general synthetic schemes A or the general synthetic scheme B, the following oxazepines of formula (I), included in the following **table 1** (ID#1 to 111), below, were prepared:

| **ID #** | **Structure** | **ID #** | **Structure** | **ID #** | **Structure** |
|---|---|---|---|---|---|
| 1 | 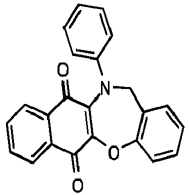 | 14 | 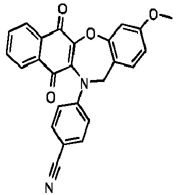 | 27 | 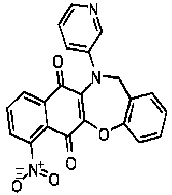 |
| 2 | 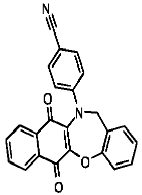 | 15 | 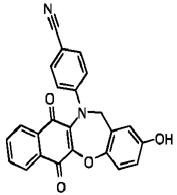 | 28 | 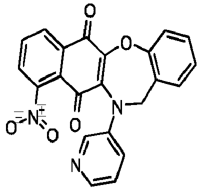 |
| 3 | 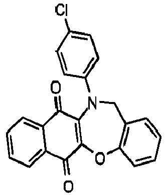 | 16 | 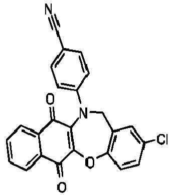 | 29 | 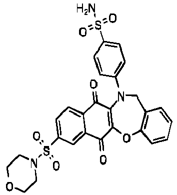 |
| 4 | 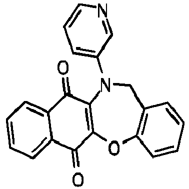 | 17 | 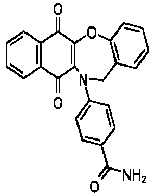 | 30 | 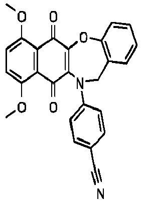 |
| 5 | 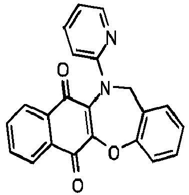 | 18 | 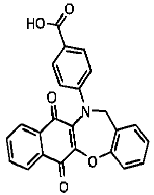 | 31 | 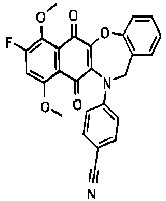 |
| 6 | 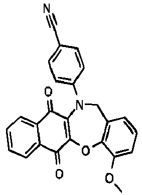 | 19 | 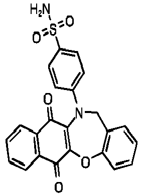 | 32 | 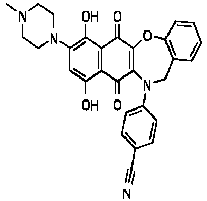 |
| 7 | 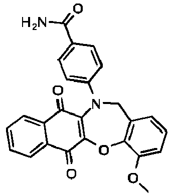 | 20 | 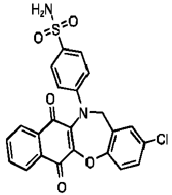 | 33 | 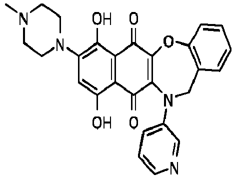 |
| 8 | 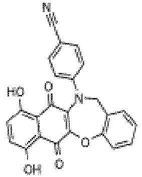 | 21 | 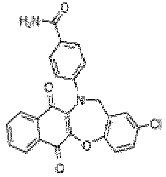 | 34 | 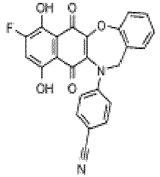 |
| 9 | 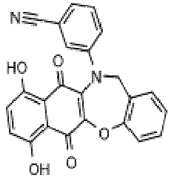 | 22 | 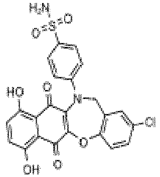 | 35 | 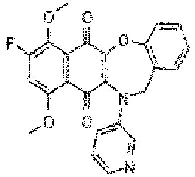 |
| 10 | 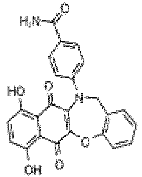 | 23 | 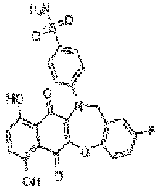 | 36 | 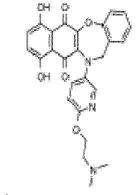 |
| 11 | 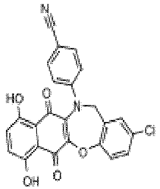 | 24 | 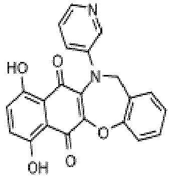 | 37 | 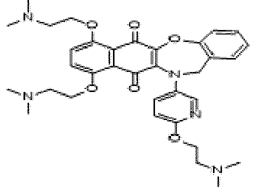 |
| 12 | 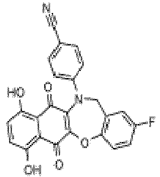 | 25 | 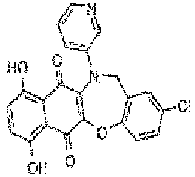 | 38 | 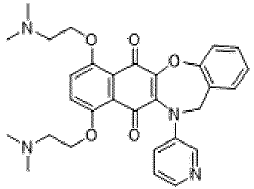 |
| 13 | 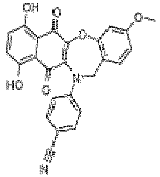 | 26 | 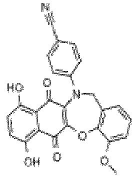 | 39 | 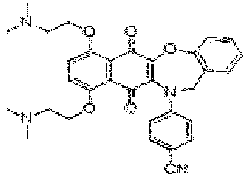 |
| 40 | 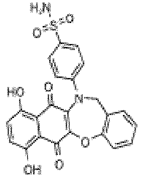 | 54 | 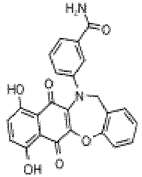 | 68 | 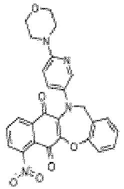 |
| 41 | 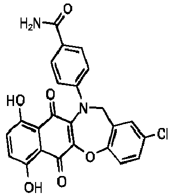 | 55 | 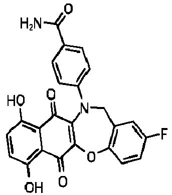 | 69 | 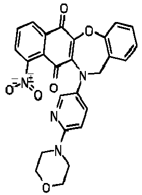 |
| 42 | 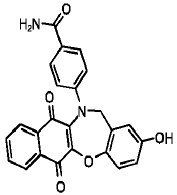 | 56 | 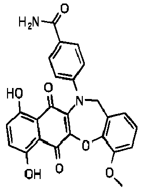 | 70 | 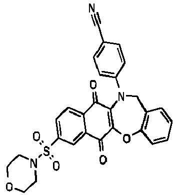 |
| 43 | 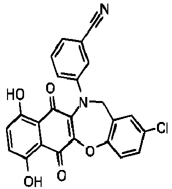 | 57 | 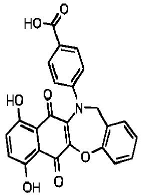 | 71 | 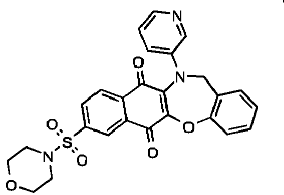 |
| 44 | 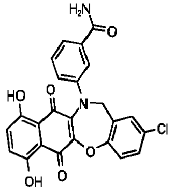 | 58 | 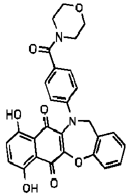 | 72 | 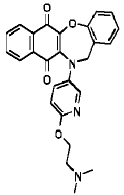 |
| 45 | 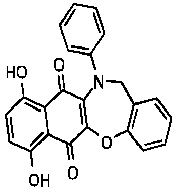 | 59 | 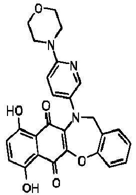 | 73 | 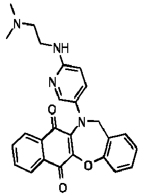 |
| 46 | 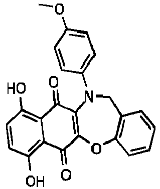 | 60 | 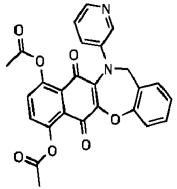 | 74 | 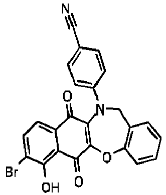 |
| 47 | 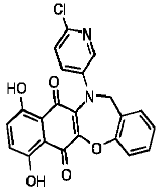 | 61 | 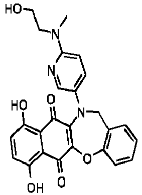 | 75 | 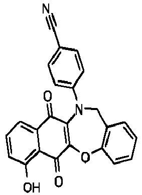 |
| 48 | 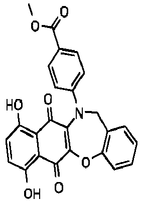 | 62 | 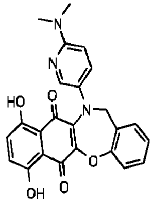 | 76 | 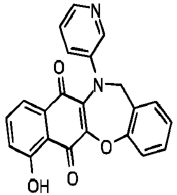 |
| 49 | 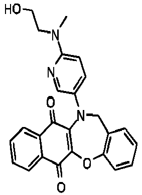 | 63 | 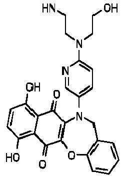 | 77 | 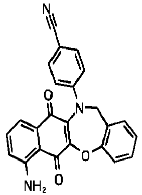 |
| 50 | 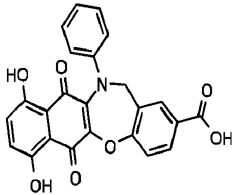 | 64 | 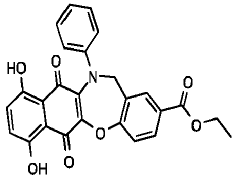 | 78 | 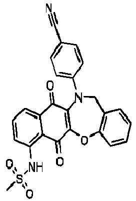 |
| 51 | 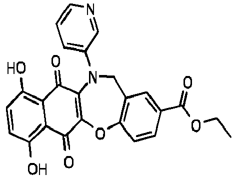 | 65 | 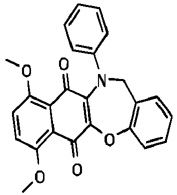 | 79 | 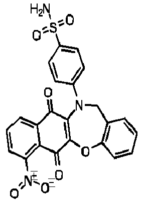 |
| 52 | 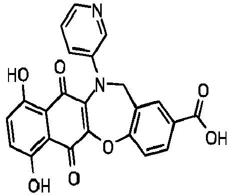 | 66 | 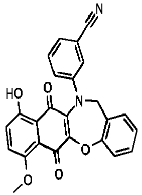 | 80 | 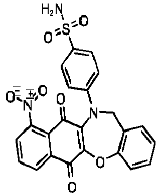 |
| 53 | 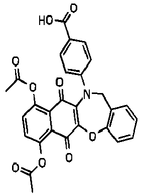 | 67 | 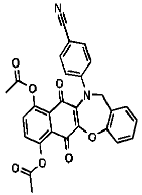 | 81 | 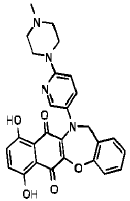 |
| 82 | 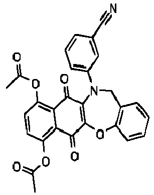 | 92 | 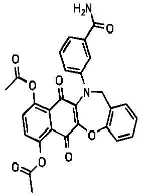 | 102 | 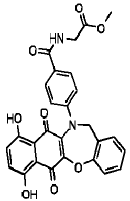 |
| 83 | 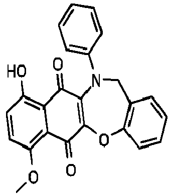 | 93 | 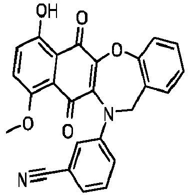 | 103 | 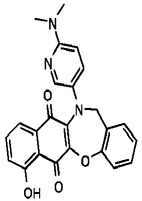 |
| 84 | 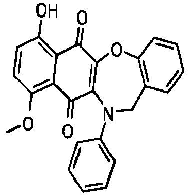 | 94 | 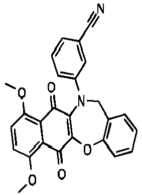 | 104 | 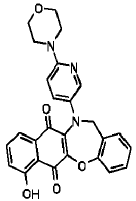 |
| 85 | 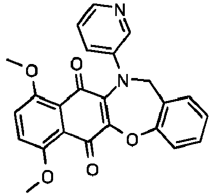 | 95 | 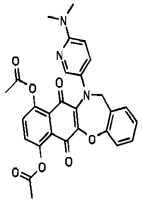 | 105 | 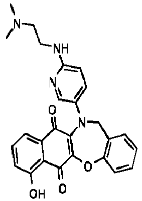 |
| 86 | 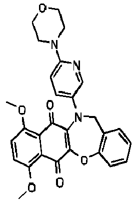 | 96 | 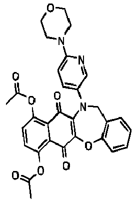 | 106 | 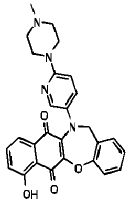 |
| 87 | 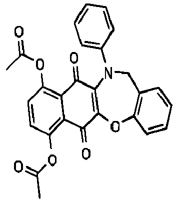 | 97 | 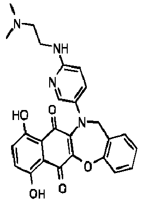 | 107 | 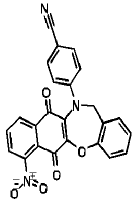 |
| 88 | 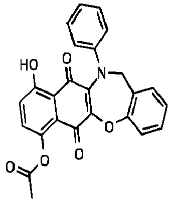 | 98 | 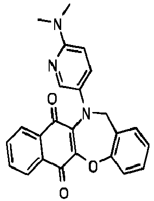 | 108 | 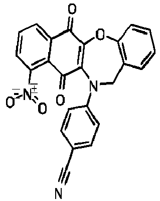 |
| 89 | 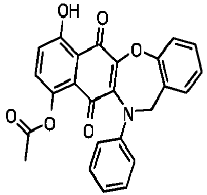 | 99 | 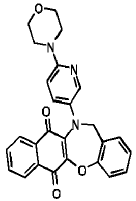 | 109 | 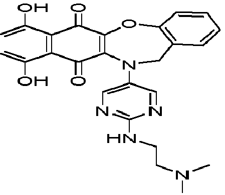 |
| 90 | 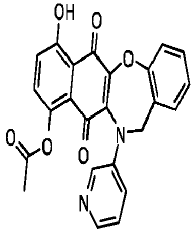 | 100 | 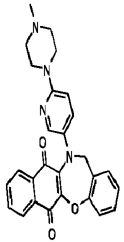 | 110 | 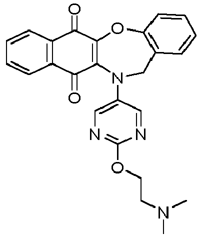 |
| 91 | 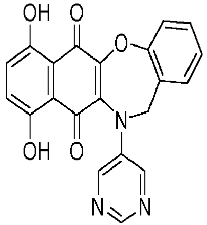 | 101 | 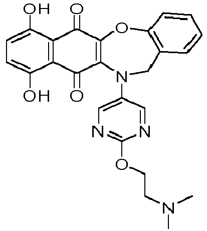 | 111 | 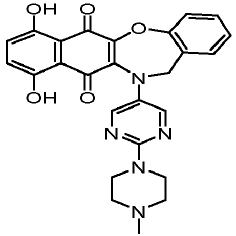 |

In the examples below, unless otherwise stated, temperatures are given in degrees Celsius (°C); operations were carried out at room, or ambient temperature (typically a range from 15-25°C); evaporation of solvent was carried out using a rotary evaporator under reduced pressure (typically 4-30mmHg/ with a bath temperature of up to 60°C; and the following conventional abbreviations are also used: mp (melting point), L (liter(s)), mL (millitres), mmol (millimoles), g (grams), mg (milligrams), min (minutes), and h (hours).

### EXAMPLE 1: Synthesis of substituted 2-((phenylamino)methyl)phenols (c) according to general scheme A.

### 1.1. Preparation of 4-((2-hydroxybenzyl)amino)benzonitrile (1A)

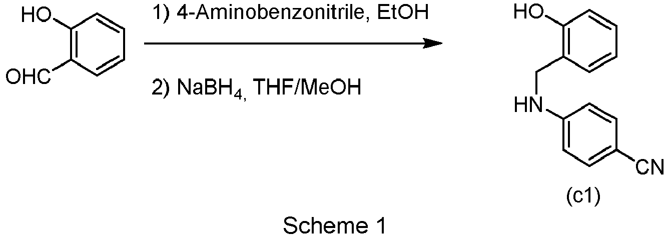

According to scheme 1 a mixture of salicylic aldehyde (400 mg, 3.3 mmol) and 4-aminobenzonitrile (389 mg, 3.3 mmol) in ethanol (5 mL) was heated at reflux overnight. On cooling to room temperature the orange solid was collected by filtration and washed with cold ethanol (5 mL). The resulting solid was dissolved in THF (5 mL). Methanol (1 mL) was added followed by the portionwise addition of sodium borohydride (60 mg, 1.6 mmol). After 30 min water (4 mL) was added. The organic solvents were removed under reduced pressure. The aqueous residue was extracted with ethyl acetate (30 mL). The organic phase was then washed with brine (10 mL), dried (MgSO₄), filtered and the solvent removed under reduced pressure to afford an off-white solid. Trituration with hot hexanes (10 mL) and collection by filtration gave the desired product as a white solid (538 mg, 73%).

### 1.2. Preparation of 4-((2-hydroxybenzyl)amino)benzenesulfonamide (c2)

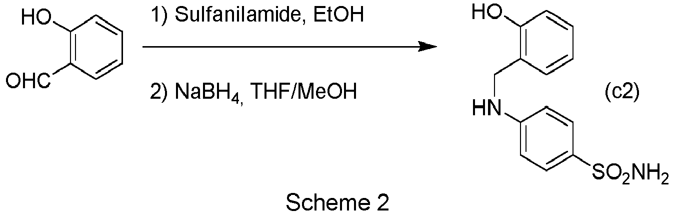

According to scheme 2 a mixture of salicylic aldehyde (1.22 g, 0.01 mol) and sulfanilamide (1.72 g, 0.01 mol) in ethanol (80 mL) was heated at reflux overnight. On cooling to room temperature the orange solid was collected by filtration and washed with cold ethanol (5 mL). The resulting solid was dissolved in THF (20 mL). Methanol (4 mL) was added followed by the portionwise addition of sodium borohydride (190 mg, 5 mmol). After 1h water (40 mL) was added. The organic solvents were removed under reduced pressure. The off-white solid was collected by filtration. The solid was sequentially triturated with hot hexanes (2 × 25 mL) and hot EtOAc (3 × 10 mL). The crude product was dried *in vacuo* at 65 °C and used without further purification (white solid, 645 mg, 23%).

### 1.3. Preparation of 4-((5-chloro-2-hydroxybenzyl)amino)benzenesulfonamide (c3)

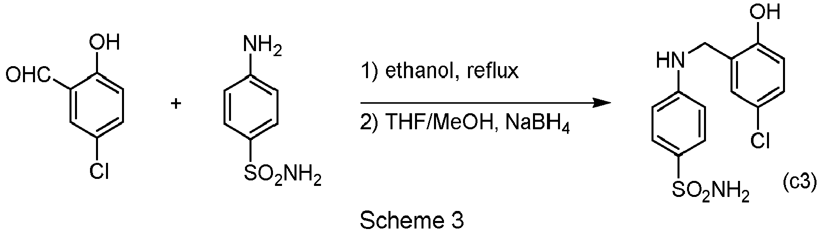

According to scheme 3 a mixture of 5-chloro-2-hydroxybenzaldehyde (417 mg, 2.67 mmol) and sulfanilamide (307 mg, 1.77 mmol) in ethanol (10 mL) was heated at reflux overnight. On cooling to room temperature the orange solid was collected by filtration and washed with cold ethanol (5 mL) and hot water (2 × 10 ml). After drying *in vacuo* at 65°C the resulting solid was dissolved in THF (20 mL). Methanol (4 mL) was added followed by the portion-wise addition of sodium borohydride (34 mg, 0.9 mmol). After 1h water (40 mL) was added. The organic solvents were removed under reduced pressure. The off-white solid was collected by filtration. The solid was sequentially triturated with hot hexanes (2 × 25 mL) and hot EtOAc (3 × 10 mL). The crude product was dried *in vacuo* at 65 °C and used without further purification (white solid, 321 mg, 58%).

### 1.4 Preparation of 3-((2-hydroxybenzyl)amino)benzonitrile (c4)

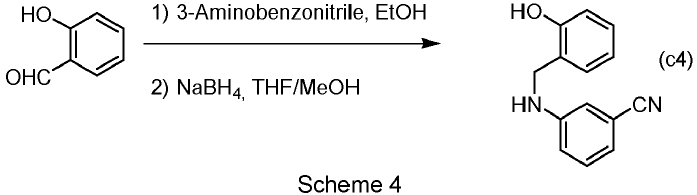

According to scheme 4 a mixture of salicylic aldehyde (517 mg, 4.2 mmol) and 3-aminobenzonitrile (500 mg, 4.2 mmol) in ethanol (10 mL) was heated at reflux overnight. On cooling to room temperature the orange solid was collected by filtration and washed with cold ethanol (10 mL). The resulting solid was dissolved in THF (10 mL). Methanol (2 mL) was added followed by the portionwise addition of sodium borohydride (80 mg, 2.1 mmol). After 30 min water (10 mL) was added. The organic solvents were removed under reduced pressure. The precipitate was collected by filtration and dried *in vacuo* at 65 °C. Trituration with hot hexanes (10 mL) gave the desired product as a white solid (814 mg, 87%).

### 1.5. Preparation of 4-((5-chloro-2-hydroxybenzyl)amino)benzonitrile (c5)

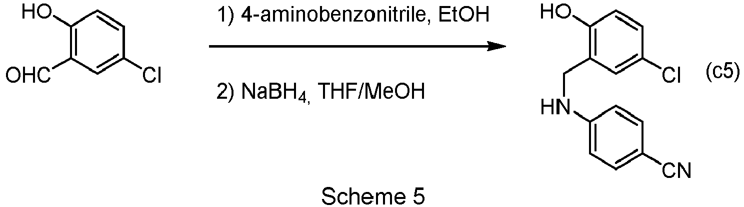

According to scheme 5 a mixture of 5-chloro-2-hydroxybenzaldehyde (500 mg, 3.2 mmol) and 4-aminobenzonitrile (375 mg, 3.2 mmol) in ethanol (5 mL) was heated at reflux overnight. On cooling to room temperature the orange solid was collected by filtration and washed with cold ethanol (10 mL). The resulting solid was dissolved in THF (8 mL). Methanol (1 mL) was added followed by the portionwise addition of sodium borohydride (61 mg, 1.6 mmol). After 30 min water (10 mL) was added. The organic solvents were removed under reduced pressure. The residue was extracted with EtOAc (2 × 10mL), washed with brine (10 mL), dried (MgSO₄), filtered and the solvent removed under reduced pressure to afford a colourless oil. Trituration with hot hexanes (10 mL) gave the desired product as a white solid (518 mg, 68%).

### 1.6. Preparation of 2-(((6-chloropyridin-3-yl)amino)methyl)phenol (c6)

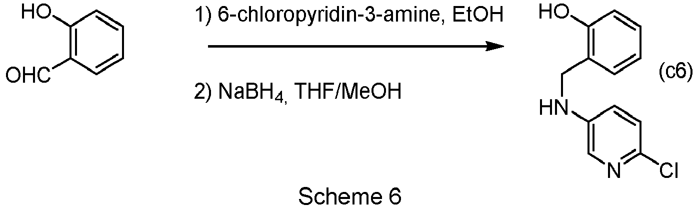

According to scheme 6 a mixture of salicylic aldehyde (244 mg, 2.0 mmol) and 6-chloropyridin-3-amine (257 mg, 2.0 mmol) in ethanol (5 mL) was heated at reflux overnight. On cooling to room temperature the solid was collected by filtration and washed with cold ethanol (10 mL). The resulting solid was dissolved in THF (8 mL). Methanol (1 mL) was added followed by the portionwise addition of sodium borohydride (38 mg, 1 mmol). After 30 min water (10 mL) was added. The organic solvents were removed under reduced pressure. The residue was extracted with EtOAc (2 × 10mL), washed with brine (10 mL), dried (MgSO₄), filtered and the solvent removed under reduced pressure to afford a colourless oil. Trituration with hot hexanes (10 mL) gave the desired product as a white solid (321 mg, 69%).

### 1.7. Preparation of 2-((pyridin-3-ylamino)methyl)phenol (c7)

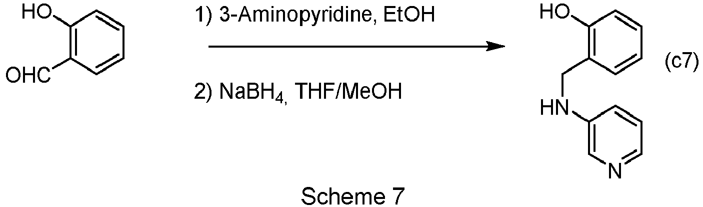

According to scheme 7 a mixture of salicylic aldehyde (366 mg, 3.0 mmol) and 3-aminopyridine (282 mg, 3.0 mmol) in ethanol (4 mL) was heated at reflux overnight. On cooling to room temperature the solid was collected by filtration and washed with cold ethanol (10 mL). The resulting solid was dissolved in THF (5 mL). Methanol (1 mL) was added followed by the portionwise addition of sodium borohydride (57 mg, 1.5 mmol). After 30 min water (10 mL) was added. The organic solvents were removed under reduced pressure. The residue was extracted with EtOAc (2 × 10mL), washed with brine (10 mL), dried (MgSO₄), filtered and the solvent removed under reduced pressure to afford a colourless oil. Trituration with hot hexanes (10 mL) gave the desired product as a white solid (442 mg, 74%).

### 1.8. Preparation of 4-((2-hydroxy-3-methoxybenzyl)amino)benzonitrile (c8)

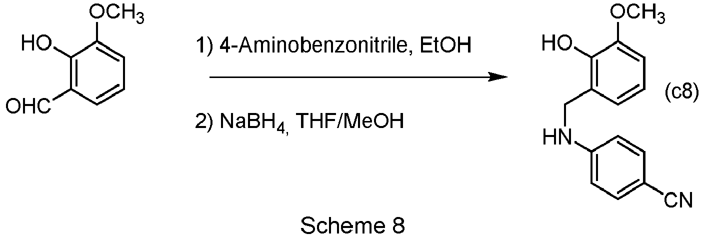

According to scheme 8 a mixture of 2-hydroxy-3-methoxybenzaldehyde (200 mg, 1.3 mmol) and 4-aminobenzonitrile (155 mg, 1.3 mmol) in ethanol (5 mL) was heated at reflux overnight. On cooling to room temperature the solid was collected by filtration and washed with cold ethanol (10 mL). The resulting solid was dissolved in THF (5 mL). Methanol (1 mL) was added followed by the portionwise addition of sodium borohydride (38 mg, 1.0 mmol). After 30 min water (10 mL) was added. The organic solvents were removed under reduced pressure. The residue was extracted with EtOAc (2 × 10mL), washed with brine (10 mL), dried (MgSO₄), filtered and the solvent removed under reduced pressure to afford a beige oil (171 mg, 52%) that was used without further purification.

### 1.9. Preparation of 4-((2-hydroxy-5-methoxybenzyl)amino)benzonitrile (c9)

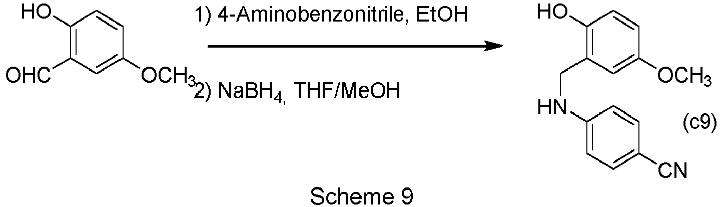

According to scheme 9 a mixture of 2-hydroxy-5-methoxybenzaldehyde (317 mg, 2.1 mmol) and 4-aminobenzonitrile (246 mg, 2.0 mmol) in ethanol (3 mL) was heated in a microwave (120 °C for 30 min). On cooling to room temperature the solid was collected by filtration and washed with cold ethanol (5 mL). The resulting solid was dissolved in THF (4 mL). Methanol (1 mL) was added followed by the portionwise addition of sodium borohydride (38 mg, 1.0 mmol). After 30 min water (10 mL) was added. The organic solvents were removed under reduced pressure. The residue was extracted with EtOAc (2 × 10mL), washed with brine (10 mL), dried (MgSO₄), filtered and the solvent removed under reduced pressure. Trituration with hot hexanes (10 mL) gave the desired product as a beige solid (255 mg, 48%) that was used without further purification.

Other substituted 2-((phenylamino)methyl)phenols (c10-c27, general synthetic scheme 10 below) were prepared following the same general chemistry as outlined in example 1, using the appropriate substituted aniline or aminopyrimidine, and a salicylic aldehyde derivative.

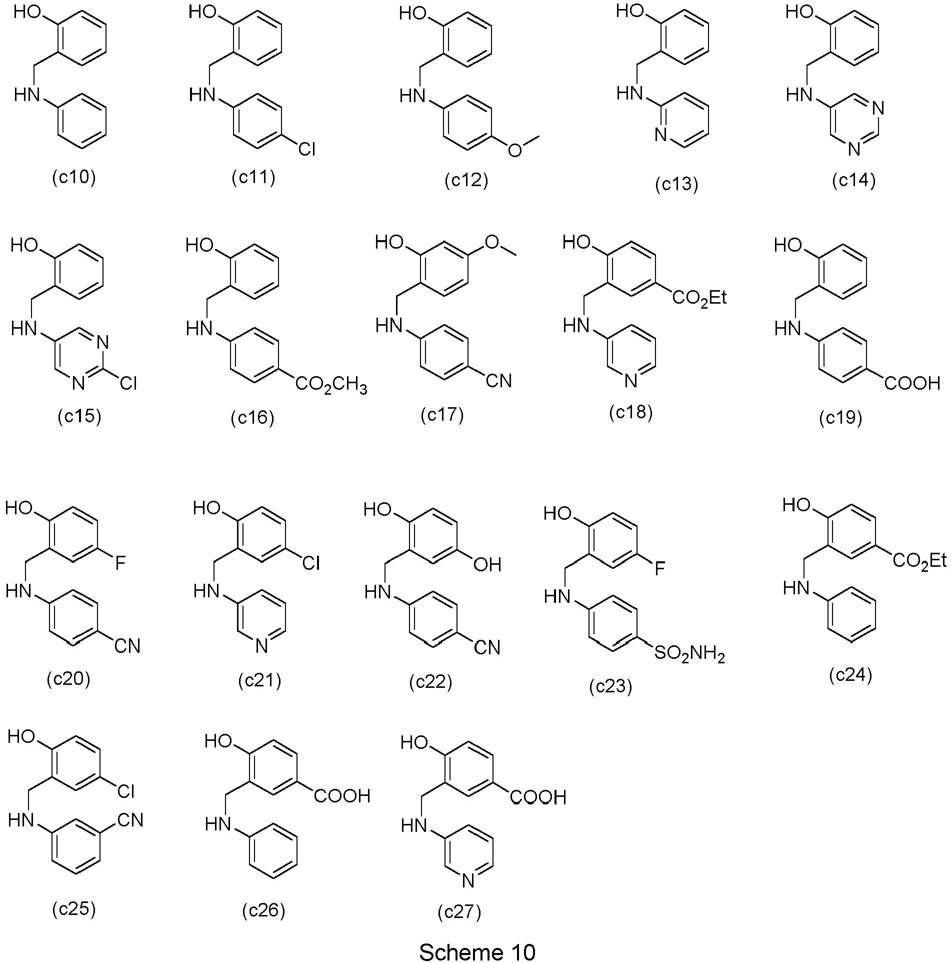

### EXAMPLE 2: Synthesis of 2-(aminomethyl)phenols bearing a substituted heteroaryl group according to general synthetic scheme B.

According to synthetic scheme B describe above herein, 2-(aminomethyl)phenol bearing an amino or alkoxy substituted heteroaryl group (i) were prepared by aromatic nucleophilic substitution of a halide group in compounds of type (f), followed by reduction of the nitro group and finally a reductive animation reaction between compounds of type (h) and (a).

### 2.1. Preparation of 2-(((6-morpholinopyridin-3-yl)amino)methyl)phenol (i1).

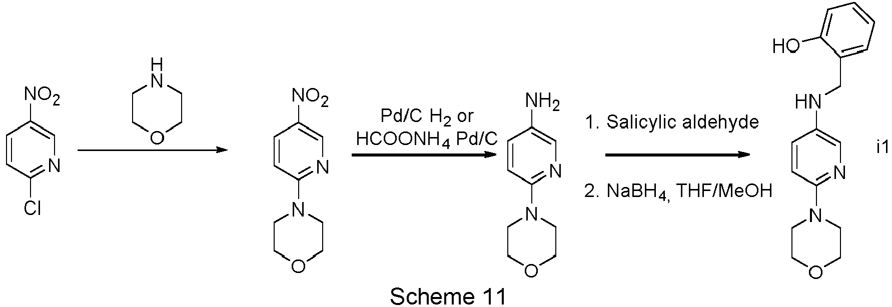

According to scheme 11, morpholine (347 µl, 4.0 mmol) was added dropwise to a stirring solution of 2-chloro-5-nitropyridine (300 mg, 1.9 mmol) in THF (10 ml) at rt. After the addition was completed the reaction was warmed to 50 °C for 1 h. EA (10 ml) was added and the organics were washed sequentially with NaHCO₃ (saturated, 10 ml), water (10 ml) and brine (10 ml). The organics were dried (Na₂SO₄) and the solvent removed under reduced pressure. The resulting bright yellow solid, 4-(5-nitropyridin-2-yl)morpholine (397 mg, 100%) was used without further purification.

Hydrogen gas was applied via a balloon to a stirring suspension of crude 4-(5-nitropyridin-2-yl)morpholine (209 mg, 1.0 mmol) and Pd/C (10%, 10 mg) in DCM (5 ml) at rt. After 18 h the suspension was filtered through celite and the solvent removed under reduced pressure to afford the desired product 6-morpholinopyridin-3-amine (138 mg, 80%) a beige solid.

Salicylic aldehyde (75 mg, 0.61 mmol) was added to a stirring solution of 6-morpholinopyridin-3-amine (100 mg, 0.56 mmol) in absolute ethanol (1 ml) at rt. Heated at reflux for 2h, after which the reaction was allowed to cool to rt. The resulting yellow precipitate was collected by filtration and washed with cold absolute ethanol (3 ml). This solid was then dissolved in THF (4 ml) and methanol (0.5 ml) at rt, and NaBH₄ (43 mg, 1.12 mmol) was added portion-wise. After a further 30 mins at rt, water (5 ml) was added and the organics were removed under reduced pressure. The organics were extracted from the aqueous phase with EA (3 × 8 ml), dried (Na₂SO₄) and the solvent removed. The resulting solid was triturated with hot hexanes (3 × 5 ml) to afford the desired product, 2-(((6-morpholinopyridin-3-yl)amino)methyl)phenol (151 mg, 95%), as a beige solid.

### 2.2. Preparation of 2-(((6-(4-methylpiperazin-1-yl)pyridin-3-yl)amino)methyl)phenol (i2) and 2-(((6-((2-hydroxyethyl)(2-(methylamino)ethyl)amino)pyridin-3-yl)amino)methyl)phenol (i10).

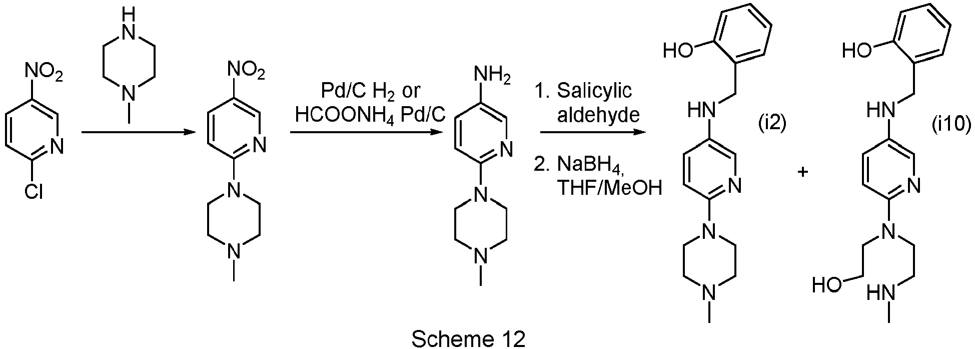

According to scheme 12, N-methylpiperazine (443 µl, 4.0 mmol) was added dropwise to a stirring solution of 2-chloro-5-nitropyridine (300 mg, 1.9 mmol) in THF (10 ml) at rt. After the addition was completed the reaction was warmed to 50 °C for 1 h. EA (10 ml) was added and the organics were washed sequentially with NaHCO₃ (saturated, 10 ml), water (10 ml) and brine (10 ml). The organics were dried (Na₂SO₄) and the solvent removed under reduced pressure. The resulting bright yellow solid, 1-methyl-4-(5-nitropyridin-2-yl)piperazine (324 mg, 77%) was used without further purification.

Hydrogen gas was applied via a balloon to a stirring suspension of crude 1-methyl-4-(5-nitropyridin-2-yl)piperazine (200 mg, 0.90 mmol) and Pd/C (10%, 50 mg) in DCM (10 ml) at rt. After 18 h the suspension was filtered through celite and the solvent removed under reduced pressure to afford the crude product 6-(4-methylpiperazin-1-yl)pyridin-3-amine, as a beige solid, which was used without any further purification.

Salicylic aldehyde (113 µl, 1.1 mmol) was added to a stirring solution of the crude 6-(4-methylpiperazin-1-yl)pyridin-3-amine in absolute ethanol (1 ml) at rt. Heated at reflux for 2h, after which the reaction was allowed to cool to rt. The solvent was removed under reduced pressure and the resulting yellow/brown solid was washed with hot hexanes (2 × 5 ml) and collected by filtration. The resulting yellow solid was then dissolved in THF (6 ml) and methanol (0.5 ml) at rt, and NaBH₄ was added portion-wise until the solution turned colourless. After a further 30 mins at rt, water (2 ml) was added and the organics were removed under reduced pressure. The organics were extracted from the aqueous phase with EA (4 × 5 ml), and washed with brine (10 ml), dried (Na₂SO₄) and the solvent removed. The resulting solid was triturated with hot hexanes (3 × 5 ml) to remove excess aldehyde. Column chromatrography (hexanes/EA; 1:1 to 100% EA) gave compound (i2) (173 mg, 65%, 2 steps), as a beige solid, and compound (i10) (61 mg, 22%) as an off-white solid.

### 2.3. Preparation of 2-(((6-((2-hydroxyethyl)(methyl)amino)pyridin-3-yl)amino)methyl)phenol (i3)

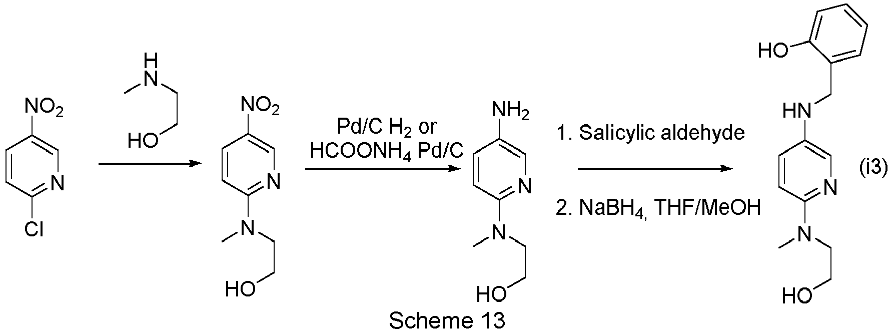

According to scheme 13, 2-(methylamino)ethan-1-ol (320 µl, 4.0 mmol) was added dropwise to a stirring solution of 2-chloro-5-nitropyridine (300 mg, 1.9 mmol) in THF (10 ml) at rt. After the addition was completed the reaction was warmed to 50 °C for 1 h. EA (10 ml) was added and the organics were washed sequentially with NaHCO₃ (saturated, 10 ml), water (10 ml) and brine (10 ml). The organics were dried (Na₂SO₄) and the solvent removed under reduced pressure. The resulting bright yellow solid, 2-(methyl(5-nitropyridin-2-yl)amino)ethan-1-ol (334 mg, 89%) was used without further purification.

Hydrogen gas was applied via a balloon to a stirring suspension of crude 1-methyl-4-(5-nitropyridin-2-yl)piperazine (200 mg, 1.0 mmol) and Pd/C (10%, 25 mg) in DCM (5 ml) at rt. After 48 h the suspension was filtered through celite and the solvent removed under reduced pressure to afford the crude product, 2-((5-aminopyridin-2-yl)(methyl)amino)ethan-1-ol, as a beige solid, which was used without any further purification.

Salicylic aldehyde (127 µl, 1.2 mmol) was added to a stirring solution of the crude 2-((5-aminopyridin-2-yl)(methyl)amino)ethan-1-ol in absolute ethanol (1 ml) at rt. Heated at reflux for 6h, after which the reaction was allowed to cool to rt. The resulting solid was collected by filtration and dissolved in THF (6 ml) and methanol (0.5 ml) at rt. NaBH₄ was added portion-wise until the solution turned colourless. After a further 30 mins at rt, water (2 ml) was added and the organics were removed under reduced pressure. The organics were extracted from the aqueous phase with EA (4 × 5 ml), and washed with brine (10 ml), dried (Na₂SO₄) and the solvent removed. The resulting solid was triturated with hot hexanes (3 × 5 ml) to afford the desired product, 2-(((6-((2-hydroxyethyl)(methyl)amino)pyridin-3-yl)amino)methyl)phenol (162 mg, 58%, 2 steps), as a beige solid.

Other 6-substituted pyridin or pyrimidin-3-yl)amino)methyl)phenols (i4-i8, scheme 14) were prepared following the same general chemistry as outlined in example 2, by first reacting either 2-chloro-5-nitropyridine or 2-chloro-5-nitropyrimidine with the appropriate nucleophile.

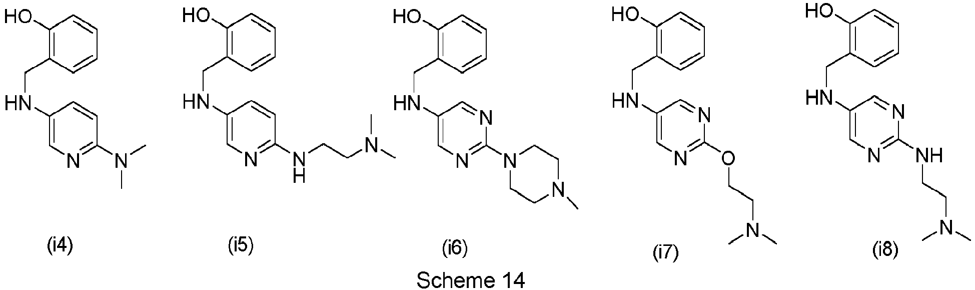

### 2.4. Preparation of 2-(((6-(2-(dimethylamino)ethoxy)pyridin-3-yl)amino)methyl) phenol (i9).

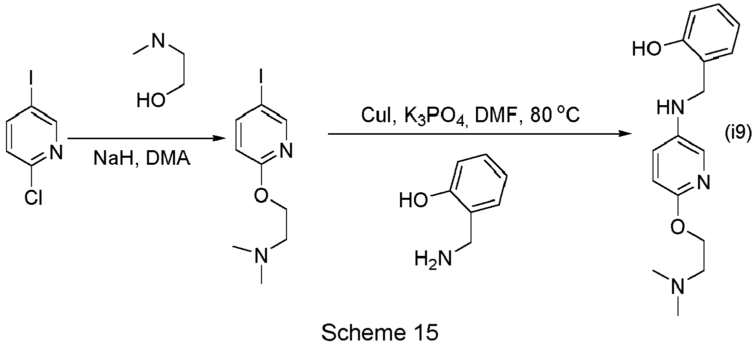

According to scheme 15, NaH (60% w/w, 90 mg, 2.26 mmol) was added in portions to a stirring solution of 2-chloro-5-iodopyridine (300 mg, 1.25 mmol) and 2-(dimethylamino)ethan-1-ol (227 µl, 2.26 mmol) in DMA (1.5 ml) at 0 °C. After the addition was complete the reaction mixture was heated to 50 °C for 3 h, cooled to 0 °C and NH₄Cl (saturated, aq, 3 ml) was added. The organics were extracted with EA (3 × 5 ml), washing with brine (10 ml), dried (Na₂SO₄) and concentrated. Purification by column chromatography (DCM/MeOH; 95:5) gave the desired product 2-((5-iodopyridin-2-yl)oxy)-N,N-dimethylethan-1-amine (349 mg, 96%) as an off-white solid.

A mixture containing 2-((5-iodopyridin-2-yl)oxy)-N,N-dimethylethan-1-amine (250 mg, 0.86 mmol), 2-(aminomethyl)phenol (264 mg, 2.15 mmol), copper (I) iodide (33 mg, 0.17 mmol) and K₃PO₄ (365 mg, 1.72 mmol) in DMF was heated at 80 °C for 5h. Cooled to rt and water (5 ml) was added. The organics were extracted with EA (3 × 8 ml), dried (Na₂SO₄) and concentrated. Purification by column chromatography (DCM/MeOH; 95:5 to 90:10) afforded the desired product 2-(((6-(2-(dimethylamino)ethoxy)pyridin-3-yl)amino)methyl)phenol (126 mg, 51%) as a beige solid.

### EXAMPLE 3: Synthesis of substituted 3,4-dihalo-naphthaquinones (d) from 3,4-dihalofuran-2,5-diones according to general synthetic scheme A.

### 3.1. Preparation of 2,3-dichloro-5,8-dihydroxynaphthalene-1,4-dione (d1).

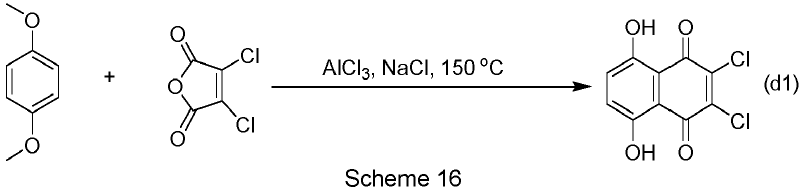

To a stirring mixture of NaCl (2.82 g, 49 mmol) and AlCl₃ (13.8 g, 97 mmol) at 150 °C was added portionwise a blended mixture of 1,4-dimethoxybenzene (1.50 g, 10.8 mmol) and 2,3-dicholoromaleic anhydride (3.60 g, 21.6 mmol). Heating was continued for 30 mins after which the reaction was allowed to cool to rt, then placed in an ice-bath. Ice (80 g) was added portion-wise followed by HCI (aq, conc, 10 ml). The mixture was then left overnight at rt. The organics were extracted with DCM (3 × 20 ml) and the combined organics were washed sequentially with water (3 × 10 ml) and brine (10 ml) and dried (Na₂SO₄). Solvent removal followed by purification by column chromatography gave the desired product 2,3-dichloro-5,8-dihydroxynaphthalene-1,4-dione as purple solid (2.30 g, 82%).

### 3.2. Preparation of 2,3-dichloro-5,8-dimethoxynaphthalene-1,4-dione (d2) and 2,3-dichloro-5-hydroxy-8-methoxynaphthalene-1,4-dione (d11).

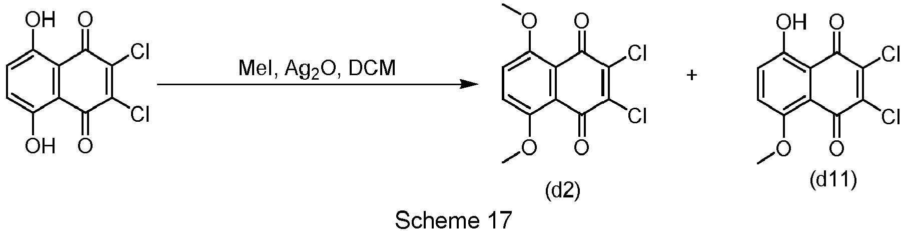

A mixture of Ag₂O (225 mg, 0.97 mmol), methyliodide (143 µl, 2.24 mmol) and 2,3-dichloro-5,8-dihydroxynaphthalene-1,4-dione (d1) (84 mg, 0.32 mmol) in CHCl₃ (1 ml) was stirred in the dark at rt for 2 days. DCM (6 ml) was added and the mixture filtered through a short pad of celite. Removal of solvent followed by column chromatography (Hex/EA; 1:1) gave the desired product 2,3-dichloro-5,8-dimethoxynaphthalene-1,4-dione (39 mg, 15%) as an orange solid and 2,3-dichloro-5-hydroxy-8-methoxynaphthalene-1,4-dione (13 mg, 5%) as a red solid

### 3.3. Preparation of 2,3-dichloro-6-fluoro-5,8-dihydroxynaphthalene-1,4-dione (d3).

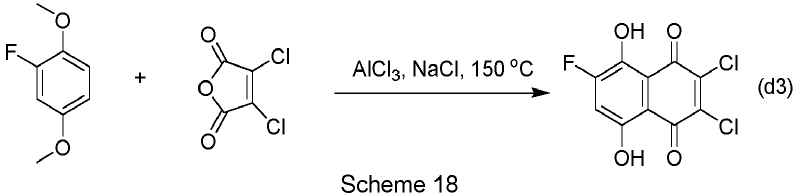

To a stirring mixture of NaCl (2.9 g, 50.0 mmol) and AlCl₃ (12.78 g, 96.1 mmol) at 150 °C was added portionwise a blended mixture of 2-fluoro-1,4-dimethoxybenzene (1.55 g, 10.0 mmol) and 2,3-dicholoromaleic anhydride (3.34 g, 20.0 mmol). Heating was continued for 30 mins after which the reaction was allowed to cool to rt, then placed in an ice-bath. Ice (80 g) was added portion-wise followed by HCI (aq, conc, 10 ml). The mixture was then left overnight at rt. The organics were extracted with DCM (3 × 20 ml) and the combined organics were washed sequentially with water (3 × 10 ml) and brine (10 ml) and dried (Na₂SO₄). Solvent removal followed by purification by column chromatography gave the desired product 2,3-dichloro-6-fluoro-5,8-dihydroxynaphthalene-1,4-dione as purple solid (0.86 g, 31%).

### 3.4. Preparation of 2,3-dichloro-6-fluoro-5,8-dimethoxynaphthalene-1,4-dione (d4).

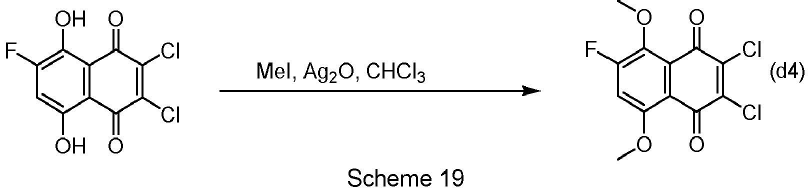

A mixture of Ag₂O (212 mg, 0.92 mmol), methyliodide (175 µl, 2.76 mmol) and 2,3-dichloro-6-fluoro-5,8-dihydroxynaphthalene-1,4-dione (d3) (130 mg, 0.46 mmol) in CHCl₃ (2 ml) was stirred in the dark at rt for 3 days. DCM (6 ml) was added and the mixture filtered through a short pad of celite. Removal of solvent followed by column chromatography (Hex/DCM; 1:1 to 0:1) gave the desired product 2,3-dichloro-6-fluoro-5,8-dimethoxynaphthalene-1,4-dione (52 mg, 36%) as an orange solid.

### 3.5. Preparation of 2,3-dichloro-5,8-dihydroxy-6-(4-methylpiperazin-1-yl)naphthalene-1,4-dione (d5).

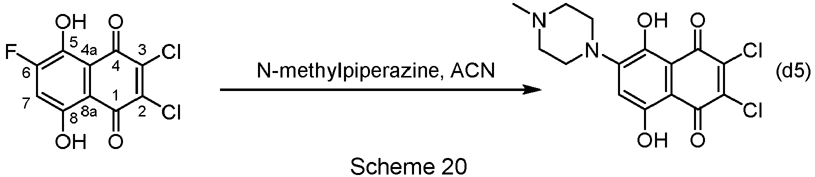

N-Methylpiperazine (16 µl, 0.144 mmol) was added dropwise to a stirring solution of 2,3-dichloro-6-fluoro-5,8-dihydroxynaphthalene-1,4-dione (d3) (40 mg, 0.144) in MeCN (2 ml) at rt. After 15 mins the solvent was removed under reduced pressure and the crude product was purified by column chromatography (DCM/MeOH; 98:2), affording the desired product 2,3-dichloro-5,8-dihydroxy-6-(4-methylpiperazin-1-yl)naphthalene-1,4-dione as a purple solid (34 mg, 66%).

### 3.6 Preparation of 2,3-dichloro-5-hydroxynaphthalene-1,4-dione (d6).

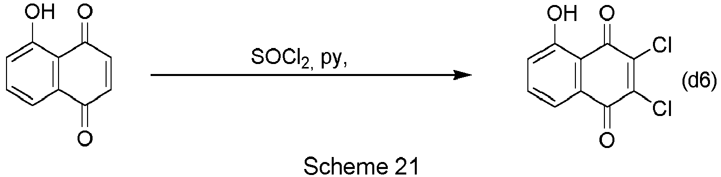

Pyridine (45 µl, 0.55 mmol) was added dropwise to a stirring solution of 5-hydroxy-1,4-naphthoquinone (28 mg 0.16 mmol) in thionyl chloride (0.23 ml) at 0 °C. After 1h the reaction was heated at reflux for 1h, after which the solvent was removed under reduced pressure. Column chromatography of the crude material (CHCl3/Hex; 1:1) gave the desired product 2,3-dichloro-5-hydroxynaphthalene-1,4-dione (22 mg, 70%) as an orange solid.

### 3.7. Preparation of 2,3-dichloro-5-nitronaphthalene-1,4-dione (d8).

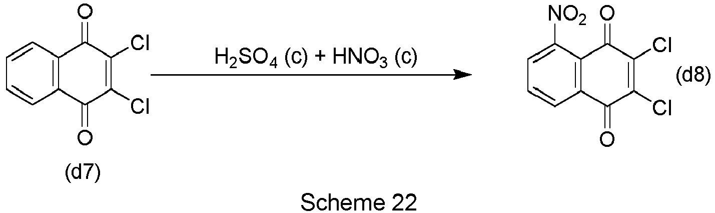

To a stirring solution a sulfuric acid (concentrated, 20 ml) and nitric acid (concentrated, 20 ml) at 40 °C was added portion-wise to commercial available 2,3-dichloronaphthalene-1,4-dione (d7) (2.27 g, 10 mmol). Stirred overnight at 50 °C, then cooled to 0 °C for 1 h. Extracted with DCM (3 × 15 ml) and the combined extracts were washed with water (20 ml), then brine (20 ml) and dried (Na₂SO₄). Solvent removal followed by column chromatography (Hex/EA/DCM; 3:1:1) gave the desired product 2,3-dichloro-5-nitronaphthalene-1,4-dione (0.533 g, 20 %) a yellow solid.

### 3.8. Preparation of 6,7-dichloro-5,8-dioxo-5,8-dihydronaphthalene-2-sulfonyl chloride (d9).

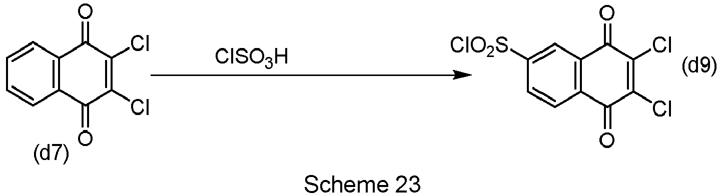

Chlorosulfonic acid (10 ml) was added dropwise to a stirring solution of commercially available 2,3-dichloronaphthalene-1,4-dione (d7) (3.33 g, 14.7 mmol) in CHCl₃ at rt. After the addition was complete the reaction temperature was increased to distill of the chloroform. The reaction was then cooled to 0 °C for and ice (100 g) was added portion-wise. The organics were extracted with DCM (3 × 15 ml) and the combined extracts were washed with water (20 ml), then brine (20 ml) and dried (Na₂SO₄). Solvent removal followed by trituration of the resulting solid with hot hexanes provided the crude product 6,7-dichloro-5,8-dioxo-5,8-dihydronaphthalene-2-sulfonyl chloride as a beige solid (0.533 g, purity 90%), that was used without further purification.

### 3.9. Preparation of 2,3-dichloro-6-(morpholinosulfonyl)naphthalene-1,4-dione (d10).

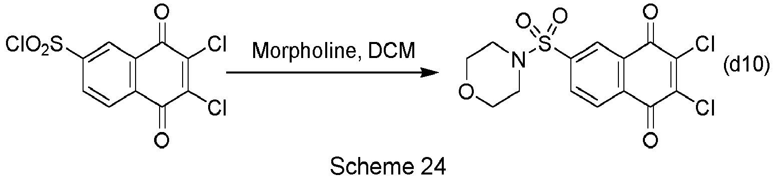

Morpholine (16 µl, 0.19 mmol) was added slowly to a stirring solution of crude 6,7-dichloro-5,8-dioxo-5,8-dihydronaphthalene-2-sulfonyl chloride (d9) (29 mg, 0.09 mmol) at 0 °C. After 2 h the solvent was removed and the crude material was purified by column chromatography (Hex/EA/DCM; 2:1:0.1) to afford the desired product 2,3-dichloro-6-(morpholinosulfonyl)naphthalene-1,4-dione as a beige solid (18 mg, 50%).

### EXAMPLE 4: synthesis of oxazepine compounds of formula (I) of present invention

This example sets forth a method for preparing oxazepine compounds of formula (I) disclosed in table 1, from 2-((arylamino)methyl)phenols, as those prepared in examples 1 and 2, and 3,4-dihalo-naphthoquinones, as those prepared in example 3.

### 4.1. General preparation of oxazepines of formula (I) where R⁴ and/or R⁷ are different from H.

A 2,3-dichloro-1,4-naphthoquinone (d1-d11) (0.4 mmol, 1.0 eq) was added to a stirring solution of a N-substituted 2-(aminomethyl)phenol (0.4 mmol, 1.0 eq) [c(1-27) or i(1-10)] in pyridine (3 mL) at 80 °C. After 1h the reaction was allowed to cool to rt. The mixture was diluted with EtOAc (15 mL) and washed sequentially with aqueous 1N HCI (3 × 10 mL), water (10 mL) and brine (10 mL). The organics were dried (MgSO₄), filtered and the solvent removed under reduced pressure. Purification of the residue by column chromatography or by crystallization gave the desired product.

### 4.2. Preparation of 12-phenyl-12,13-dihydrobenzo[f]naphtho[2,3-b][1,4]oxazepine-6,11-dione (1).

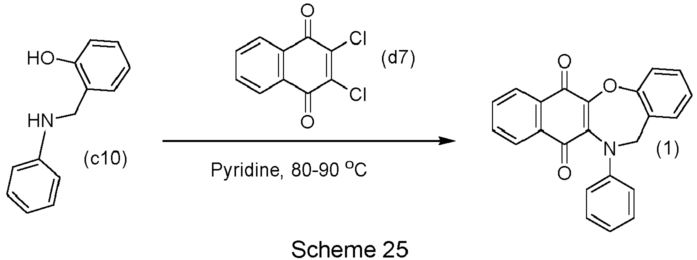

Crystallization (CH₂Cl2/hexanes); purple solid; 65% yield.

### 4.3. Preparation of 4-(6,11-dioxo-6,11-dihydrobenzo[f]naphtho[2,3-b][1,4]oxazepin-12(13H)-yl)benzonitrile (2).

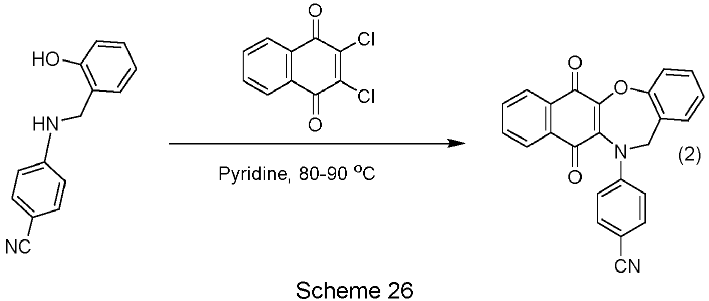

Column chromatography (hexanes/EtOAc; 5:1); dark red solid; 70% yield.

### 4.4. Preparation of 12-(pyridin-3-yl)-12,13-dihydrobenzo[f]naphtho[2,3-b][1,4]oxazepine-6,11-dione (4).

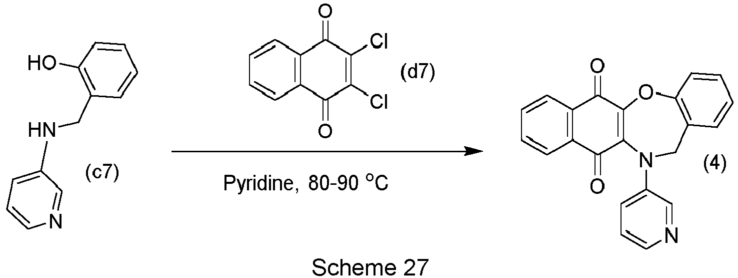

Column chromatography (hexanes/EtOAc; 1:1); dark red solid; 60% yield.

### 4.5. Preparation 12-(pyridin-2-yl)-12,13-dihydrobenzo[f]naphtho[2,3-b][1,4]oxazepine-6,11-dione (5).

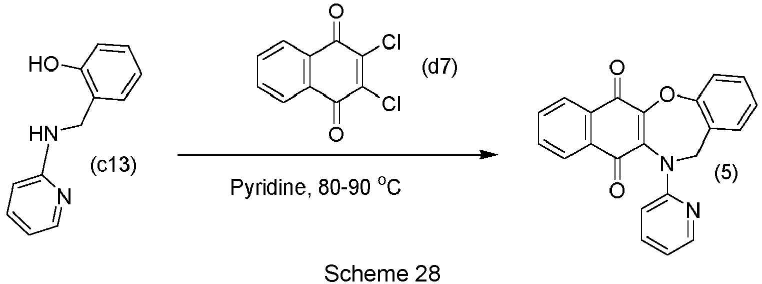

Crystallization (CH₂Cl2/hexanes); purple solid; 78% yield.

### 4.6. Preparation 12-(6-(4-methylpiperazin-1-yl)pyridin-3-yl)-12,13-dihydrobenzo[f]naphtho [2,3-b][1,4]oxazepine-6,11-dione (100).

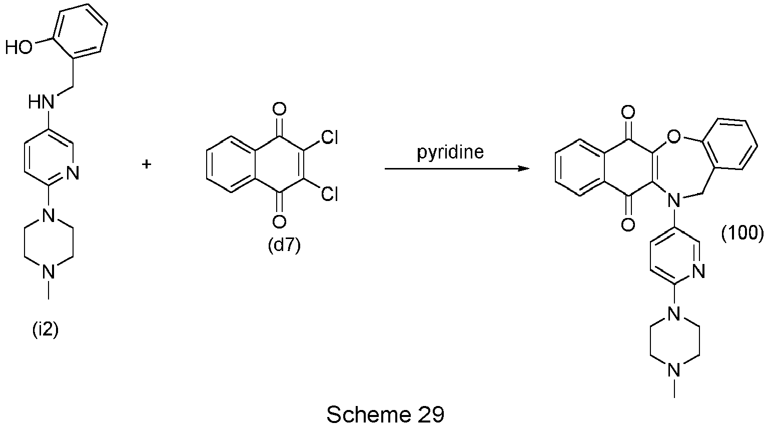

Column chromatography (DCM/MeOH 95:5); dark red solid; 37% yield.

This same procedure using combinations of compounds c1-c27 or i1-i10, with compounds d1-d11, was used as shown in the following **table 2** to prepare the following compounds of formula (I) identified with ID# number indicated in table 1:

**Table 2: oxazepines of formula (I) obtained according to general method of example 4.**

| **Compound** | | | | | | | | |
|---|---|---|---|---|---|---|---|---|
| **(c) or (i)** | **(d)** | **ID #** | **(c) or (i)** | **(d)** | **ID #** | **(c) or (i)** | **(d)** | **ID #** |
| c11 | d7 | **3** | i3 | d7 | **49** | c1 | d8 | **108** |
| c8 | d7 | **6** | i1 | d8 | **68** | i7 | d7 | **110** |
| c17 | d7 | **14** | i1 | d8 | **69** | | | |
| c22 | d7 | **15** | c1 | d10 | **70** | | | |
| c5 | d7 | **16** | c7 | d10 | **71** | | | |
| c19 | d7 | **18** | i9 | d7 | **72** | | | |
| c2 | d7 | **19** | i5 | d7 | **73** | | | |
| c3 | d7 | **20** | c2 | d8 | **79** | | | |
| c7 | d8 | **27** | c2 | d8 | **80** | | | |
| c7 | d8 | **28** | i1 | d2 | **86** | | | |
| c2 | d10 | **29** | c4 | d2 | **94** | | | |
| c1 | d2 | **30** | i4 | d7 | **98** | | | |
| c1 | d4 | **31** | i1 | d7 | **99** | | | |
| c7 | d4 | **35** | c1 | d8 | **107** | | | |

### 4.7. General procedure for the preparation of oxazepines of formula (I) in which R⁴ and/or R⁷ are -OR¹¹.

The required naphthoquinone (d1-d11 1 eq) was added directly to a stirring solution of substituted 2-(aminomethyl)phenols (c1-c27 or i1-i10) (1 eq) and Et₃N (2.25 eq) in acetonitrile (8 mL/mmol) at 80 °C. After 30-90 min the reaction was cooled to rt and EtOAc (30 mL/mmol) was added. The organics were washed sequentially with 1N HCI (aq, 2 × 5 mL) and brine (10 mL), then dried (Na₂SO₄) and filtered. Removal of the solvent followed by purification using column chromatography (hexanes/EtOAc) afforded the desired products.

### 4.8. Preparation of 7,10-dihydroxy-12-phenyl-12,13-dihydrobenzo[f]naphtho[2,3-b][1,4]oxazepine-6,11-dione (45).

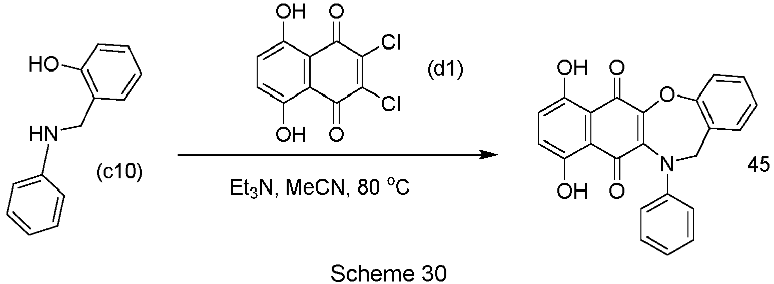

Column chromatography (hexanes/EtOAc; 2:1); purple solid; 92% yield.

### 4.9. Preparation of 4-(7,10-dihydroxy-6,11-dioxobenzo[f]naphtho[2,3-b][1,4]oxazepin-12(6H,11H,13H)-yl)benzonitrile (8).

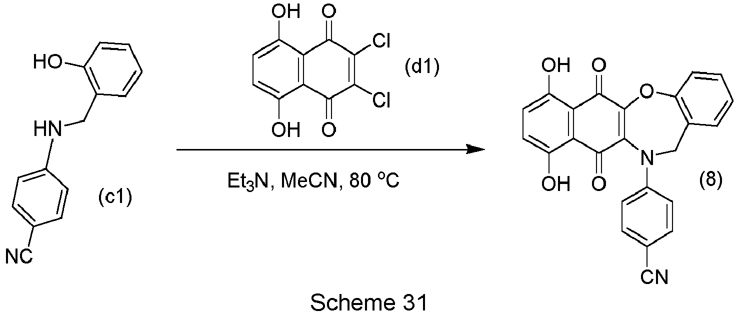

Column chromatography (hexanes/EtOAc; 2:1); deep red solid; 64 % yield.

### 4.10. Preparation of 7,10-dihydroxy-12-(pyridin-3-yl)-12,13-dihydrobenzo[f]naphtho[2,3-b][1,4]oxazepine-6,11-dione (24).

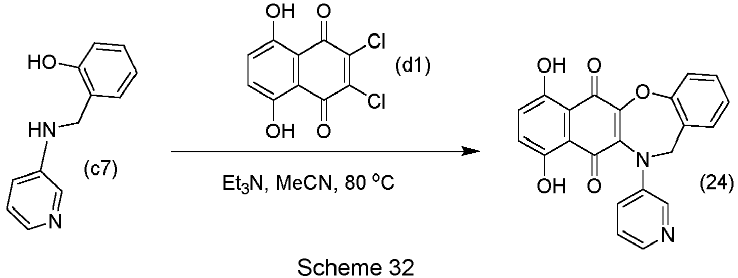

Column chromatography (hexanes/EtOAc; 1:1); purple solid; 75% yield.

### 4.11. Preparation of 3-(7,10-dihydroxy-6,11-dioxo-6,11-dihydrobenzo[f]naphtho[2,3-b][1,4]oxazepin-12(13H)-yl)benzonitrile (9).

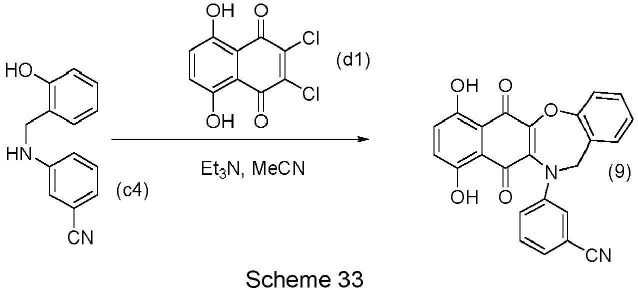

Column chromatography (hexanes/EtOAc; 3:1); Deep purple solid; 72% yield.

### 4.12. Preparation of 12-(6-(dimethylamino)pyridin-3-yl)-7,10-dihydroxy-12,13-dihydrobenzo[f]naphtho[2,3-b][1,4]oxazepine-6,11-dione (62).

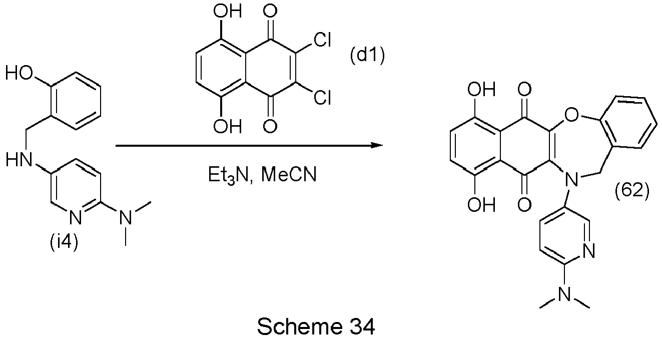

Column chromatography (hexanes/EtOAc; 3:1); Deep purple solid; 53% yield.

### 4.13. Preparation of 7,10-dihydroxy-12-(6-(4-methylpiperazin-1-yl)pyridin-3-yl)-12,13-dihydrobenzo[f]naphtho[2,3-b][1,4]oxazepine-6,11-dione (81).

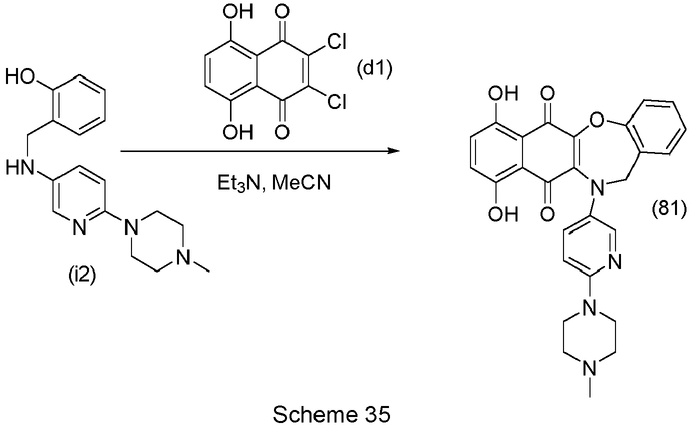

Column chromatography (hexanes/EtOAc; 3:1); Deep purple solid; 54% yield.

### 4.14. Preparation of 12-(6-((2-(dimethylamino)ethyl)amino)pyridin-3-yl)-7,10-dihydroxy-12,13-dihydrobenzo[f]naphtho[2,3-b][1,4]oxazepine-6,11-dione (97).

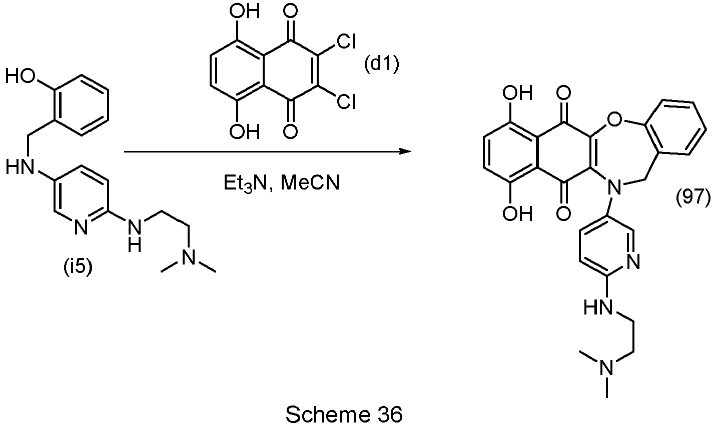

Column chromatography (DCM/MeOH; 95:5); Deep purple solid; 47% yield.

### 4.15. Preparation of 7,10-dimethoxy-12-(pyridin-3-yl)-12,13-dihydrobenzo[f]naphtho[2,3-b][1,4]oxazepine-6,11-dione (85).

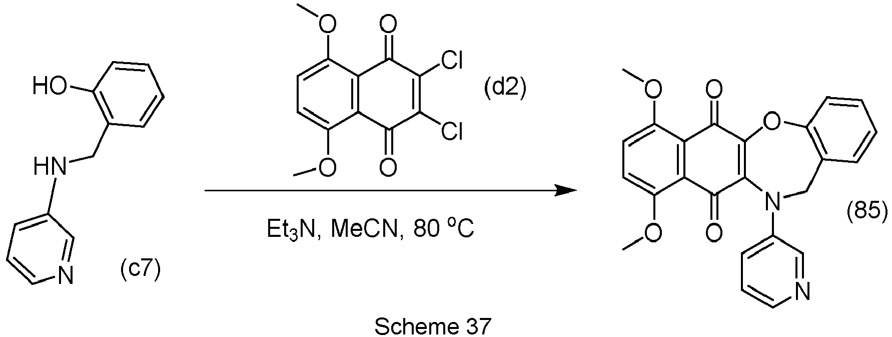

Column chromatography (hexanes/EtOAc; 3:1); orange solid; 71% yield.

### 4.16. Preparation of 12-(6-chloropyridin-3-yl)-7,10-dihydroxy-12,13-dihydrobenzo[f]naphtho[2,3-b][1,4]-oxazepine-6,11-dione (47)

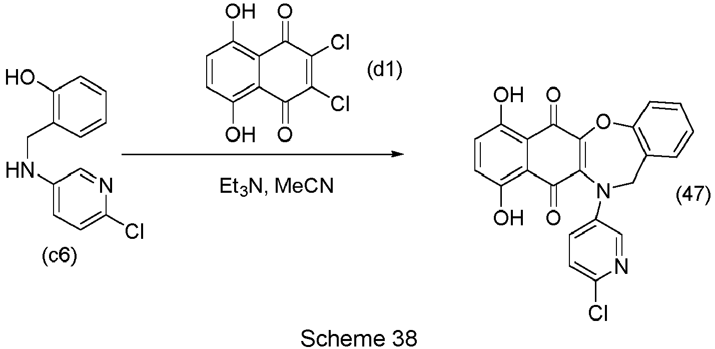

Column chromatography (hexanes/EtOAc; 3:1); Deep purple solid; 58% yield.

### 4.17. Preparation of 4-(7,10-dihydroxy-6,11-dioxo-6,11-dihydrobenzo[f]naphtho[2,3-b][1,4]oxazepin-12(13H)-yl)benzenesulfonamide (40).

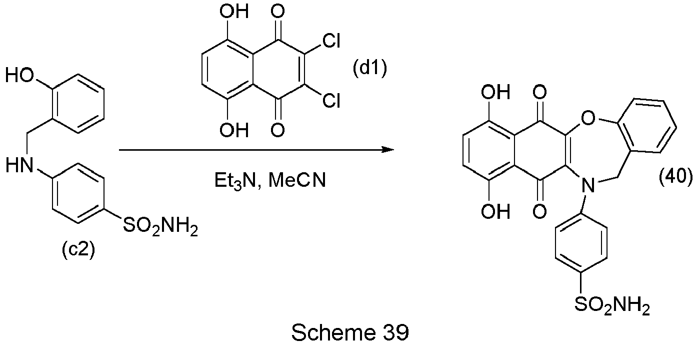

Column chromatography (hexanes/EtOAc; 3:1); Deep purple solid; 47% yield.

### 4.18. Preparation of 7,10-dimethoxy-12-phenyl-12,13-dihydrobenzo[f]naphtho[2,3-b][1,4]oxazepine-6,11-dione (65).

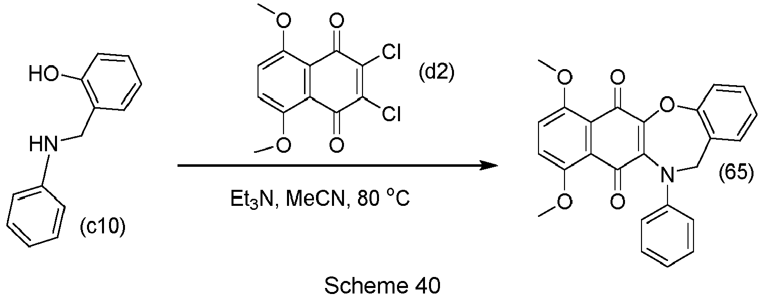

Column chromatography (hexanes/EtOAc; 3:1); orange solid; 84% yield.

This same general procedure shown in example 4.7 using combinations of compounds c1-c27 or i1-i10, with compounds d1-d11, was used to prepare the following compounds of formula (I) shown in the following **table 3**, identified with ID# number indicated in table 1:

**Table 3: oxazepines of formula (I) obtained according to general method of example 4.7**

| Compound | | | | | | | | |
|---|---|---|---|---|---|---|---|---|
| (c) or (i) | (d) | ID# | (c) or (i) | (d) | ID# | (c) or (i) | (d) | ID# |
| c5 | d1 | **11** | c16 | d1 | **48** | c10 | d11 | **83** |
| c20 | d1 | **12** | i3 | d7 | **49** | c10 | d11 | **84** |
| c17 | d1 | **13** | c26 | d1 | **50** | c14 | d1 | **91** |
| c3 | d1 | **22** | c18 | d1 | **51** | c4 | d11 | **93** |
| c23 | d1 | **23** | c27 | d1 | **52** | i7 | d1 | **101** |
| c21 | d1 | **25** | c19 | d1 | **57** | i4 | d6 | **103** |
| c8 | d1 | **26** | c4 | d11 | **66** | i1 | d6 | **104** |
| c1 | d5 | **32** | c1 | d6 | **75** | i5 | d6 | **105** |
| c7 | d5 | **33** | i1 | d1 | **59** | i2 | d6 | **106** |
| c1 | d3 | **34** | i3 | d1 | **61** | i8 | d1 | **109** |
| i9 | d1 | **36** | i10 | d1 | **63** | i6 | d1 | **111** |
| c25 | d1 | **43** | c24 | d1 | **64** | | | |
| c12 | d1 | **46** | c7 | d6 | **76** | | | |

### 4.19. General procedure for the conversion of cyan substituted oxazepines of formula (I) into amido substituted oxazepines of formula (I).

A mixture of cyano oxazepine derivative (1.15 mmol) in acetic acid/sulphuric acid (1.4 mL; 1:1) was heated at 80 °C for 1h. After cooling to room temperature the solution was poured onto ice (5g). The resulting solid was collected by filtration, washed with water (2 × 5 mL) and dried *in vacuo* at 65 °C. If necessary, the resulting material was purified by column chromatography or crystallization.

### 4.20. Preparation of 3-(7,10-dihydroxy-6,11-dioxo-6,11-dihydrobenzo[f]naphtho[2,3-b][1,4]oxazepin-12(13H)-yl)benzamide (54).

The reaction was carried out departing from oxazepine (9) obtained in example 4.11 above herein and following the general procedure of example 4.19:

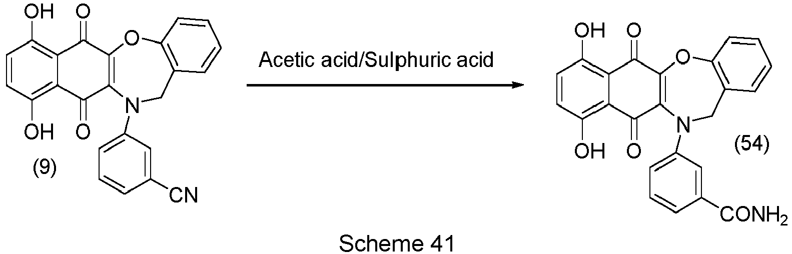

Deep purple solid; 81% yield.

### 4.21. Preparation of 4-(7,10-dihydroxy-6,11-dioxo-6,11-dihydrobenzo[f]naphtho[2,3-b][1,4]oxazepin-12(13H)-yl)benzamide (10).

The reaction was carried out departing from oxazepine (8) obtained in example 4.9 above herein and following the general procedure of example 4.19:

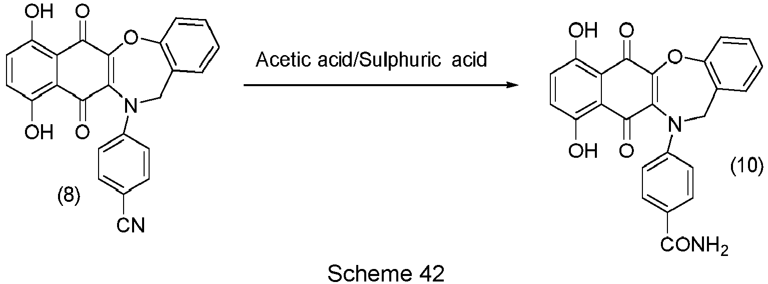

Deep purple solid; 75% yield.

### 4.22. Preparation of 4-(6,11-dioxo-6,11-dihydrobenzo[f]naphtho[2,3-b][1,4]oxazepin-12(13H)-yl)benzamide (17).

The reaction was carried out departing from oxazepine (2) obtained in example 4.3 above herein and following the general procedure of example 4.19:

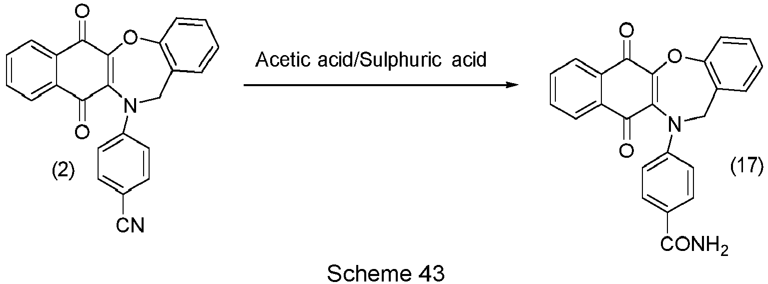

Pale yellow solid; 68% yield.

This same procedure was used to convert the following oxazepines of formula (I) indicated as "departing oxazepine" in table 4 below into the oxazepines of formula (I) indicated as "oxazepine obtained" in table 4 below (all oxazepines identified with ID# number from table 1):

**Table 4: Amido-derived oxazepines of formula (I) obtained according to general method 4.19**

| Departing oxazepine ID# | Oxazepine obtained ID# |
|---|---|
| 6 | **7** |
| 16 | **21** |
| 11 | **41** |
| 15 | **42** |
| 43 | **44** |
| 12 | **55** |
| 26 | **56** |

### 4.23. General method for the conversion of compounds containing phenolic groups to their corresponding acetate derivatives.

In general, the phenolic oxazepine compound is dissolved in pyridine (1 mL/50 mg of compound) at room temperature and a slight excess (1.2 equivalents/OH group) of acetic anhydride was added. The resulting solution was stirred overnight, after which ethyl acetate (10 ml/50 mg of compound) was added. The organics were washed sequentially with water (5 × 10 ml) and 1N HCI aqueous (5 ml) and then brine (5 ml). The organics were dried (Na₂SO₄) and filtered. Column chromatography (hexanes/ethyl acetate) afforded the desired acetylated products.

### 4.24. 12-(3-cyanophenyl)-6,11-dioxo-6,11,12,13-tetrahydrobenzo[f]naphtho[2,3-b][1,4] oxazepine-7,10-diyl diacetate (82)

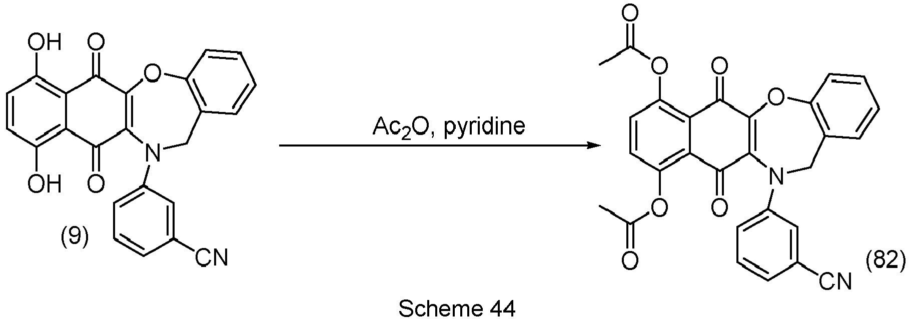

Pale yellow solid. Yield 73%.

### 4.25. 6,11-dioxo-12-phenyl-6,11,12,13-tetrahydrobenzo[f]naphtho[2,3-b][1,4]oxazepine-7,10-diyl diacetate (87)

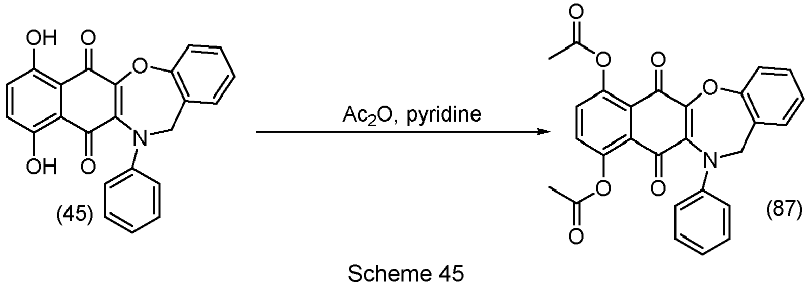

Pale yellow solid. Yield 66%

### 4.26. 12-(3-carbamoylphenyl)-6,11-dioxo-6,11,12,13-tetrahydrobenzo[f]naphtho[2,3-b][1,4] oxazepine-7,10-diyl diacetate (92)

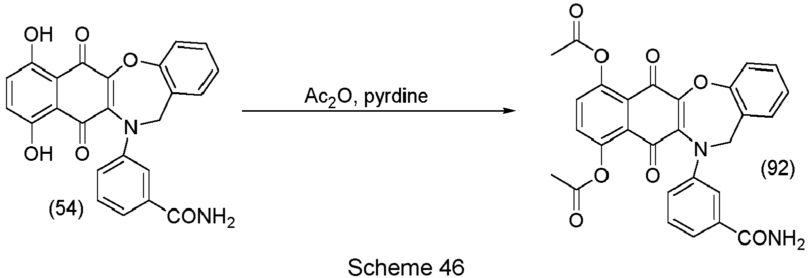

Pale yellow solid. Yield 57%

### 4.27. 12-(4-cyanophenyl)-6,11-dioxo-6,11,12,13-tetrahydrobenzo[f]naphtho[2,3-b][1,4] oxazepine-7,10-diyl diacetate (67).

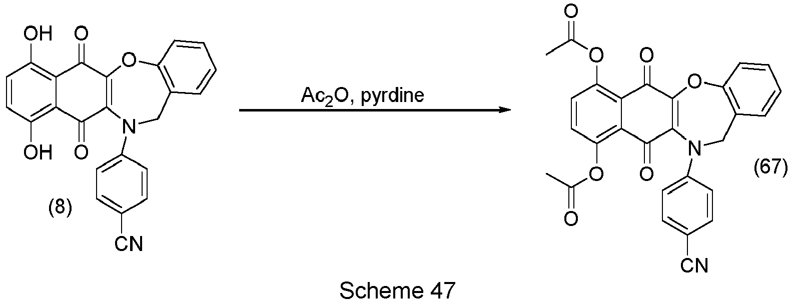

Pale yellow solid. Yield 76%

### 4.28. 12-(6-(dimethylamino)pyridin-3-yl)-6,11-dioxo-6,11,12,13-tetrahydrobenzo[f]naphtha [2,3-b][1,4]oxazepine-7,10-diyl diacetate (95)

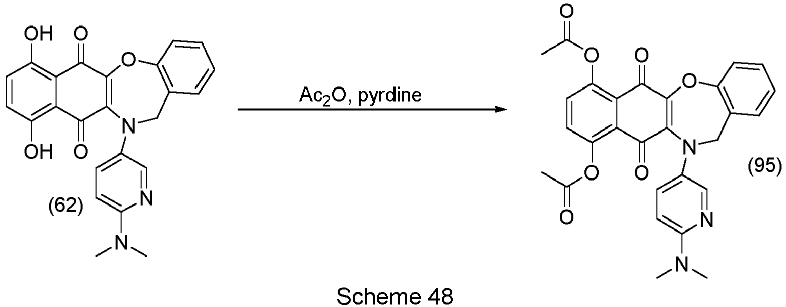

Pale yellow solid. Yield 43%

### 4.29. 12-(6-morpholinopyridin-3-yl)-6,11-dioxo-6,11,12,13-tetrahydrobenzo[f]naphtha [2,3-b][1,4] oxazepine-7,10-diyl diacetate (96)

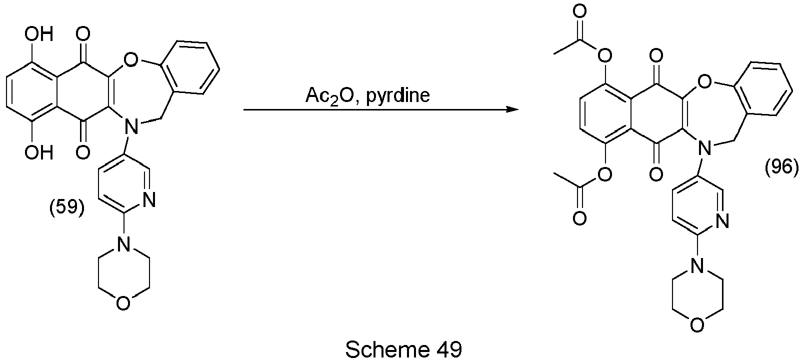

Yellow solid. Yield 51%

### 4.30. 6,11-dioxo-12-(pyridin-3-yl)-6,11,12,13-tetrahydrobenzo[f]naphtho[2,3-b][1,4] oxazepine-7,10-diyl diacetate (60).

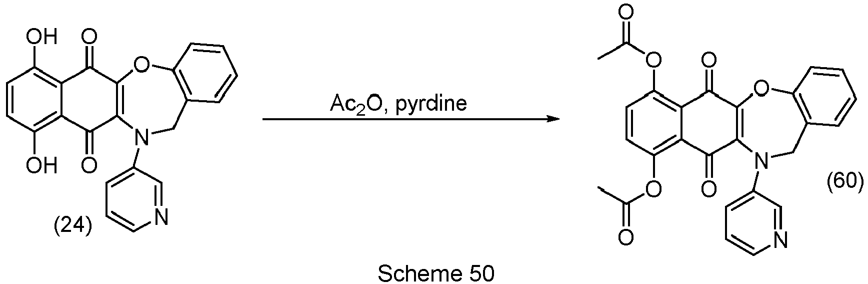

Yellow solid. Yield 63%

This same procedure was used to convert the following oxazepines of formula (I) indicated as "departing oxazepine" in table 5 below into the oxazepines of formula (I) indicated as "oxazepine obtained" in table 5 below (all oxazepines identified with ID# number from table 1):

**Table 5: Acetylated oxazepines of formula (I) obtained according to general method 4.23**

| **Departing oxazepine ID#** | **Oxazepine obtained ID#** |
|---|---|
| 57 | **53** |
| 45 | **88** |
| 45 | **89** |
| 24 | **90** |

### 4.31. Preparation of 7,10-dihydroxy-12-(4-(morpholine-4-carbonyl)phenyl)-12,13-dihydrobenzo[f]naphtho[2,3-b][1,4]oxazepine-6,11-dione (58).

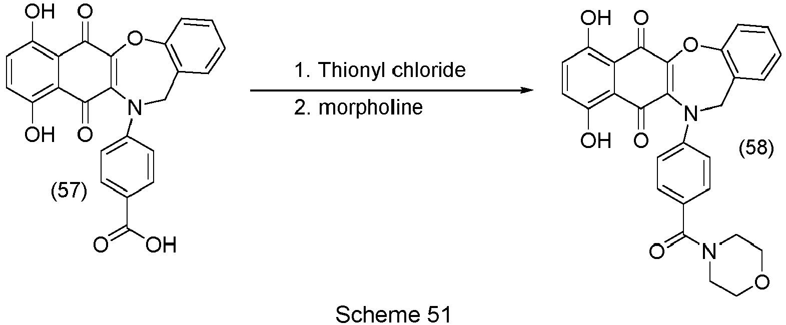

Thionyl chloride (7 uL, 0.09 mmol) was added to a solution of compound 57 (20 mg, 0.05 mmol) in 1,2-dichloroethane (0.2 mL) at RT. The solution was stirred for 30 mins and then morpholine (10 µL) was added. The reaction was then heated at reflux for 1h. Cooled to RT, and the solvent removed by rotary evaporation. Column chromatography (hexanes /EA; 1:1) gave the desired product as a dark red solid (2.4 mg, 10% yield).

### 4.32. Preparation of methyl (4-(7,10-dihydroxy-6,11-dioxo-6,11-dihydrobenzo[f]naphtho[2,3-b][1,4]oxazepin-12(13H)-yl)benzoyl)glycinate (102).

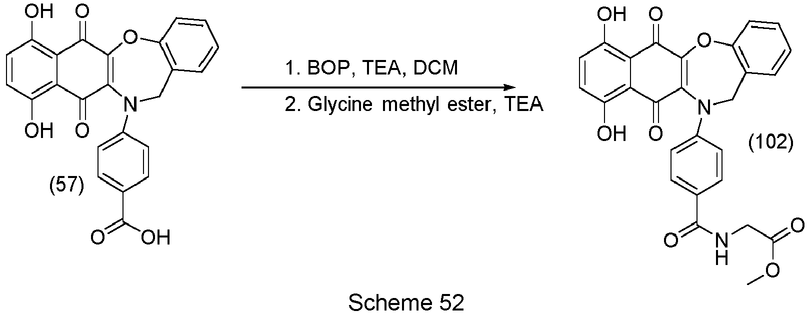

BOP (10 mg, 0.023 mmol) was added to a stirring mixture of compound 57 (10 mg, 0.023 mmol) and trimethylamine (10 µl, 0.069 mmol) in DCM (1 ml) at RT. After 20 mins glycine methyl ester. HCI (3 mg, 0.023 mmol) was added and the mixture stirred for another 1 h. The reaction was then diluted with EA (5 ml) and washed sequentially with water (2 × 5 ml), 1N HCl (5 ml) and brine (5 ml). The organics were then dried (Na₂SO₄) and the solvent removed. Column chromatography (DCM/EA; 3:1) gave the desired product as a dark red solid (4.3 mg, 40 %).

### 4.33. Preparation of 4-(7-amino-6,11-dioxo-6,11-dihydrobenzo[f]naphtho[2,3-b][1,4]oxazepin-12(13H)-yl)benzonitrile (77).

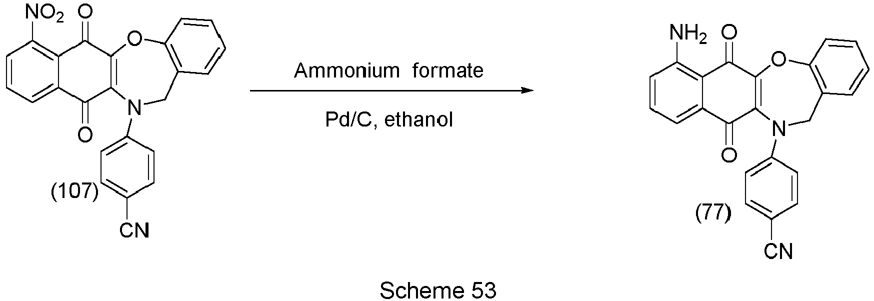

A mixture of Pd/C (10 mg, 10% w/w), compound 107 (35 mg, 0.083 mmol) and ammonium formate (23 mg, 0.37 mmol) in absolute ethanol (2 ml), was heated at reflux for 2h. Cooled to RT, filtered through a pad of celite and then purified by column chromatography (hexanes/EA; 3:2) gave the desired product as a dark orange solid (25 mg, 74%).

### 4.34. Preparation of N-(12-(4-cyanophenyl)-6,11-dioxo-6,11,12,13-tetrahydrobenzo[f]naphtha [2,3-b][1,4]oxazepin-7-yl)methanesulfonamide (78).

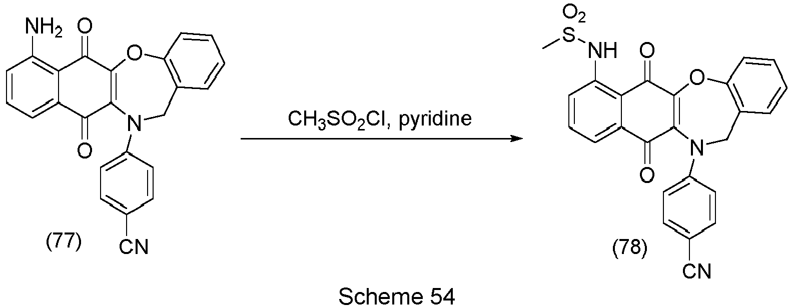

A solution of compound 77 (10 mg, 0.025 mmol), and methanesulphonyl chloride (1.3 eq) in pyridine (1 ml) was heated at reflux for 1h. Cooled to RT, and the solvent removed under reduced pressure. Column chromatography (hexanes/EA; 3:2) gave the desired product as a brown solid (7 mg, 59%).

### 4.35. Preparation of 4-(8-bromo-7-hydroxy-6,11-dioxo-6,11-dihydrobenzo[f]naphtho[2,3-b][1,4]oxazepin-12(13H)-yl)benzonitrile (74).

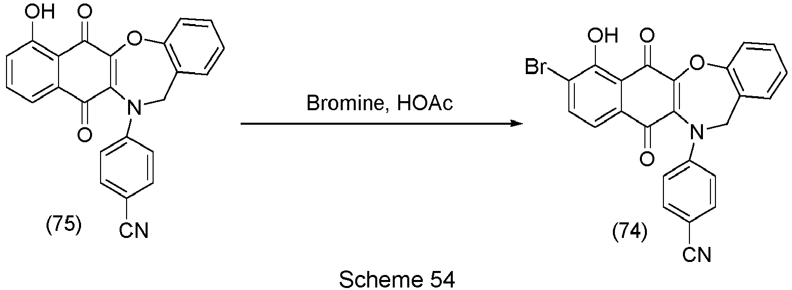

Bromine (5 µl, 2.25 eq) was added to a stirring solution of compound 75 (39 mg, 0.1 mmol) in acetic acid (1 ml) at RT. After 20 mins the reaction was heated to 60 °C for 1h. Cooled to RT, and diluted with EA (5 ml). Washed with water (2 × 5 ml), then saturated NaHCO₃ (2 × 5 ml) and finally brine (5 ml). Organics dried (Na₂SO₄) and concentrated. Column chromatography (toluene, EA; 10:1) gave the desired product as a brown solid (27 mg, 57%).

### 4.36 General procedure for the preparation of compounds (37), (38) and (39).

To a solution containing the corresponding oxazepine phenol derivative (1.0 mmol) and Ph₃P (1.05 g, 4.0 mmol) in THF (10 mL) was added N,N-dimethylethanolamine (356 mg, 4.0 mmol) and DIAD (606 mg, 3.0 mmol). The mixture was stirred at RT for 16 h. The reaction mixture was diluted with EtOAc (20 mL) and extracted with water (2 × 10 mL). The organic layer was washed with brine (10 mL), dried over anhydrous Na₂SO₄, and concentrated to give the crude product. This residue was purified with silica gel column.

### 4.37. Conversion of compound (8) into compound (39)

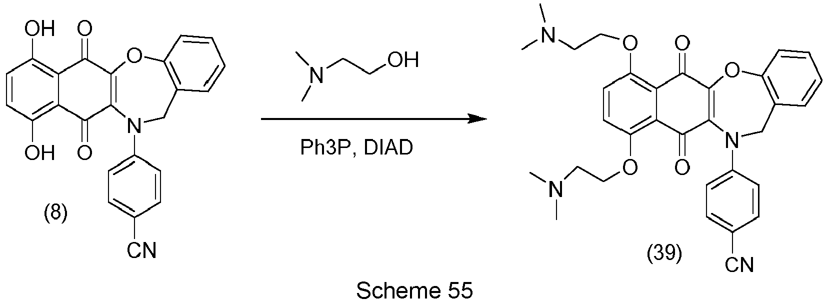

This procedure was used to convert the following oxazepines of formula (I) indicated as "departing oxazepine" in table 6 below into the oxazepines of formula (I) indicated as "oxazepine obtained" in table 6 below (all oxazepines identified with ID# number from table 1):

**Table 6: O-alkylated oxazepines of formula (I) obtained according to general method 4.36**

| **Departing oxazepine ID#** | **Oxazepine obtained ID#** |
|---|---|
| 36 | **37** |
| 24 | **38** |

### EXAMPLE 5: Biological experiments

Experiments were performed as shown below for the compounds of formula (I) of present invention prepared in the examples described above herein.

### 5.1. Method used to identify PRDX1 as a target protein for a representative compound of formula (I) of present invention, compound (24)

The immobilization of compound (**24**) and the identification of its targets was accomplished using a previously described method [Orive-Ramos A, Seoane S, Ocaña A, Pandiella A, Montero JC. Regulation of the prometastatic neuregulin-MMP13 axis by SRC family kinases: therapeutic implications. Mol Oncol. 2017 Dec; 11(12): 1788-1805] (ref. 32). Briefly, 5 mg of the compound was dissolved in a coupling solution (50% dimethylformamide, 50% 0.1 M Na₂CO₃), and incubated with 0.5 mg of epoxy-activated sepharose 6B (GE Healthcare Life Sciences; Piscataway, NJ, USA), obtaining a CM728-coupled resin. A control resin was prepared without the compound (uncoupled). Both resins were incubated with 25 mg of cell extracts from MDA-MB-231 and BT-549 cancer cells.

The resulting complexes were retrieved by centrifugation, washed with lysis buffer, re-suspended and boiled in loading buffer and resolved in SDS-PAGE. The gel was silver stained and the bands of interest were excised, digested by trypsin (Figure 1). The peptide mass fingerprint was obtained on an Ultraflex MALDI-TOF mass spectrometer (Bruker Daltonics, Bremen, Germany), and Mascot search engine (Matrix Science, London, UK) against Swiss-Prot database was used to identify proteins (for a more complete description of this type of assay see: Blood, 2007, 110, 4055-4063 (ref. 33).

The score obtained was 223, wherein proteins score above 56 were considered to be significant (p < 0.05).

### 5.2. Method used to determine the manner in which compound (24) binds to PRDX1 and/or PRDX2.

Using MDA-MB-231 [American Type Culture Collection (ATCC) reference number HTB-26] cell lysates the process described in example 5.1 was repeated (lanes 1 and 2, in **figure 2**). In a separate experiment the cell lysates were first incubated (1.5 hours) with 50 micrograms of free compound **24** prior to the addition of the resins. SDS-PHAGE and silver staining analysis was then performed (lanes 3 & 4).

In another experiment the resins collected after being incubated with cell lysates (analyzed in lanes 1 and 2) were further treated with free compound **24** to elute non-covalent compound **24** specific, binding proteins. The eluents were concentrated and subjected to the same SDS-PHAGE/silver staining analysis (lanes 9 & 10). Finally, the washed resins were boiled in loading buffer and subjected to SDS-PAGE and silver staining analysis (lanes 5-8).

The intense band at around 20 kDa was identified by MALDI-TOF analysis to be PRDX1, and/or PRDX2 **(****figure 2**).

This experiment indicated that compound **24** interacts with PRDX1 and/or PRDX2 in a non-reversible covalent manner.

### 5.3. Validation of Prdx1 as target of compound 24 in triple negative breast cancer cells.

As indicated in the brief description of the figures, the treatment of untreated MDA-MB-231 cell lysates with a resin coupled to compound 24 (24-coupled resin) were subjected to immunoprecipitation analysis for PRDX1 [for the methodology to perform immunoprecipitation and western blot analysis of PRDXs see Am J Physiol Heart Circ Physiol. 2008 Jul; 295(1): H425-H433 (ref. 34); and Oncogene. 2013 November 7; 32(45): 5302-5314 (ref. 35)].

The results demonstrated that PRDX1 was clearly trapped by the 24-coupled resin. Conversely, as expected it was not able to trap PRDX1 from lysates pre-treated with unbound 24 **(****Figure 3**).

### 5.4. Method used to determine the changes in PRDX1 upon addition of compound 24.

To that end, compound **24** 1 µM was added to MDA-MB-231 cells and followed by Western Blot analysis. β-actin was used as control.

The results indicate that compound 24 causes:
- a rapid conversion of PRDX1 from its basal active monomeric form, into its inactive dimeric form, in non-reducing conditions (using SDS non-reduced sample buffer [100 mM Tris·HCl (pH 6.8), 4% SDS, 0.02% bromphenol blue, and 20% glycerol] before SDS-PAGE), as shown in **figure 4**.
- a significant increase in the levels of inactive hyperoxidized 2-cystiene PRDX1 (marked as SO_{2/3}-Prdx), in reducing conditions (reduced samples were prepared with 10% (vol/vol) mercaptoethanol), as shown in **figure 5**.

Non-reducing and reducing conditions used are those commonly used to assess the status of PRDX1 (see ref. 34 and 35) *ref. 35*

### 5.5. Method used to prepare shPRDX1s and their effects upon ROS levels, and cell viability, in TNBC cells.

Various short-hairpin Peroxiredoxin-1 (shPRDX1) were prepared following a general method previously described (ref. 32).

Briefly, HEK 293T cells were cotransfected with 4 µg of plasmids (pMDLg/RRE, pRSV-Rev and pMD2.G) (Addgene; Cambridge, MA, USA), and 8 µg of the pLKO.1 lentiviral plasmid containing the short hairpin RNA control (shCtrl or shC) or targeting PRDX1 (sh#09, sh#10, sh#11, sh#12 or sh#13) (Dharmacon; Lafayette, CO, USA), using jetPEI transfection reagent (Polyplustransfection; IIIkirch, France).

The lentiviral particles formed after 48 h were used to infect MDA-MB-231 and BT-549 cells, which were allowed to grow to get an efficient knockdown and then selected with 3 µg/mL of puromycin (Sigma-Aldrich). Among the five different shRNA sequences targeting PRDX1 tested, sh#10, 11 and 12 were selected for use in the following experiments (**Figure 6**).

PRDX1 knockdown cell lines, MDA-MB-231 or BT-549, obtained as described above, were seeded at a density of 250,000 cells/well in 6-well plates and incubated at 37°C. After 24 h, 2'-7'dichlorofluorescin diacetate (DCFH-DA) 5 µM was added to the cells and incubated for a further 1 h. Cells were then trypsinized, and collected at 500 x *g* for 5 min at 4 °C. Pellets were washed, centrifuged and re-suspended in cold PBS. DCFH-DA fluorescence was analyzed by flow cytometry using a BD FACS Verse cytometer (BD Biosciences). The cell viability of PRDX1 knock-downed cell lines was analyzed using the MTT assay. For a more detailed description of this assay see example 5.7 below.

**Figure 6** highlights the results of these experiments, wherein it is seen that the downregulation of PRDX1 via the use of short-hairpin constructs (shown in figure 6, top) induced an increase in the levels of ROS (% of DCFH-DA, figure 6 middle), and a decrease in the percentage of viable cells (MTT assay, figure 6 bottom).

### Example 5.6: Comparison of the effects of compound 24, and shPRDX1 induced downregulation of PRDX1, on signaling pathways involved in cell survival and proliferation in two human derived TNBC cell lines (Figure 7).

MDA-MB-231 cells were incubated with compound 24 (5 µM, 1 h), then washed with cold PBS-vanadate (1 mM). The same process was done with BT-549 cells with the exception that the cells were incubated with compound 24 at 1 µM of 3 h. PRDX1 knockdown cells, or compound 24 treated cells, were then lysed with cold RIPA containing protease and phosphatase inhibitors or with a buffer containing 20 mM Tris-HCI, pH = 7, 140 mM NaCl, 50 mM EDTA, 10% glycerol, 1% NP-40, and protease and phosphatase inhibitors. Lysates were centrifuged at 20,000 × *g*, at 4 °C for 10 min, and supernatants were collected. Protein concentration of lysis extracts was measured by BCA. Electrophoresis sample buffer containing 10% β-mercaptoethanol was added to 60 µg of extracts. In the case of non-reducing electrophoresis, cells were incubated with cold PBS containing 25 mM NEM for 30 min before lysis, and β-mercaptoethanol was omitted in the electrophoresis sample buffer. Samples were separated by sodium dodecyl sulfate - polyacrylamide gel electrophoresis (SDS-PAGE) and transferred onto nitrocellulose or PVDF (polyvinylidene difluoride) membranes. After blocking non-specific bindings with 1% blotto, 1% bovine serum albumin (BSA) or with Tris-buffered saline (100 mM Tris, pH = 7.5; 150 mM NaCl, 0.05% tween) containing 0.05% tween and 1% BSA, membranes were incubated overnight with primary antibodies at 4°C. HRP-conjugated secondary antibodies were used to detect the proteins. Protein bands were visualized with the Immun-Star Western chemiluminescence kit (Bio-rad) using the ChemiDoc XRS System and the image analysis program Quantity One (Bio-Rad). Densitrometric analyses were also performed using the same instrumentation.

### 5.7: The effects of the compounds of the present invention on the cell viability of various human derived cancer cell lines.

### Cell lines.

K562 [American Type Culture Collection (ATCC) reference number CCL-243]. This a human cancer cell line derived from the bone marrow of a 53-year-old female with CML.

HEL 92.1.7 [American Type Culture Collection (ATCC) reference number TIB-180]. This a human cancer cell line derived from the bone marrow of a 30-year-old male with erythroleukemia, which is a form of AML.

DU-145 [American Type Culture Collection (ATCC) reference number HTB-81]. This a human prostate cancer cell line derived from a metastatic site (brain) of a 68-year-old male.

PANC-1 [American Type Culture Collection (ATCC) reference number CRL-1469]. This a human cancer cell line derived from the pancreas of a 56-year-old male.

SKBR3 [American Type Culture Collection (ATCC) reference number HTB-30]. This a human breast cancer cell line derived from a metastatic site from a 43-year-old female. It is designated as a Herceptin positive (HER+) breast cancer.

U2 OS [American Type Culture Collection (ATCC) reference number HTB-96]. This a human cancer cell line derived from the bone of a 15-year-old female.

MDA-MB-231 [American Type Culture Collection (ATCC) reference number HTB-26]. This a human breast cancer cell line derived from a metastatic site from a 51-year-old female.

### MTT Cell viability assay.

Cell viability was assayed by measuring the mitochondrial reduction of the tetrazolium salt 3-(4,5-methyltiazol-2yl)-2,5diphenyl-tetrazolium bromide] (MTT) (Applichen; A2231,0010) [Mosmann T. J Immunol Methods. 1983 16;65(1-2):55-63 (ref. 36); Chuan Y-C et al 2010. Oncogene. 29 (10), 1531-1542 (ref. 37)]. Cells were seeded in 96-well plates in medium (either RPMI, DMEM or McCoys depending on the recommendations from the ATCC) supplemented with 10% FBS (Lonza Cat. N°: 14-502F), 2mM L-Glut (Lonza Cat. N°: BE17-605E) and 100U/ml PEST (Lonza Cat. N°: DE17-605E) at a cell density of 1000-5000 cells/well (96 well plate). Cells were seeded at a density according to their exponential growth phase (normally 1000 to 5000/well). The cells were cultured at 37 °C in the dark in air containing 5% CO₂. After 24h either vehicle (0.05% DMSO) or drug were added to culture medium at the indicated concentrations (10 to 0,01microM). After another 48 h, MTT (0.3 mg/ml) was added to cells, followed by incubation for 2-4h at 37 °C in the dark. The medium was then removed and the precipitated formazan was solubilized by the addition of DMSO (200 µL).

The optical density was measured at 595 nm with the iMark Microplate Reader (BioRad, USA). Data was expressed as percent growth above the level in controls and the results in figures were plotted as means ± SEM of each test point from 3 replicates.

Statistical analysis. The concentration of drug that provokes 50% reduction of cell viability (IC₅₀) was determined by using non-linear regression analysis and data were fitted to a log concentration vs normalized response curve in GraphPad software v5 (San Diego, CA).

### Results

Table 7 below sets forth potencies of representative compounds of the invention in a variety of human derived cancer cell lines that present elevated levels of peroxiredoxins.

**Table 7**

| **Compound #** | **IC₅₀ (µM) K562** | **IC₅₀ (µM) HEL** | **IC₅₀ (µM) MDA-MB-231** | **IC₅₀ (µM) PANC-1** | **IC₅₀ (µM) MCF-7** | **IC₅₀ (µM) DU-145** |
|---|---|---|---|---|---|---|
| 1 | 0.98 | 0.45 | 1.59 | - | - | - |
| 2 | 0.57 | 0.35 | 4.02 | 5.60 | 4.22 | >10 |
| 3 | 0.90 | - | - | - | - | - |
| 4 | 0.98 | - | 1.52 | 4.36 | 5.12 | 8.90 |
| 5 | 0.43 | - | - | - | - | - |
| 7 | 0.89 | - | - | - | - | - |
| 8 | 0.23 | 0.10 | 0.36 | 0.32 | 2.59 | 6.04 |
| 9 | 0.08 | - | 0.38 | 0.46 | 1.15 | 2.53 |
| 10 | 0.09 | - | 0.63 | 0.36 | 1.85 | 6.18 |
| 11 | 0.48 | - | 1.31 | 0.46 | 2.83 | - |
| 12 | 0.43 | - | 1.67 | 0.46 | 2.79 | - |
| 13 | 0.32 | - | 0.78 | 0.46 | 2.75 | 3.72 |
| 14 | 0.97 | - | - | - | - | - |
| 17 | 0.65 | - | - | - | 5.91 | 5.77 |
| 18 | >10 | - | - | - | - | - |
| 19 | 1.39 | 1.00 | - | - | 7.90 | 8.36 |
| 20 | 1.42 | - | - | - | - | - |
| 21 | 1.12 | - | - | - | - | - |
| 22 | 0.61 | - | 3.21 | 0.51 | 3.64 | 7.99 |
| 23 | 0.54 | - | 2.54 | 0.57 | 3.23 | 8.16 |
| 24 | 0.10 | 0.09 | 0.09 | 0.32 | 1.72 | 3.19 |
| 25 | 0.89 | - | 0.20 | 0.34 | 1.95 | 3.27 |
| 26 | 0.13 | - | 0.39 | 0.47 | 3.17 | 7.35 |
| 27 | - | - | 1.73 | - | - | - |
| 28 | - | - | 2.00 | - | - | - |
| 29 | - | - | >10 | - | - | - |
| 30 | - | - | 0.89 | - | - | - |
| 31 | - | - | 1.51 | - | - | - |
| 32 | - | - | 0.79 | - | - | - |
| 33 | - | - | 0.72 | - | - | - |
| 34 | - | - | 0.74 | - | - | - |
| 35 | - | - | 0.80 | - | - | - |
| 36 | - | - | 0.15 | - | - | - |
| 40 | 0.59 | 0.21 | 1.96 | 0.63 | 2.52 | 6.13 |
| 41 | 0.22 | - | 0.98 | 0.39 | 1.53 | 3.74 |
| 43 | 0.76 | - | 0.81 | 0.74 | 1.30 | - |
| 44 | 0.06 | - | 0.73 | 0.32 | 0.97 | - |
| 45 | 0.09 | 0.09 | 0.13 | - | - | 0.57 |
| 46 | - | 0.11 | 0.25 | - | - | - |
| 47 | - | - | 0.33 | - | - | - |
| 48 | - | 0.10 | 0.27 | - | - | - |
| 49 | - | 1.22 | 2.63 | 3.43 | - | - |
| 50 | - | 2.42 | - | - | - | - |
| 51 | - | 3.31 | 0.60 | - | - | - |
| 52 | - | >10 | >10 | - | - | - |
| 53 | - | 0.12 | 0.64 | - | - | - |
| 54 | 0.07 | 0.09 | 0.19 | 0.38 | 1.28 | 3.41 |
| 55 | 0.33 | - | 3.35 | 0.89 | 3.45 | - |
| 56 | 0.19 | - | 0.40 | - | 3.04 | 7.22 |
| 57 | - | 0.13 | 1.17 | - | - | - |
| 58 | - | - | 0.50 | - | - | - |
| 59 | 0.05 | 0.06 | 0.21 | 0.28 | 0.72 | 0.54 |
| 60 | - | 0.08 | 0.41 | - | - | - |
| 61 | 0.16 | 0.10 | 0.41 | - | - | 0.92 |
| 62 | 0.10 | 0.07 | 0.27 | 1.55 | 3.00 | 1.32 |
| 63 | - | 0.09 | 0.25 | - | - | - |
| 64 | 1.30 | 5.89 | - | - | - | - |
| 66 | - | 0.05 | 1.21 | - | 2.34 | - |
| 67 | - | 0.23 | 0.28 | - | - | - |
| 68 | - | - | 2.40 | - | - | - |
| 70 | - | - | 7.10 | - | - | - |
| 71 | - | - | 3.20 | - | - | - |
| 72 | - | - | 0.72 | - | - | - |
| 73 | - | - | 1.64 | 1.70 | 2.27 | 2.70 |
| 74 | - | - | 1.80 | - | - | - |
| 75 | - | - | 2.70 | 5.49 | 2.36 | 3.89 |
| 76 | - | - | 1.80 | 1.86 | 2.00 | 2.30 |
| 78 | - | - | 6.90 | - | - | - |
| 79 | - | - | 6.80 | - | - | - |
| 80 | - | - | 6.60 | - | - | - |
| 81 | 0.02 | 0.05 | 0.32 | 0.26 | 0.91 | 0.82 |
| 82 | - | 0.18 | 0.80 | - | - | - |
| 84 | - | 3.71 | 1.18 | - | 3.98 | - |
| 85 | 0.38 | 0.33 | 1.03 | 1.78 | 2.57 | - |
| 86 | - | 0.59 | 1.28 | 3.89 | - | - |
| 87 | - | 0.08 | 0.15 | - | - | - |
| 88 | - | 0.08 | - | - | - | - |
| 90 | - | 0.09 | - | - | - | - |
| 92 | - | 0.12 | 0.15 | - | - | - |
| 94 | - | - | 1.00 | - | - | - |
| 95 | - | - | 0.15 | - | - | - |
| 96 | - | - | 0.13 | - | - | - |
| 97 | 0.44 | - | 0.35 | 0.42 | 0.79 | 0.68 |
| 98 | - | - | 2.17 | 2.94 | 3.54 | 4.70 |
| 99 | - | - | 2.09 | 2.61 | 2.28 | 4.30 |
| 100 | - | - | 1.63 | 1.80 | 1.95 | 3.40 |
| 102 | 0.42 | - | 0.17 | - | - | 1.00 |
| 103 | - | - | 1.40 | 1.41 | 1.74 | 1.80 |
| 104 | - | - | 1.02 | 1.23 | 1.82 | 1.60 |
| 105 | - | - | 0.71 | 0.69 | 1.99 | 1.51 |
| 106 | - | - | 0.74 | 0.71 | - | - |
| 107 | - | - | 5.90 | - | - | - |
| 108 | - | - | 2.95 | - | - | - |

| | | | | | | |
|---|---|---|---|---|---|---|
| - represents experiment not done. | | | | | | |

### References

(1) Rhee, SG, Overview on Peroxiredoxin, Mol. Cells 2016; 39(1): 1-5
(2) Karplus PA: A primer on peroxiredoxin biochemistry. Free Radic Biol Med 80: 183-190, 2015.
(3) Winterbourn CC: Reconciling the chemistry and biology of reactive oxygen species. Nat Chem Biol 4: 278-286, 2008.
(4) Fujii J and Ikeda Y: Advances in our understanding of peroxiredoxin, a multifunctional, mammalian redox protein. Redox Rep 7: 123-130, 2002.
(5) Kim YJ, Lee WS, Ip C, Chae HZ, Park EM, Park YM. Prx1 suppresses radiation-induced c-Jun NH2-terminal kinase signaling in lung cancer cells through interaction with the glutathione S-transferase Pi/c-Jun NH2-terminal kinase complex. Cancer Res. 2006; 66: 7136-42.
(6) Wen ST, Van Etten RA. The PAG gene product, a stress-induced protein with antioxidant properties, is an Abl SH3- binding protein and a physiological inhibitor of c-Abl tyro¬sine kinase activity. Genes Dev. 1997; 11: 2456-67.
(7) Egler RA, Fernandes E, Rothermund K, Sereika S, de Souza-Pinto N, Jaruga P, Dizdaroglu M, Prochownik EV. Regulation of reactive oxygen species, DNA damage, and c-Myc function by peroxiredoxin 1. Oncogene. 2005; 24: 8038-50.
(8) Cao J, Schulte J, Knight A, Leslie NR, Zagozdzon A, Bronson R, Manevich Y, Beeson C, Neumann CA. Prdx1 inhibits tumorigenesis via regulating PTEN/AKT activity. EMBO J. 2009; 28: 1505-17.
(9) Hu X, Weng Z, Chu CT, et al. Peroxiredoxin-2 protects against 6-hydroxydopamine-induced dopaminergic neurodegeneration via attenuation of theapoptosis signal-regulating kinase (ASK1) signaling cascade. J Neurosci 2011; 31: 247-261.
(10) Sobotta MC, Liou, W, Stöcker S, Talwar D, Oehler M, Ruppert T, Scharf AND, Dick TP; Nature chemical biology 2015, 11, 64-70.
(11) Rhee SG, Chae HZ and Kim K: Peroxiredoxins: A historical overview and speculative preview of novel mechanisms and emerging concepts in cell signaling. Free Radic Biol Med 38: 1543-1552, 2005.
(12). Seo MS, Kang SW, Kim K, Baines IC, Lee TH and Rhee SG: Identification of a new type of mammalian peroxiredoxin that forms an intramolecular disulfide as a reaction intermediate. J Biol Chem 275: 20346-20354, 2000.
(13) .Kang SW, Baines IC and Rhee SG: Characterization of a mammalian peroxiredoxin that contains one conserved cysteine. J Biol Chem 273: 6303-6311, 1998.
(14). Fujii J, Ikeda Y, Kurahashi T and Homma T: Physiological and pathological views of peroxiredoxin 4. Free Radic Biol Med 83: 373-379, 2015
(15) Wood ZA, Poole LB, Hantgan RR and Karplus PA: Dimers to Doughnuts: Redox-sensitive oligomerization of 2-cysteine peroxiredoxins. Biochemistry 41: 5493-5504, 2002.
(16) Barranco-Medina S, Lázaro JJ and Dietz KJ: The oligomeric conformation of peroxiredoxins links redox state to function. FEBS Lett 583: 1809-1816, 2009.
(17) Wood ZA, Schroder E, Robin Harris J, Poole LB. Structure, mechanism and regulation of peroxiredoxins. Trends Biochem Sci. 2003; 28: 32-40.
(18) Nicolussi A, D'INZEO S , CAPALBO C, GIANNINI G, COPPA A; The role of peroxiredoxins in cancer Mol Clin Oncol 6: 139-153, 2017
(19) Kim Y, Jang HH, The Role of Peroxiredoxin Family in Cancer Signaling, J Cancer Prev. 2019 Jun; 24(2): 65-71.
(20) Ishii T, ¡Warabi E, Yanagawa T; Novel roles of peroxiredoxins in inflammation, cancer and innate immunity; J. Clin. Biochem. Nutr., 2012 (50); 2; 91-105
(21) Zhang B, Wang Y, Su Y. Peroxiredoxins, a novel target in cancer radiotherapy. Cancer Lett. 2009; 286:154-160
(22) Chen MF, Keng PC, Shau H, Wu CT, Hu YC, Liao SK, and Chen WC. Inhibition of lung tumor growth and augmentation of radiosensitivity by decreasing peroxiredoxin I expression. Int J Radiat Oncol Biol Phys 64: 581-591, 2006.
(23) Kalinina EV, Berezov TT, Shtil AA, Chernov NN, Glazunova VA, Novichkova MD, and Nurmuradov NK. Expression of peroxiredoxin 1, 2, 3, and 6 genes in cancer cells during drug resistance formation. Bull Exp Biol Med 153: 878-881, 2012.
(24) (a) Liu C-X, et. al. Adenanthin targets peroxiredoxin land IIto induce differentiation of leukemic cells, Nature chemical biology 2012, 8, 486-493. (b) Hou, J-K, et.al. Adenanthin targets peroxiredoxin I/II to kill hepatocellular carcinoma cells, Cell Death and Disease (2014) 5, e1400.
(25) Trzeciecka A, et. Al. Dimeric peroxiredoxins are druggable targets in human Burkitt lymphoma, Oncotarget, 2016, 7: 1717 1731
(26) Yang YJ, et.al. Effective Killing of Cancer Cells Through ROS-Mediated Mechanisms by AMRI-59 Targeting Peroxiredoxin IAntioxid Redox Signal. 2016 Mar 10;24(8):453-69.
(27) Frenolicin B targets Peroxiredoxin 1 and Glutaredoxin 3 to trigger ROS/4E-BP1-mediated antitumor effects, Cell Chem. Biol. 2019, 26, 366-377.
(28) Dunn, R.L., et al., Eds. POLYMERIC DRUGS AND DRUG DELIVERY SYSTEMS, ACS Symposium Series Vol. 469, American Chemical Society, Washington, D.C. 1991
(29) T. W. Greene and P. G.M. Wuts, "Protective Groups in Organic Synthesis", 3rd Ed., Wiley & Sons, Inc, New York (1999)
(30) Brühne, F.; Wright, E. (2005), "Benzaldehyde", Ullmann's Encyclopedia of Industrial Chemistry, Weinheim: Wiley-VCH (30)
(31) Trond Vidar Hansen; Lars Skattebøl (2005), Ortho-Formylation of Phenols; Preparation of 3-Bromosalicylaldehyde, Org. Synth. 82:64 (31).
(32) Orive-Ramos A, Seoane S, Ocaña A, Pandiella A, Montero JC. Regulation of the prometastatic neuregulin-MMP13 axis by SRC family kinases: therapeutic implications. Mol Oncol. 2017 Dec;11(12):1788-1805
(33) Rix et al., Blood, 2007, 110, 4055-4063
(34) Schroder et al., Am J Physiol Heart Circ Physiol. 2008 Jul; 295(1): H425-H433
(35) Turner-Ivey, B. et al., Oncogene. 2013 November 7; 32(45): 5302-5314
(36) Mosmann T. J Immunol Methods. 1983 16;65(1-2):55-63;
(37) Chuan Y-C et al 2010. Oncogene. 29 (10), 1531-1542

## Claims

1. A compound of formula (I), or a pharmaceutically acceptable salt thereof: 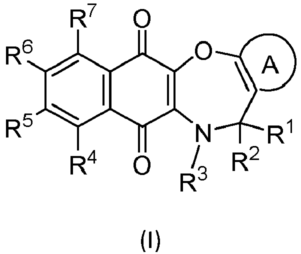 wherein
A is an aromatic carbocyclic 5- or 6-membered ring or an aromatic heterocyclic 5- or 6-membered ring comprising one or more heteroatoms selected from the group consisting of O, N and S, wherein said aromatic carbocyclic ring or said aromatic heterocyclic ring is unsubstituted or substituted at one or more atoms of C with one or more R⁸ substituents; wherein each R⁸ substituent is independently selected from the group consisting of: H, halogen, C₁-C₆ haloalkyl, nitro, cyano, C₁-C₆ alkyl, -OR⁹ and -C(O)OR⁹, and wherein R⁹ is selected from the group consisting of: H and C₁-C₆ alkyl;
R¹ and R² are independently selected from the group consisting of: H, F and C₁-C₆ alkyl;
R³ is an aromatic carbocyclic 5- or 6-membered ring or an aromatic heterocyclic 5- or 6-membered ring comprising one or more heteroatoms selected independently from the group consisting of O, N and S, wherein said aromatic carbocyclic ring or said aromatic heterocyclic ring is unsubstituted or substituted at one or more atoms of C with one or more R¹⁰ substituents; wherein each R¹⁰ substituent is independently selected from the group consisting of: H, halogen, C₁-C₆ alkyl, C₁-C₆ haloalkyl, nitro, cyano, -C(O)NR¹¹R¹², -OR¹¹, -C(O)OR¹¹_{,} -NR¹¹R¹², -SR¹¹, -SO₂R¹¹, and -SO₂NR¹¹R¹²;
R⁴, R⁵, R⁶ and R⁷ are each independently selected from the group consisting of: H, halogen, C₁-C₆ alkyl, C₁-C₆ haloalkyl, -OR¹¹, -OC(O)R¹¹, nitro, cyano, -NR¹¹R¹², -NR¹¹SO₂R¹⁶, -SR¹¹, -SO₂R¹¹ and -SO₂NR¹¹R¹²;
wherein R¹¹ and R¹² are each independently selected from the group consisting of: H, C₁-C₆ alkyl, -R¹³-COOR¹⁴, -R¹³-OR¹⁴ and -R¹³-NR¹⁴R¹⁵, wherein R¹³ is a group -(CH₂)*n-*where *n* is 1, 2, 3 or 4, and wherein R¹⁴ and R¹⁵ are each independently H or C₁-C₆ alkyl;
or wherein R¹¹ and R¹² when taken with the atom to which they are both attached, complete a 5- or 6-membered substituted or unsubstituted cycloalkyl or a 5- or 6-membered substituted or unsubstituted heterocycloalkyl group comprising at least one heteroatom selected from the group consisting of O, N and S; and wherein R¹⁶ is C₁-C₆ alkyl or C₁-C₆ haloalkyl.

2. A compound of formula (I), or a pharmaceutically acceptable salt thereof, according to claim 1, wherein:
A is a phenyl ring unsubstituted or substituted with one or more R⁸ substituents, wherein each R⁸ substituent is independently selected from the group consisting of: H, halogen, -OR⁹ and -C(O)OR⁹, and wherein R⁹ is selected from the group consisting of: H and C₁-C₆ alkyl;
R¹ and R² are both H;
R³ is a phenyl ring, a pyridyl ring or a pyrimidinyl ring, and wherein said ring is unsubstituted or substituted at one or more atoms of C with one or more R¹⁰ substituents, wherein each R¹⁰ substituent is independently selected from the group consisting of: H, halogen, cyano, -C(O)NR¹¹R¹², -OR¹¹, -C(O)OR¹¹, -NR¹¹R¹² and -SO₂NR¹¹R¹²;
R⁴ and R⁵ are each independently selected from the group consisting of: H, -OR¹¹, -OC(O)R¹¹, nitro, -NR¹¹R¹² and -NR¹¹SO₂R¹⁶; and
R⁵ and R⁶ are each independently selected from the group consisting of: H, halogen, -NR¹¹R¹² and -SO₂NR¹¹R¹²;
wherein R¹¹ and R¹² are each independently selected from the group consisting of: H, C1-C6 alkyl, -R¹³-C(O)OR¹⁴, -R¹³-OR¹⁴ and -R¹³-NR¹⁴R¹⁵, wherein R¹³ is a group -(CH₂)*n-*where *n* is 1 or 2, and wherein R¹⁴ and R¹⁵ are each independently H or C₁-C₆ alkyl;
or wherein R¹¹ and R¹² when taken with the atom to which they are both attached, complete a 6-membered substituted or unsubstituted heterocycloalkyl group comprising one or two heteroatoms selected from the group consisting of O and N; and wherein R¹⁶ is C₁-C₆ alkyl.

3. An antibody-drug conjugate comprising a compound of formula (I), or a pharmaceutically acceptable salt thereof, according to any of claims 1 or 2, and a human or humanized monoclonal or polyclonal antibody.

4. A pharmaceutical composition comprising at least one compound of formula (I), or a pharmaceutically acceptable salt thereof, according to any of claims 1 or 2, or an antibody-drug conjugate according to claim 3, and at least one pharmaceutically acceptable excipient or carrier.

5. A pharmaceutical composition according to claim 4, further comprising at least one additional active compound or agent selected from the group consisting of chemotherapeutic, cell therapy and immunotherapeutic agents.

6. A compound of formula (I), or a pharmaceutically acceptable salt thereof, according to any of claims 1 or 2, for use as a medicament.

7. A compound of formula (I), or a pharmaceutically acceptable salt thereof, according to any of claims 1 or 2, for use in the prevention and/or treatment of cancer.

8. A compound for use according to claim 7, wherein the cancer is a chemo/radio-resistant cancer or a metastatic cancer.

9. A compound for use according to any of claims 7 or 8, wherein the cancer is selected from the group consisting of: sarcoma, breast cancer, prostate cancer, head and neck cancer, brain tumour, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.

10. A compound for use according to any of claims 6 to 9, wherein said compound is administered as an antibody-drug conjugate.

11. A compound for use according to any of claims 6 to 10, wherein said compound is administered together, prior to, or subsequently to radiotherapy, immunotherapy or chemotherapy.

12. A pharmaceutical composition according to any of claims 4 or 5, for use as a medicament.

13. A pharmaceutical composition according to any of claims 4 or 5, for use in the prevention and/or treatment of cancer.

14. A pharmaceutical composition for use according to claim 13, wherein the cancer is a chemo/radio-resistant cancer or a metastatic cancer.

15. A pharmaceutical composition for use according to any of claims 13 or 14, wherein the cancer is selected from the group consisting of: sarcoma, breast cancer, prostate cancer, head and neck cancer, brain tumour, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.
